# EUROPÄISCHE PATENTSCHRIFT

(11) **EP 1 434 485 B1**
(45) Veröffentlichungstag und Bekanntmachung des Hinweises auf die Patenterteilung: **16.05.2007**
(21) Anmeldenummer: 02777107.0
(22) Anmeldetag: 14.09.2002
(51) Int. Cl.: A01N 43/40, A01N 43/50, A61K 31/455, A61K 31/506, C07D 405/12, C07D 409/12, C07D 401/12, C07D 409/14, C07D 451/02, C07F 7/08

(54) **NICOTINSÄURE-HETEROCYCLYL-AMIDE UND ANALOGE PYRIMIDIN-DERIVATE ALS SCHÄDLINGSBEKÄMPFUNGSMITTEL**
NICOTINIC ACID-HETEROCYCLYL-AMIDES AND ANALOGOUS PYRIMIDINE DERIVATIVES FOR USE AS PESTICIDES
HETEROCYCLYLAMIDES D'ACIDE NICOTINIQUE ET DERIVES DE PYRIMIDINE ANALOGUES POUR L'UTILISATION COMME PESTICIDES

(30) Priorität: 29.09.2001 DE 10148290
(43) Veröffentlichungstag der Anmeldung: 07.07.2004
(73) Patentinhaber: Merial Limited, Duluth, GA 30096 (US)
(72) Erfinder: SCHAPER, Wolfgang, 86420 Diedorf (DE); BECKMANN, Marion, 65195 Wiesbaden (DE); DÖLLER, Uwe, 63110 Rodgau (DE); KRAUTSTRUNK, Gerhard, 61118 Bad Vilbel (DE); JANS, Daniela, 61348 Bad Homburg (DE); WAIBEL, Jutta,M., 60529 Frankfurt (DE); HEMPEL, Waltraut., 65835 Liederbach (DE)
(74) Vertreter: Harding, Charles Thomas
(86) Internationale Anmeldenummer: PCT/EP2002/010330
(87) Internationale Veröffentlichungsnummer: WO 2003/028459

(56) Entgegenhaltungen:
- EP-A- 0 580 374
- EP-A- 0 799 825
- WO-A-01/70692
- WO-A-96/11690
- PATENT ABSTRACTS OF JAPAN vol. 1998, no. 12, 31. Oktober 1998 (1998-10-31) & JP 10 195072 A (SUMITOMO CHEM CO LTD (JP)), 28. Juli 1998 (1998-07-28)

## Beschreibung

Die Erfindung betrifft heterocyclische Amide, Verfahren zu ihrer Herstellung, sie enthaltende Mittel sowie ihre Verwendung zur Bekämpfung von tierischen Schädlingen, insbesondere Arthropoden, wie Insekten und Acarina, und Helminthen.

Wegen des enormen Schadens, den Insekten beispielsweise durch Fraß an Nutzpflanzen, Lebensmittelvorräten, Holz und Textilien oder auch durch Krankheitsübertragung auf Mensch, Haustiere und Nutzpflanzen verursachen, ist die Verwendung von Insektiziden oder Repellentien nach wie vor unverzichtbar. Insektizide sind ein wichtiger Bestandteil der integrierten Schädlingskontrolle und tragen entscheidend zu Ernteertrag und Kontinuität der Ernten in aller Welt bei.

Aus EP-A 0 580 374 sind Trifluormethylpyridinamide als Schädlingsbekämpfungs-mittel bekannt.

JP-A 10 195072 offenbart pestizid wirksame Pyridin-3-carboxamide, wobei das Amid mit zwei Heteroaromaten substituiert ist. Es werden jedoch nur zwei spezielle Heteroaromaten offenbart. Eine Übertragung auf andere Aromaten oder gar aliphatische heterozyklische Substituenten wird weder offenbart noch nahe gelegt.

WO 01 70692 offenbart pestizid wirksame Sulfimide. Die Benutzung von heterozyklisch substituierten Pyridin- oder Pyrimidinamiden wird dagegen weder offenbart noch nahe gelegt.

EP-A 0799825 offenbart Pyridin-2,3-dicarbonsäureamide, wobei das Amid mit einem Phenylrest substituiert ist. Eine Substitution des Amids mit einem aliphatischen Heterozyklus wird weder offenbart noch nahegelegt.

WO 96 11690 offenbart PDE IV-Inhibitoren auf Diazepin-Indol-Basis, wobei ein Beispiel strukturelle Ähnlichkeit zu den erfindungsgemäßen Verbindungen zeigt. Eine Verwendung der offenbarten Verbindungen als Pflanzenschutzmittel wird weder offenbart noch nahegelegt.

Da sich aber die ökologischen und ökonomischen Anforderungen an moderne Insektizide laufend erhöhen, beispielsweise was Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Insektizide zu entwickeln, die zumindest in Teilbereichen Vorteile gegenüber den bekannten aufweisen.

Es wurde gefunden, daß Verbindungen der allgemeinen Formel (I), gegebenenfalls auch als Salze, ein gutes Wirkungsspektrum gegenüber tierischen Schädlingen bei gleichzeitig guter Pflanzenverträglichkeit und günstigen toxikologischen Eigenschaften gegenüber Säugetieren und aquatischen Lebewesen aufweisen.

Gegenstand der Erfindung sind daher Amide der Formel (I) und deren Salze, 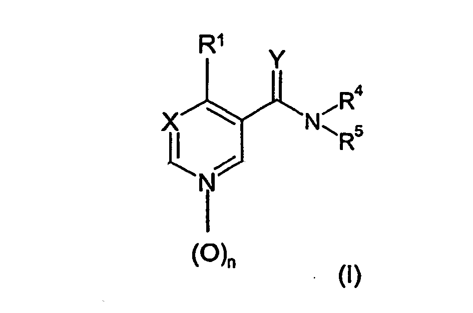 wobei die Symbole und Indizes folgende Bedeutungen haben:
- X: ist CH oder N;
- Y: ist O oder S;
- n: ist 0 oder 1;
- R¹: ist (C₁-C₄)-Haloalkyl;

R⁴ ist Wasserstoff, (C₁-C₁₀)-Alkyl, (C₃-C₁₀)-Cycloalkyl, (C₃-C₁₀)-Alkenyl, oder (C₃-C₁₀)-Alkinyl, wobei in den genannten Alkyl-, Cycloalkyl-, Alkenyl- oder Alkinyl-Gruppen bis zu drei Wasserstoffatome durch Halogen, bevorzugt Fluor oder Chlor, ersetzt sein können, im Falle von Fluor auch bis zur Maximalanzahl;

R⁵ ist ein nicht-aromatischer Heterocyclus, der vorzugsweise vier- bis achtgliedrig ist, enthaltend mindestens ein Sauerstoff-, Schwefel-, Stickstoff- und/oder Siliziumringatom, und der gegebenenfalls Teil eines spirocyclischen, kondensierten oder bicyclischen Ringsystems sein kann.

R⁵ ist vorzugsweise ein vier-bis achtgliedriger nicht-aromatischer Heterocyclus, der ein Sauerstoffringatom, ein Schwefelringatom, ein Stickstoffringatom, ein Siliziumringatom, zwei Sauerstoffringatome oder zwei Schwefelringatome aufweist, wobei die Schwefelringatome in der Form -S(O)ₚ- vorliegen, und p 0, 1 oder 2 bedeutet.

Ganz besonders bevorzugt ist R⁵ ein vier- bis achtgliedriger nicht-aromatischer Heterocyclus der Formel (II), 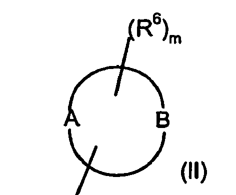 worin A und/oder B eine mindestens ein Sauerstoff-, Schwefel-, Stickstoff oder Siliziumringatom enthaltende Einheit, vorzugsweise -O-, -S(O)_{0,1,2}-, -NR⁸- oder -SiR⁹R¹⁰- bedeuten, und der Ring zusätzlich eine oder zwei Carbonylgruppen enthalten kann, die zusammen mit den Heteroeinheiten gegebenenfalls eine Lacton-, Lactam- oder Imid-Einheit bilden; und, falls A Sauerstoff und B -NR⁸- bedeutet, diese Einheiten unmittelbar benachbart sein können und in allen anderen Fällen, für die A und B Heteroatomeinheiten bedeuten, diese durch mindestens eine gesättigte Kohlenstoff-Einheit getrennt sein müssen;
- m: 0 oder 1 bis 6, bevorzugt 0, 1 oder 2 bedeutet,
- R⁶: in Abhängigkeit von m gleich oder verschieden und jeweils einen einwertigen Substituenten des heterocycloaliphatischen Ringes bedeutet, wie (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl, das jeweils mit einem oder mehreren gleichen oder verschiedenen Resten substituiert sein kann, oder einen Rest R⁷, wobei
- R⁷: Halogen, Cyano, Nitro, Hydroxy, Thio, Amino, (C₁-C₁₀)-Alkanoyl, (C₃-C₁₀)-Alkenoyl, (C₃-C₁₀)-Alkinoyl, (C₃-C₁₀)-Cycloalkanoyl, (C₁-C₁₀)-Alkoxy, (C₃-C₁₀)-Alkenyloxy, (C₃-C₁₀)-Alkinyloxy, (C₃-C₁₀)-Cycloalkoxy, (C₄-C₁₀)-Cycloalkenyloxy, (C₃-C₈)-Cycloalkyl-(C₁-C₄)-alkoxy, (C₄-C₈)-Cycloalkenyl-(C₁-C₄)-alkoxy, (C₃-C₈)-Cycloalkyl-(C₃-C₄)-alkenyloxy, (C₄-C₈)-Cycloalkenyl-(C₃-C₄)-alkenyloxy, (C₁-C₄)-Alkyl-(C₃-C₈)-cycloalkoxy, (C₂-C₄)-Alkenyl-(C₃-C₈)-cycloalkoxy, (C₂-C₄)-Alkinyl-(C₃-C₈)-cycloalkoxy, (C₁-C₄)-Alkyl-(C₄-C₈)-cycloalkenyloxy, (C₂-C₄)-Alkenyl-(C₄-C₈)-cycloalkenyloxy, (C₁-C₄)-Alkoxy-(C₁-C₄)-alkoxy, (C₁-C₄(Alkoxy-(C₃-C₄)-alkenyloxy, Carbamoyl, (C₁-C₈)-Mono- oder -Dialkylcarbamoyl, (C₃-C₈)-Mono- oder -Dicycloalkylcarbamoyl, (C₁-C₈)-Alkoxycarbonyl, (C₃-C₈)-Cycloalkoxycarbonyl, (C₁-C₈)-Alkanoyloxy, (C₃-C₈)-Cycloalkanoyloxy, (C₁-C₈)-Alkanoylamino, (C₃-C₈)-Alkenoylamino, (C₃-C₈)-Cycloalkanoyl-amino, (C₃-C₈)-Cycloalkyl-(C₁-C₄)-alkanoylamino, die N-(C₁-C₄)-Alkylamino-Analogen der vier letztgenannten Reste, (C₁-C₁₀)-Alkylthio, (C₃-C₁₀)-Alkenylthio, (C₃-C₁₀)-Alkinylthio, (C₃-C₈)-Cycloalkylthio, (C₄-C₈)-Cycloalkenylthio, (C₃-C₈)-Cycloalkyl-(C₁-C₄)-alkylthio, (C₄-C₈)-Cycloalkenyl-(C₁-C₄)-alkylthio, (C₃-C₈)-Cycloalkyl-(C₃-C₄)-alkenylthio, (C₄-C₈)-Cycloalkenyl-(C₃-C₄)-alkenylthio, (C₁-C₄)-Alkyl-(C₃-C₈)-cycloalkylthio, (C₂-C₄)-Alkenyl-(C₃-C₈)-cycloalkylthio, (C₂-C₄)-Alkinyl-(C₃-C₈)-cycloalkylthio, (C₁-C₈)-Alkyl-(C₄-C₈)-cycloalkenylthio, (C₂-C₆)-Alkenyl-(C₄-C₈)-cycloalkenylthio, (C₁-C₁₀)-Alkylsulfinyl, (C₃-C₄)-Alkenylsulfinyl, (C₃-C₄)-Alkinylsulfinyl, (C₃-C₁₀)-Cycloalkylsulfinyl, (C₄-C₁₀)-Cycloalkenylsulfinyl, (C₃-C₈)-Cycloalkyl-(C₁-C₄)-alkylsulfinyl, (C₄-C₈)-Cycloalkenyl-(C₁-C₄)-alkylsulfinyl, (C₃-C₈)-Cycloalkyl-(C₃-C₄)-alkenylsulfinyl, (C₄-C₈)-Cycloalkenyl-(C₃-C₄)-alkenylsulfinyl, (C₁-C₄)-Alkyl-(C₃-C₈)-cycloalkylsulfinyl, (C₂-C₄)-Alkenyl-(C₃-C₈)-cycloalkylsulfinyl, (C₂-C₄)-Alkinyl-(C₃-C₈)-cycloalkylsulfinyl, (C₁-C₄)-Alkyl-(C₄-C₈)-cycloalkenylsulfinyl, (C₂-C₃)-Alkenyl-(C₄-C₈)-cycloalkenylsulfinyl, (C₁-C₁₀)-Alkylsulfonyl, (C₃-C₁₀)-Alkenylsulfonyl, (C₃-C₁₀)-Alkinylsulfonyl, (C₃-C₁₀)-Cycloalkylsulfonyl, (C₄-C₁₀)-Cycloalkenylsulfonyl, (C₃-C₈)-Cycloalkyl-(C₁-C₄)-alkylsulfonyl, (C₄-C₈)-Cycloalkenyl-(C₁-C₄)-alkylsulfonyl, (C₃-C₈)-Cycloalkyl-(C₃-C₄)-alkenylsulfonyl, (C₄-C₈)-Cycloalkenyl-(C₃-C₄)-alkenylsulfonyl, (C₁-C₄)-Alkyl-(C₃-C₈)-cycloalkylsulfonyl, (C₂-C₄)-Alkenyl-(C₃-C₈)-cycloalkylsulfonyl, (C₃-C₆)-Alkinyl-(C₃-C₈)-cycloalkylsulfonyl, (C₁-C₄)-Alkyl-(C₄-C₈)-cycloalkenylsulfonyl, (C₃-C₄)-Alkenyl-(C₄-C₈)-cycloalkenylsulfonyl, (C₁-C₁₀)-Alkylamino, (C₃-C₁₀)-Alkenylamino, (C₃-C₁₀)-Alkinylamino, (C₃-C₈)-Cycloalkylamino, (C₄-C₈)-Cycloalkenylamino, (C₃-C₈)-Cycloalkyl-(C₁-C₄)-alkylamino, (C₄-C₈)-Cycloalkenyl-(C₁-C₄)-alkylamino, (C₃-C₈)-Cycloalkyl-(C₃-C₄)-alkenylamino, (C₄-C₈)-Cycloalkenyl-(C₃-C₄)-alkenylamino, (C₁-C₄)-Alkyl-(C₃-C₈)-cycloalkylamino, (C₂-C₄)-Alkenyl-(C₃-C₈)-cycloalkylamino, (C₂-C₄)-Alkinyl-(C₃-C₈)-cycloalkylamino, (C₁-C₄)-Alkyl-(C₄-C₈)-cycloalkenylamino, (C₂-C₄)-Alkenyl-(C₄-C₈)-cycloalkenylamino, die N-(C₁-C₄)-Alkylamino-Analogen der vierzehn letztgenannten Reste, (C₁-C₁₀)-Trialkylsilyl, Aryl, Aroyl, Heterocyclylcarbonyl, Aryloxy, Arylthio, Arylamino, N-(C₁-C₄)-Alkyl-arylamino, die N-(C₁-C₄)-Alkylamino-Analogen der zwei letztgenannten Reste, Aryl-(C₁-C₄)-alkoxy, Aryl-(C₃-C₄)-alkenyloxy, Aryl-(C₁-C₄)-alkylthio, Aryl-(C₃-C₄)-alkenylthio, Aryl-(C₁-C₄)-alkylamino, Aryl-(C₃-C₄)-alkenylamino, das N-(C₁-C₄)-Alkylamino-Analoge des letztgenannten Restes, Aryl-(C₁-C₈)-dialkylsilyl, Diaryl-(C₁-C₈)-alkylsilyl, Triarylsilyl, Heterocyclyl, Heterocyclyloxy, Heterocyclylthio, oder Heterocyclylamino bedeutet, wobei der cyclische Teil der genannten Aryl- oder Heterocyclylreste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Dialkylamino, Trimethylsilyl oder (C₁-C₄)-Alkanoyl substituiert ist;
wobei gegebenenfalls zwei Alkylreste R⁶, wenn sie an das gleiche Kohlenstoffatom gebunden sind, zusammen mit diesem Kohlenstoffatom ein spirocyclisches Ringsystem bilden können, oder gegebenenfalls zwei Alkylreste R⁶, die an verschiedene Kohlenstoffatome gebunden sind, zusammen mit dem aliphatischen Heterocyclus der Formel (II) ein kondensiertes oder bicyclisches Ringsystem bilden oder weiterhin das heteroaliphatische Ringsystem der Formel (II) und ein zusätzliches Aryl- oder Heteroaryl-System zusammen ein kondensiertes Ringsystem bilden; und, falls R⁶ (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl bedeutet, die genannten Reste gegebenenfalls ein- oder mehrfach, vorzugsweise durch Reste R¹¹, substituiert sein können und
- R¹¹: die oben zu R⁷ angegebenen Bedeutungen hat;
- R⁸: Wasserstoff, (C₁-C₁₀)-Alkyl, (C₃-C₁₀)-Alkenyl, (C₃-C₁₀)-Alkinyl, (C₃-C₁₀)-Cycloalkyl, (C₄-C₁₀)-Cycloalkenyl, Aryl, Heterocyclyl, (C₁-C₁₀)-Alkanoyl, (C₃-C₁₀)-Alkenoyl, (C₃-C₁₀)-Alkinoyl, (C₄-C₈)-Cycloalkanoyl, Aroyl, Heterocyclylcarbonyl, Carbamoyl, (C₁-C₆)-Mono- oder -Dialkylcarbamoyl, (C₃-C₁₀)-Mono- oder -Dicycloalkylcarbamoyl, (C₁-C₁₀)-Alkoxycarbonyl, (C₃-C₁₀)-Cycloalkoxycarbonyl; (C₁-C₁₀)-Alkylsulfonyl, (C₃-C₁₀)-Alkenylsulfonyl, (C₃-C₆)-Alkinylsulfonyl, (C₃-C₁₀)-Cycloalkylsulfonyl, (C₄-C₁₀)-Cycloalkenylsulfonyl, (C₃-C₁₀)-Cycloalkyl-(C₁-C₁₀)-Alkylsulfonyl, (C₄-C₈)-Cycloalkenyl-(C₁-C₄)-alkylsulfonyl, (C₃-C₈)-Cycloalkyl-(C₃-C₄)-alkenylsulfonyl, (C₄-C₈)-Cycloalkenyl-(C₃-C₄)-alkenylsulfonyl, (C₁-C₄)-Alkyl-(C₃-C₈)-cycloalkylsulfonyl, (C₂-C₄)-Alkenyl-(C₃-C₈)-cycloalkylsulfonyl, (C₂-C₄)-Alkinyl-(C₃-C₈)-cycloalkylsulfonyl, (C₁-C₄)-alkyl-(C₄-C₈)-cycloalkenylsulfonyl, (C₂-C₄)-Alkenyl-(C₄-C₈)-cycloalkenylsulfonyl, Hydroxy, (C₁-C₁₀)-Alkoxy, (C₃-C₁₀)-Alkenyloxy, (C₃-C₁₀)-Alkinyloxy, (C₃-C₁₀)-Cycloalkoxy, (C₄-C₁₀)-Cycloalkenyloxy, (C₃-C₈)-Cycloalkyl-(C₁-C₄)-alkoxy, (C₄ C₈)-Cycloalkenyl-(C₁-C₄)-alkoxy, (C₃-C₈)-Cycloalkyl-(C₃-C₄)-alkenyloxy, (C₄-C₈)-Cycloalkenyl-(C₃-C₄)-alkenyloxy, (C₁-C₄)-Alkyl-(C₃-C₈)cycloalkoxy, (C₂-C₄)-Alkenyl-(C₃-C₈)-cycloalkoxy, (C₂-C₄)-Alkinyl-(C₃-C₈)-cyclo-alkoxy, (C₁-C₄)-Alkyl-(C₄-C₈)-cycloalkenyloxy, (C₂-C₄)-Alkenyl-(C₄-C₈)-cycloalkenyloxy, (C₁-C₄)-Alkoxy-(C₁-C₄)-alkoxy, (C₁-C₄)-Alkoxy-(C₃-C₄)alkenyloxy, Aryloxy, Aryl-(C₁-C₁₀)-alkoxy, Aryl-(C₃-C₁₀)-alkenyloxy oder Aryl-(C₃-C₁₀)-alkinyloxy bedeutet,
und die vorstehend zu R⁸ genannten Reste gegebenenfalls durch einen oder mehrere, Reste aus der Gruppe Cyano, Nitro, Amino, Hydroxy, Thio, (C₁-C₄)-Alkyl, (C₃-C₁₀)-Cycloalkyl, (C₁-C₄)-Alkoxy, (C₁-C₄)-Alkylthio, (C₁-C₄)-Alkylamino und (C₁-C₄)-Alkanoyl substituiert sein können;
- R⁹ und R¹⁰: (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl, (C₈-C₁₀)-Cycloalkinyl, Aryl, Aryl-(C₁-C₄)-alkyl oder Heterocyclyl, bevorzugt Methyl, bedeutet
und gegebenenfalls in allen zu R⁵, R⁶ , R⁷, R⁸, R⁹, R¹⁰ und R¹¹ genannten Gruppen an Kohlenstoff gebundener Wasserstoff durch ein bis drei Halogenatome, im Falle von Fluor auch bis zur Maximalanzahl, ersetzt sein kann.

Bevorzugt haben die Symbole und Indizes in der Formel (I) folgende Bedeutungen:
- X: ist vorzugsweise -CH-.
- Y: ist vorzugsweise -O-.
- n: ist vorzugsweise 0.
- R¹: ist vorzugsweise ein- oder mehrfach durch F und/oder Cl substituiertes (C₁-C₄)-Alkyl, besonders bevorzugt CF₃, CHF₂ oder CF₂Cl, ganz besonders bevorzugt CF₃.
- R⁴: ist vorzugsweise Wasserstoff und Methyl, besonders bevorzugt Wasserstoff.

Bevorzugt sind weiterhin Verbindungen der Formel (I), für die
- R⁶: (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl bedeutet und die genannten Reste gegebenenfalls ein- oder mehrfach durch Reste R¹¹ substituiert sind, wobei R¹¹ eine der oben angegebenen Bedeutungen hat; besonders bevorzugt bedeutet R⁶ Wasserstoff oder (C₁-C₄)-Alkyl.

Bevorzugt sind weiterhin Verbindungen der Formel (I), für die
- R⁵: vorzugsweise ein fünf- bis siebengliedriger aliphatischer Heterocyclus mit einer oder zwei Heteroatomeinheiten A und/oder B ist, ganz besonders bevorzugt ein fünf- bis siebengliedriger aliphatischer Heterocyclus mit ein oder zwei Heteroatomeinheit(en) und gegebenenfalls zusätzlich einer Carbonylgruppe im Ring, die zusammen mit einer Heteroeinheit eine Lacton- oder Lactam-Einheit bildet.

Bevorzugt sind weiterhin Verbindungen der Formel (I), in denen
- R⁷: Aryl, Aroyl, Heterocyclylcarbonyl, Aryloxy, Arylthio, Arylamino, N-(C₁-C₄)-Alkyl-arylamino, die N-(C₁-C₄)-Alkylamino-Analogen der zwei letztgenannten Reste, Aryl-(C₁-C₄)-alkoxy, Aryl-(C₃-C₄)-alkenyloxy, Aryl-(C₁-C₄)-alkylthio, Aryl-(C₃-C₄)-alkenylthio, Aryl-(C₁-C₄)-alkylamino, Aryl-(C₃-C₄)-alkenylamino, das N-(C₁-C₄)-Alkylamino-Analoge des letztgenannten Restes, Aryl-(C₁-C₈)-dialkylsilyl, Diaryl-(C₁-C₈)alkylsilyl, Triarylsilyl, Heterocyclyl, Heterocyclyloxy, Heterocyclylthio oder Heterocyclylamino bedeutet, wobei der cyclische Teil der genannten Aryl- oder Heterocyclylreste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Alkylthio, (C₁-C₄)-Alkylamino oder (C₁-C₄)-Alkanoyl substituiert ist

Bevorzugt sind weiterhin Verbindungen der Formel (I), für die
- R⁸: (C₁-C₄)-Alkyl, (C₁-C₄)-Alkanoyl, (C₁-C₄)-Alkoxycarbonyl, (C₁-C₄)-Alkyisulfonyl oder (C₁-C₄)-Dialkylcarbamoyl bedeutet.

Bevorzugt sind weiterhin Verbindungen der Formel (I), für die
- A oder B: Sauerstoff, Schwefel, SO, SO₂ oder NR⁸ bedeutet, insbesondere Sauerstoff, Schwefel, SO oder SO₂.

Besonders bevorzugt sind weiterhin Verbindungen der Formel (I), für die
- R⁵: ein fünf- oder sechsgliedriger aliphatischer Heterocyclus mit einer Heteroatomeinheit A oder B ist und diese Heteroatomeinheit vorzugsweise Sauerstoff; Schwefel, S(O) oder S(O)₂ bedeutet und, falls A oder B Sauerstoff bedeutet, gegebenenfalls mit einer zusätzlichen Carbonylgruppe im Ring, die zusammen mit dem Sauerstoffatom eine Lactongruppe bildet.

Die Bezeichnung "Halogen" umfasst Fluor, Chlor, Brom, Iod.

Unter dem Ausdruck "(C₁-C₄)-Alkyl" ist ein unverzweigter oder verzweigter aliphatischer und gesättigter Kohlenwasserstoffrest mit 1, 2, 3 oder 4 Kohlenstoffatomen zu verstehen, wie z. B. der Methyl-, Ethyl-, Propyl-, Isopropyl-, 1-Butyl-, 2-Butyl-, Isobutyl- oder tert-Butyl-Rest.

Entsprechend ist unter Alkylresten mit einem größeren Bereich an Kohlenstoffatomen ein unverzweigter oder verzweigter aliphatischer und gesättigter Kohlenwasserstoffrest zu verstehen, der eine Anzahl an Kohlenstoffatomen enthält, die dieser Bereichsangabe entspricht. Der Ausdruck "(C₁-C₁₀)-Alkyl" umfaßt demnach die die vorgenannten Alkylreste, sowie z. B. den Pentyl-, 2-Methylbutyl-, 1,1-Dimethylpropyl-, Hexyl-, Heptyl-, Octyl-, tert.-Octyl-, Nonyl- oder Decyl-Rest.

Unter "(C₁-C₄)-Haloalkyl" ist eine unter dem Ausdruck "(C₁-C₄)-Alkyl" genannte Alkylgruppe zu verstehen, in der ein oder mehrere Wasserstoffatome durch die gleiche Anzahl gleicher oder verschiedener Halogenatome, bevorzugt Chlor oder Fluor, ersetzt sind, wie die Trifluormethyl-, die 1- oder 2-Fluorethyl-, die 2,2,2-Trifluorethyl-, die Chlormethyl-, Fluormethyl-, die Difluormethyl- oder die 1,1,2,2-Tetrafluorethylgruppe.

Die Bezeichnungen "Alkenyl" und "Alkinyl" mit einer vorangestellten Bereichsangabe von Kohlenstoffatomen bedeuten einen geradkettigen oder verzweigten Kohlenwasserstoffrest mit einer dieser Bereichsangabe entsprechenden Kohlenstoffatomzahl, der mindestens eine Mehrfachbindung beinhaltet, wobei sich diese an beliebiger Position des betreffenden ungesättigten Restes befinden kann. "(C₃ C₁₀)-Alkenyl" steht demnach z.B. für die Allyl-, 2-Methylpropenyl-, 1- oder 2-Butenyl-, Pentenyl-, 2-Methylpentenyl-, Hexenyl-, Heptenyl-, Octenyl-, Nonenyl- oder Decenyl-Gruppe. "(C₂-C₁₀)- Alkenyl" steht z. B. für die vorgenannten Gruppen, sowie zusätzlich die Vinyl-Gruppe.

"(C₃-C₁₀)-Alkinyl" steht z.B. für die Propargyl-, 2-Methylpropinyl-, 2-Butinyl-, Pentinyl-, 2-Methylpentinyl-, Hexinyl-, Heptinyl-, Octinyl-, Noninyl- oder die Decinyl-Gruppe. Unter "(C₂-C₁₀)-Alkinyl" sind die vorstehend genannten Reste sowie die Ethinyl-Gruppe zu verstehen.

"(C₃-C₁₀)-Cycloalkyl" steht für monocyclische und gesättigte Alkylreste, wie den Cyclopropyl-, Cyclobutyl-, Cyclopentyl-, Cyclohexyl-, Cycloheptyl-, Cyclooctyl- oder Cyclodecyl-Rest; für bicyclische und gesättigte Alkylreste, wie den Norbomyl- oder Bicyclo[2.2.2]octyl-Rest, oder auch für kondensierte und gesättigte Systeme, wie z. B. den Dekahydronaphthyl-Rest.

"(C₃-C₁₀)-Cycloalkenyl" steht für monocyclische, mindestens eine Mehrfachbindung enthaltende Alkylreste, wie den Cyclobutenyl-, Cyclopentenyl-, Cyclohexenyl-, Cycloheptenyl-, Cyclooctenyl- oder Cyclodecenyl-Rest; für bicyclische, mindestens eine Mehrfachbindung enthaltende Alkylreste, wie den Norbomenyl- oder Bicyclo[2.2.2]octenyl-Rest oder auch für kondensierte, mindestens eine Mehrfachbindung enthaltende Systeme, wie z. B. den Tetra-, Hexa- oder Oktahydronaphthyl-Rest.

"(C₈-C₁₀)-Cycloalkinyl" steht für den Cyclooctinyl-, Cyclononinyl- oder Cyclodecinyl-Rest.

"(C₁-C₁₀)-Alkanoyl" steht z.B. für die Formyl-, Acetyl-, Propionyl-, Butyryl-, 2-Methylbutyryl-, Pivaloyl-, Octanoyl- oder Decanoyl-Gruppe.

"(C₃-C₁₀)-Alkenoyl" steht z.B. für die Acryl-, Methacryl-, Crotonoyl-, Dimethylacryl- oder Octenoyl-Gruppe.

"(C₃-C₁₀)-Alkinoyl" steht z.B. für die Propinoyl-, Butinoyl-, Hexinoyl- oder Octinoyl-Gruppe.

"(C₃-C₁₀)-Cycloalkanoyl" steht z. B. für Cyclopropylcarbonyl, Cyclobutylcarbonyl, Cyclopentylcarbonyl-, Cyclohexylcarbonyl- oder Cyclooctylcarbonyl.

Unter "(C₁-C₄)-Alkoxy" und "(C₁-C₁₀)-Alkoxy" sind Alkoxygruppen zu verstehen, deren Kohlenwasserstoffreste die unter den Ausdrücken "(C₁-C₄)-Alkyl "und "(C₁-C₁₀)-Alkyl" angegebenen Bedeutungen haben.

Unter "(C₃-C₁₀)-Alkenyloxy", "(C₃ C₁₀)-Alkinyloxy", "(C₃-C₁₀)-Cycloalkoxy" und "(C₄-C₁₀)-Cycloalkenyloxy" sind Alkoxygruppen zu verstehen, deren Kohlenwasserstoffreste die unter den Ausdrücken "(C₃-C₁₀)-Alkenyl", "(C₃-C₁₀)-Alkinyl", "(C₃-C₁₀)-Cycloalkyl" und "(C₄-C₁₀)-Cycloalkenyl" Bedeutungen haben.

"(C₃-C₈)-Cycloalkyl-(C₁-C₄)-alkoxy" steht z. B. für die Cyclopropylmethoxy-, Cyclopropylethoxy-, Cyclobutylmethoxy-, Cyclopentylmethoxy-, Cyclohexylmethoxy- oder die Cyclohexylethoxy-Gruppe.

"(C₄-C₈)-Cycloalkenyl-(C₁-C₄)-alkoxy" steht z. B. für die Cyclobutenylmethoxy-, Cyclopentenylmethoxy-, Cyclohexenylmethoxy- oder die Cyclohexenylethoxy-Gruppe.

"(C₃-C₈)-Cycloalkyl-(C₃-C₄)-alkenyloxy" steht z. B. für die Cyclopropylallyloxy-, Cyclobutylallyloxy- oder die Cyclopentylallyloxy-Gruppe.

"(C₄-C₈)-Cycloalkenyl-(C₃-C₄)-alkenyloxy" steht z. B. für die Cyclobutenylallyloxy- oder die Cyclopentenylallyloxy-Gruppe.

"(C₁-C₄)-Alkyl-(C₃-C₈)-cycloalkoxy" steht z. B. für die Methylcyclopentyloxy-, Ethylcyclopentyloxy-, Methylcyclohexyloxy- oder die Ethylcyclohexyloxy-Gruppe.

"(C₂-C₄)-Alkenyl-(C₃-C₈)-cycloalkoxy" steht z. B. für die Vinylcyclopentyloxy-, Allylcyclopentyloxy-, Vinylcyclohexyloxy- oder die Allylcyclohexyloxy-Gruppe.

"(C₂-C₄)-Alkinyl-(C₃-C₈)-cycloalkoxy" steht z. B. für die Ethinylcyclopentyloxy-, Propinylcyclopentyloxy-, Ethinylcyclohexyloxy- oder die Propinylcyclohexyloxy-Gruppe.

"(C₁-C₄)-Alkyl-(C₄-C₈)-cycloalkenyloxy" steht z. B. für die Methylcyclopentenyloxy-, Ethylcyclopentenyloxy-, Methylcyclohexenyloxy- oder die Ethylcyclohexenyloxy-Gruppe.

"(C₂-C₄)-Alkenyl-(C₃-C₈)-cycloalkoxy" steht z. B. für die Vinylcyclopentyloxy-, Allylcyclopentyloxy-, Vinylcyclohexyloxy- oder die Allylcyclohexyloxy-Gruppe.

"(C₂-C₄)-Alkenyl-(C₃-C₈)-cycloalkenyloxy" steht z. B. für die Vinylcyclopentenyloxy-, Allylcyclopentenyloxy-, Vinylcyclohexenyloxy- oder die Allylcyclohexenyloxy-Gruppe.

"(C₁-C₄)-Alkoxy-(C₁-C₄)-alkoxy" bedeutet eine wie vorstehend definierte Alkoxy-Gruppe, die durch eine weitere Alkoxy-Gruppe substituiert ist, wie z.B. die Ethoxymethoxy-, 1-Methoxyethoxy-, 1-Ethoxyethoxy- oder die 1-Methoxypropoxy-Gruppe.

"(C₁-C₄)-Alkoxy-(C₃-C₄)-alkenyloxy" bedeutet z. B. die Methoxyallyloxy- oder die Ethoxyallyloxy- Gruppe.

"(C₁-C₈)-Mono- oder -Dialkylcarbamoyl" bedeutet z. b. die Methyl-, Ethyl-, Propyl-, Isopropyl-, Butyl- oder tert.-Butyl-carbamoyl-Gruppe oder die Dimethyl-, Diethyl-, Methyl-ethyl- oder die Diisopropylcarbamoyl-Gruppe, aber auch cyclische Derivate wie z. B. die Pyrrolidino- oder die Piperidino-carbamoyl-Gruppe.

"(C₃-C₈)-Mono- oder-Dicycloalkylcarbamoyl" bedeutet z. b. die Cyclopropyl-, Cyclobutyl-, Cyclopentyl- oder Cyclohexyl-carbamoyl-Gruppe, oder die Dicyclopropyl-, Dicyclobutyl-, Dicyclopentyl- oder die Dicyclohexylcarbamoyl-Gruppe.

"(C₁-C₈)-Alkoxycarbonyl" bedeutet z. B. die Methoxycarbonyl-, Ethoxycarbonyl-, Propoxycarbonyl-, Isopropoxycarbonyl-, Butoxycarbonyl-, Isobutoxycarbonyl-, sec.-Butoxycarbonyl- oder die tert. Butoxycarbonyl-Gruppe.

"(C₃-C₈)-Cycloalkoxycarbonyl" bedeutet z. B. die Cyclopropoxycarbonyl-, Cyclobutoxycarbonyl-, Cyclopentyloxycarbonyl- oder die Cyclohexyloxycarbonyl-Gruppe.

"(C₁-C₈)-Alkanoyloxy" bedeutet z. B. die Acetoxy-, Propionyloxy-, Butanoyloxy- oder die Pivaloyloxy-Gruppe.

"(C₃-C₈)-Cycloalkanoyloxy" bedeutet z. B. die Cyclopropylcarbonyloxy-, Cyclobutylcarbonyloxy-, Cyclopentylcarbonyloxy- oder die Cyclohexylcarbonyloxy-Gruppe.

"(C₁-C₈)-Alkanoylamino" bedeutet z. B. die Formylamino-, Acetylamino-, Propionylamino-, Isopropionylamino-, Butanoylamino- oder die Pivaloylamino-Gruppe.

"(C₃-C₈)-Alkenoylamino" bedeutet z. B. die Acrylamino-, Methacrylamino-, Dimethylacrylamino- oder die Crotonylamino-Gruppe.

"(C₃-C₈)-Cycloalkanoylamino" bedeutet z. B. die Cyclopropanoylamino-, Cyclobutanoylamino-, Cyclopentanoylamino- oder die Cyclohexanoylamino-Gruppe.

"(C₃-C₈)-Cycloalkyl-(C₁-C₄)-alkanoylamino" bedeutet z. B. die Cyclopropylacetylamino- oder die Cyclopentylacetylamino-Gruppe.

"(C₁-C₁₀)-Alkylthio" steht für eine Alkylthiogruppe, deren Kohlenwasserstoffrest die unter dem Ausdruck "(C₁-C₁₀)-Alkyl" angegebene Bedeutung hat.

"(C₃-C₁₀)-Alkenylthio" steht für eine Alkenylthiogruppe, deren Kohlenwasserstoffrest die unter dem Ausdruck "(C₃-C₁₀)-Alkenyl" angegebene Bedeutung hat.

"(C₃-C₁₀)-Alkinylthio" steht für eine Alkinylthiogruppe, deren Kohlenwasserstoffrest die unter dem Ausdruck "(C₃-C₁₀)-Alkinyl" angegebene Bedeutung hat.

"(C₃-C₁₀)-Cycloalkylthio" steht für eine Cycloalkylthiogruppe, deren Kohlenwasserstoffrest die unter dem Ausdruck "(C₃-C₁₀)-Cycloalkyl" angegebene Bedeutung hat.

"(C₄-C₁₀)-Cycloalkenylthio" steht für eine Cycloalkenylthiogruppe, deren Kohlenwasserstoffrest die unter dem Ausdruck "(C₄-C₁₀)-Cycloalkenyl" angegebene Bedeutung hat.

"(C₃-C₈)-Cycloalkyl-(C₁-C₄)-alkylthio" steht z.B. für die Cyclopropylmethylthio-, Cyclopropylethylthio-, Cyclopentylmethylthio-, oder die Cyclohexylmethylthio-Gruppe.

"(C₄-C₈)-Cycloalkenyl-(C₁-C₄)-alkylthio" steht z.B. für die Cyclopentenylmethylthio- oder die Cyclohexenylmethylthio-Gruppe.

"(C₃-C₈)-Cycloalkyl-(C₃-C₄)-alkenylthio" steht z.B. für die Cyclopropylallylthio-, Cyclopentylallylthio- oder die Cyclohexylallylthio-Gruppe.

"(C₄-C₈)-Cycloalkenyl-(C₃-C₄)-alkenylthio" steht z.B. für die Cyclopentenylallylthio- oder die Cyclohexenylallylthio-Gruppe.

"(C₁-C₄)-A)ky)-(C₃-C₈)-cycloalkylthio" steht z.B. für die Methylcyclopentylthio- oder die Methylcyclohexylthio-Gruppe.

"(C₁-C₄)-Alkyl-(C₄-C₈)-cycloalkenylthio" steht z.B. für die Methylcyclopentenylthio- oder die Methylcyclohexenylthio-Gruppe.

"(C₂-C₄)-Alkenyl-(C₃-C₈)-cycloalkylthio" steht z.B. für die Vinylcyclopentylthio-, Allylcyclopentylthio-, Vinylcyclohexylthio- oder die Allylcyclohexylthio-Gruppe.

"(C₂-C₆)-Alkinyl-(C₃-C₈)-cycloalkylthio" steht z.B. für die Ethinylcyclopentylthio-, Propargylcyclopentylthio-, Ethinylcyclohexylthio- oder die Propargylcyclohexylthio-Gruppe.

"(C₂-C₄)-Alkenyl-(C₄-C₈)-cycloalkenylthio" steht z.B. für die Allylcyclopentenylthio- oder die Allylcyclohexenylthio-Gruppe.

"(C₁-C₈)-Alkylsulfinyl" steht z.B. für die Methyl-, Ethyl-, Propyl-, Isopropyl-, Butyl-, Isobutyl-, sek.-Butyl-, tert.-Butyl- oder Octylsulfinyl-Gruppe.

"(C₃-C₈)-Alkenylsulfinyl" steht z.B. für die Allyl-, Methylallyl-, Butenyl- oder Octenylsulfinyl-Gruppe.

"(C₃-C₈)-Alkinylsulfinyl" steht z.B. für die Propargyl-, Butinyl- oder Octinylsulfinyl-Gruppe.

"(C₃-C₁₀)-Cycloalkylsulfinyl" steht für eine Cycloalkylsulfinylgruppe, deren Kohlenwasserstoffrest die unter dem Ausdruck "(C₃-C₁₀)-Cycloalkyl" angegebene Bedeutung hat.

"(C₄-C₁₀)-Cycloalkenylsulfinyl" steht für eine Cycloalkenylsulfinylgruppe, deren Kohlenwasserstoffrest die unter dem Ausdruck "(C₄-C₁₀)-Cycloalkenyl" angegebene Bedeutung hat.

"(C₃-C₈)-Cycloalkyl-(C₁-C₄)-alkylsulfinyl" steht z.B. für die Cyclopropylmethylsulfinyl-, Cyclopropylethylsulfinyl-, Cyclopentylmethylsulfinyl-, oder die Cyclohexylmethylsutfinyl-Gruppe.

"(C₄-C₈)-Cycloalkenyl-(C₁-C₄)-alkylsulfinyl" steht z.B. für die Cyclopentenylmethylsulfinyl- oder die Cyclohexenylmethylsulfinyl-Gruppe.

"(C₃-C₈)-Cycloalkyl-(C₃-C₄)-alkenylsulfinyl" steht z.B. für die Cyclopropylallylsulfinyl-, Cyclopentylallylsulfinyl- oder die Cyclohexylallylsulfinyl-Gruppe.

"(C₄-C₈)-Cycloalkenyl-(C₃-C₄)-alkenylsulfinyl" steht z.B. für die Cyclopentenylallylsulfinyl- oder die Cyclohexenylallylsulfinyl-Gruppe.

"(C₁-C₄)-Alkyl-(C₃-C₈)-cycloalkylsulfinyl" steht z.B. für die Methylcyclopentylsulfinyl- oder die Methylcyclohexylsulfinyl-Gruppe.

"(C₁-C₈)-Alkyl-(C₄-C₈)-cycloalkenylsulfinyl" steht z.B. für die Methylcyclopentenylsulfinyl- oder die Methylcyclohexenylsulfinyl-Gruppe.

"(C₂-C₄)-Alkenyl-(C₃-C₈)-cycloalkylsulfinyl" steht z.B. für die Vinylcyclopentylsulfinyl-, Allylcyclopentylsulfinyl-, Vinylcyclohexylsulfinyl- oder die Allylcyclohexylsulfinyl-Gruppe.

"(C₂-C₄)-Alkinyl-(C₃-C₈)-cycloalkylsulfinyl" steht z.B. für die Ethinylcyclopentylsulfinyl-, Propargylcyclopentylsulfinyl-, Ethinylcyclohexylsulfinyl- oder die Propargylcyclohexylsulfinyl-Gruppe.

"(C₂-C₄)-Alkenyl-(C₄-C₈)-cycloalkenylsulfinyl" steht z.B. für die Vinylcyclopentenylsulfinyl-, Allylcyclopentenylsulfinyl-, Vinylcyclohexenylsulfinyl- oder die Allylcyclohexenylsulfinyl-Gruppe.

"(C₁-C₁₀)-Alkylsulfonyl" steht z.B. für die Methyl-, Ethyl-, Propyl-, Isopropyl-, Butyl-, Isobutyl-, sek.-Butyl-, tert.-Butyl- oder Octylsulfonyl-Gruppe.

"(C₃-C₁₀)-Alkenylsulfonyl" steht z.B. für die Allyl-, Methylallyl-, Butenyl- oder Octenylsulfonyl-Gruppe.

"(C₃-C₁₀)-Alkinylsulfonyl" steht z.B. für die Propargyl-, Butinyl- oder Octinylsulfonyl-Gruppe.

"(C₃-C₁₀)-Cycloalkylsulfonyl" steht für eine Cycloalkylsulfonylgruppe, deren Kohlenwasserstoffrest die unter dem Ausdruck "(C₃-C₁₀)-Cycloalkyl" angegebene Bedeutung hat.

"(C₄-C₁₀)-Cycloalkenylsulfonyl" steht für eine Cycloalkenylsulfonylgruppe, deren Kohlenwasserstoffrest die unter dem Ausdruck "(C₄-C₁₀)-Cycloalkenyl" angegebene Bedeutung hat.

"(C₃-C₈)-Cycloalkyl-(C₁-C₄)-alkylsulfonyl" steht z.B. für die Cyclopropylmethylsulfonyl- , Cyclopropylethylsulfonyl-, Cyclopentylmethylsulfonyl- oder die Cyclohexylmethylsulfonyl-Gruppe.

"(C₄-C₈)-Cycloalkenyl-(C₁-C₄)-alkylsulfonyl" steht z.B. für die Cyclopentenylmethylsulfonyl- oder die Cyclohexenylmethylsulfonyl-Gruppe.

"(C₃-C₈)-Cycloalkyl-(C₃-C₄)-alkenylsulfonyl" steht z.B. für die Cyclopropylallylsulfonyl-Cyclopentylallylsulfonyl-, oder die Cyclohexylallylsulfonyl-Gruppe.

"(C₄-C₈)-Cycloalkenyl-(C₃-C₄)-alkenylsulfonyl" steht z.B. für die Cyclopentenylallylsulfonyl- oder die Cyclohexenylallylsulfonyl-Gruppe.

"(C₁-C₄)-Alkyl-(C₃-C₈)-cycloalkylsulfonyl" steht z.B. für die Methylcyclopentylsulfonyl- oder die Methylcyclohexylsulfonyl-Gruppe.

"(C₁-C₄)-Alkyl-(C₄-C₈)-cycloalkenylsulfonyl" steht z.B. für die Methycyclopentenylsulfonyl- oder die Methylcyclohexenylsulfonyl-Gruppe.

"(C₂-C₄)-Alkenyl-(C₃-C₈)-cycloalkylsulfonyl" steht z.B. für die Vinylcyclopentylsulfonyl- , Allylcyclopentylsulfonyl-, Vinylcyclohexylsulfonyl- oder die Allylcyclohexylsulfonyl-Gruppe.

"(C₂-C₄)-Alkinyl-(C₃-C₈)-cycloalkylsulfonyl" steht z.B. für die Ethinylcyclopentylsulfonyl-, Propargylcyclopentylsulfonyl-, Ethinylcyclohexylsulfonyl- oder die Propargylcyclohexylsulfonyl-Gruppe.

"(C₂-C₄)-Alkenyl-(C₄-C₈)-cycloalkenylsulfonyl" steht z.B. für die Vinylcyclopentenylsulfonyl-, Allylcyclopentenylsulfonyl-, Vinylcyclohexenylsulfonyl- oder die Allylcyclohexenylsulfonyl-Gruppe.

"(C₁-C₁₀)-Alkylamino" steht für ein Stickstoffatom, das durch ein oder zwei, gleiche oder verschiedene Alkylreste der obigen Definition substituiert ist.

"(C₃-C₁₀)-Alkenylamino" steht für ein Stickstoffatom, das durch ein oder zwei, gleiche oder verschiedene Alkenylreste der obigen Definition substituiert ist.

"(C₃-C₁₀)-Alkinylamino" steht für ein Stickstoffatom, das durch ein oder zwei, gleiche oder verschiedene Alkinylreste der obigen Definition substituiert ist.

"(C₃-C₈)-Cycloalkylamino" steht für ein Stickstoffatom, das durch ein oder zwei, gleiche oder verschiedene Cycloalkylreste der obigen Definition substituiert ist.

"(C₃-C₈)-Cycloalkyl-(C₁-C₄)-alkylamino" steht z.B. für die Cyclopropylmethylamino-, Cyclopropylethylamino-, Cyclopentylmethylamino- oder die Cyclohexylmethyamino-Gruppe.

"(C₄-C₈)-Cycloalkenyl-(C₁-C₄)-alkylamino" steht z.B. für die Cyclopentenylmethylamino- oder die Cyclohexenylmethylamino-Gruppe.

"(C₃-C₈)-Cycloalkyl-(C₃-C₄)-alkenylamino" steht z.B. für die Cyclopropylallylamino-Cyclopentylallylamino- oder die Cyclohexylallylamino-Gruppe.

"(C₄-C₈)-Cycloalkenyl-(C₃-C₄)-alkenylamino" steht z.B. für die Cyclopentenylallylamino- oder die Cyclohexenylallylamino-Gruppe.

"(C₁-C₄)-Alkyl-(C₃-C₈)-cycloalkylamino" steht z.B. für die Methylcyclopentylamino- oder die Methylcyclohexylamino-Gruppe.

"(C₁-C₈)-Alkyl-(C₄-C₈)-cycloalkenylamino" steht z.B. für die Methylcyclopentenylamino- oder die Methylcyclohexenylamino-Gruppe.

"(C₂-C₄)-Alkenyl-(C₃-C₈)-cycloalkylamino" steht z.B. für die Vinylcyclopentylamino-, Allylcyclopentylamino-, Vinylcyclohexylamino- oder die Allylcyclohexylamino-Gruppe.

"(C₂-C₄)-Alkinyl-(C₃-C₈)-cycloalkylamino" steht z.B. für die Ethinylcyclopentylamino-, Propargylcyclopentylamino-, Ethinylcyclohexylamino- oder die Propargylcyclohexylamino-Gruppe.

"(C₂-C₄)-Alkenyl-(C₄-C₈)-cycloalkenylamino" steht z.B. für die Vinylcyclopentenylamino-, Allylcyclopentenylamino-, Vinylcyclohexenylamino- oder die Allylcyclohexenylamino-Gruppe.

Neben einfach N-substituierten Aminogruppen sind auch die entsprechenden N,N-disubstituierten Derivate umfasst, insbesondere Derivate, bei denen ein Wasserstoff durch eine (C₁-C₄)-Alkylgruppe ersetzt ist.

Der Ausdruck "(C₁-C₁₀)-Trialkylsilyl" bedeutet ein Siliziumatom, das drei gleiche oder verschiedene Alkylreste gemäß der obigen Definition trägt. Analog stehen "Aryl-(C₁-C₆)-Dialkylsilyl" für ein Siliziumatom, das einen Arylrest und zwei gleiche oder verschiedene Alkylreste gemäß der obigen Definition trägt, "Diaryl-(C₁-C₆)-Alkylsilyl" für ein Siliziumatom, das einen Alkylrest und zwei gleiche oder verschiedene Arylreste gemäß der obigen Definition trägt, und "Triarylsilyl" für ein Siliziumatom, das drei gleiche oder verschiedene Arylreste gemäß der obigen Definition trägt.

Unter dem Ausdruck "Aryl" ist ein carbocyclischer, d.h. aus Kohlenstoffatomen aufgebauter, aromatischer Rest mit vorzugsweise 6 bis 14, insbesondere 6 bis 12 C-Atomen, wie beispielsweise Phenyl, Naphthyl oder Biphenylyl, vorzugsweise Phenyl, zu verstehen.

"Aroyl" bedeutet demnach einen wie vorstehend definierter Arylrest, der über eine Carbonyl-Gruppe gebunden ist, wie z.B. die Benzoyl-Gruppe.

Der Ausdruck "vier- bis achtgliedriger nicht-aromatischer Heterocyclus enthaltend mindestens ein Sauerstoff-, Schwefel-, Stickstoff- und/oder Siliziumringatom" steht für einen cyclischen Rest, der vollständig gesättigt oder teilweise ungesättigt, nicht aber aromatisch sein kann und der durch ein oder mehrere, gleiche oder verschiedene, vorzugsweise ein oder zwei, Atome aus der Gruppe Stickstoff, Schwefel, Sauerstoff und Silizium unterbrochen ist, wobei jedoch nicht zwei Sauerstoffatome direkt benachbart sein dürfen. Der Heterocyclus weist vier bis acht Ringatome auf, welche neben den oben genannten Heteroatomen Kohlenstoffatome sind.

Der Ausdruck "Heterocyclyl" steht vorzugsweise für einen cyclischen Rest, der vollständig gesättigt, teilweise ungesättigt oder vollständig ungesättigt sein kann und der durch mindestens ein oder mehrere gleiche oder verschiedene, vorzugsweise ein bis drei Atome aus der Gruppe Stickstoff, Schwefel oder Sauerstoff unterbrochen sein kann, wobei jedoch nicht zwei Sauerstoffatome direkt benachbart sein dürfen und noch mindestens ein Kohlenstoffatom im Ring vorhanden sein muß, wie z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, 4H-Chinolizin, Piperidin, Pyrrolidin, Oxazolin, Tetrahydrofuran, Tetrahydropyran, Isoxazolidin oder Thiazolidin. Der Ausdruck "Heteroaromat" umfaßt demnach von den vorstehend unter "Heterocyclyl" genannten Bedeutungen jeweils die vollständig ungesättigten aromatischen heterocyclischen Verbindungen.

Heterocyclyl bedeutet besonders bevorzugt ein gesättigtes, teilgesättigtes oder aromatisches Ringsystem mit 3 bis 6 Ringgliedern und 1 bis 4 Heteroatomen aus der Gruppe O, S und N, wobei mindestens ein Kohlenstoffatom im Ring vorhanden sein muß.

Ganz besonders bevorzugt bedeutet Heterocyclyl ein Radikal des Pyridin, Pyrimidin, (1,2,4)-Oxadiazol, (1,3,4)-Oxadiazol, Pyrrol, Furan, Thiophen, Oxazol, Thiazol, Imidazol, Pyrazol, Isoxazol, 1,2,4-Triazol, Tetrazol, Pyrazin, Pyridazin, Oxazolin, Thiazolin, Tetrahydrofuran, Tetrahydropyran, Morpholin, Piperidin, Piperazin, Pyrrolin, Pyrrolidin, Oxazolidin, Thiazolidin, Oxiran und Oxetan.

"Aryloxy" bedeutet demnach einen wie vorstehend definierter Arylrest, der über ein Sauerstoffatom gebunden ist, wie z.B. die Phenoxy- oder Naphthyloxy-Gruppe.

"Arylthio" bedeutet einen über ein Schwefelatom verknüpften Arylrest, z.B. den Phenylthio- oder den 1- oder 2-Naphthylthio-Rest.

"Arylamino" bedeutet einen über ein Stickstoffatoatom verknüpften Arylrest, z.B. den Anilino- oder den 1- oder 2-Naphthamino-Rest.

"N-(C₁-C₄)-Alkyl-arylamino" bedeutet z.B. den N-Methyl- oder N-Ethyl-anilino-Rest.

"Aryl-(C₁-C₄)-alkoxy" steht für einen über eine (C₁-C₄)-Alkoxygruppe verknüpften Arylrest, z.B. den Benzyloxy-, Phenylethoxy-, Phenylbutoxy- oder Naphthylmethoxy-Rest.

"Aryl-(C₃ C₄)-alkenyloxy" steht für einen über eine (C₃-C₄)-Alkenyloxygruppe verknüpften Arylrest, z.B. den 1-, 2-, oder 3-Phenylallyloxy-Rest.

"Aryl-(C₁-C₄)-alkylthio" steht für einen Arylrest, der über einen Alkylthiorest verknüpft ist, z.B. der Benzylthio-, Naphthylmethylthio- oder den 1- oder 2-Phenylethylthio-Rest.

"Aryl-(C₃-C₄)-alkenylthio" steht für einen über eine (C₃-C₄)-Alkenylthiogruppe verknüpften Arylrest, z.B. den 1,- 2- oder 3-Phenylallylthio-Rest.

"Aryl-(C₃-C₄)-alkenylamino" steht für einen über eine (C₃-C₄)-Alkenylaminogruppe verknüpften Arylrest, z.B. den 1-, 2- oder 3-Phenylallylamino-Rest.

"Aryl-(C₁-C₈)-dialkylsilyl" steht z. B. für eine Phenyl- oder Naphthyl-dimethylsilyl-Gruppe.

"Diaryl-(C₃-C₄)-alkylsilyl" steht z. B. für eine Diphenyl-, Phenyl-naphthyl-, oder Dinaphthyl-methylsilyl-Gruppe.

"Triarylsilyl" steht z. B. für eine Triphenyl-, Diphenyl-naphthyl- oder Trinaphthylsilyl-Gruppe.

Weiterhin werden unter dem Ausdruck daß "gegebenenfalls in allen zu R⁵, R⁶, R⁷, R⁸, R⁹, R¹⁰ und R¹¹ genannten Gruppen an Kohlenstoff gebundener Wasserstoff durch bis zu drei Halogenatome, im Falle von Fluor auch bis zu Maximalanzahl, ersetzt sein kann" z. B. folgende Reste erfaßt:
Haloalkyl, wie z.B. die 1- oder 2-Fluorethyl-, die Trifluormethyl-, die 2,2,2-Trifluorethyl-, die Chlormethyl-, Fluormethyl-, die Difluormethyl-oder die 1,1,2,2-Tetrafluorethylgruppe;
Haloalkenyl, wie z.B. die 1-, 2- oder 3-Fluorallyl-Gruppe oder die 1,1-Difluorpropen-3-yl-Gruppe;
Haloalkoxy, wie z. B. Trifluormethoxy oder 2,2,2-Trifluorethoxy;
Haloalkylthio, wie z.B. Trifluormethylthio ;
Haloalkylsulfinyl, wie z.B. Trifluormethylsulfinyl ;
Haloalkylsulfonyl, wie z.B. Trifluormethylsulfonyl;
Halocyclopropyl, wie z. B. 1,1-Difluorcycloprop-2-yl.

Je nach Art der oben definierten Substituenten weisen die Verbindungen der allgemeinen Formel (1) saure oder basische Eigenschaften auf und können Salze bilden. Tragen die Verbindungen der allgemeinen Formel (I) beispielsweise Gruppen wie Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der allgemeinen Formel (I) beispielsweise Gruppen wie Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide, akarizide und mitizide Eigenschaften auf.

Die Verbindungen der allgemeinen Formel (I) können ein oder mehrere asymmetrische Kohlenstoffatome oder Stereoisomere an Doppelbindungen aufweisen. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Isomeren aufgetrennt werden. Racemate können nach üblichen Methoden in die Enantiomeren aufgetrennt werden.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die vorliegende Erfindung betrifft auch Verfahren zur Herstellung von Verbindungen der allgemeinen. Formel (I).

Zur Herstellung von Verbindungen der allgemeinen Formel (I), in welchen R¹, R⁴, R⁵, A, Y und n die zur Formel (I) angegebene Bedeutungen haben, wird beispielsweise so verfahren, daß man eine Carbonsäure der Formel (III), 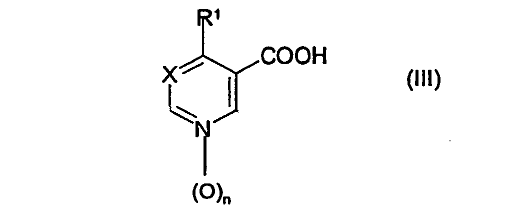 worin X, R¹, und n die oben zur Formel (I) angegebenen Bedeutungen haben, in Form eines aktivierten Derivats dieser Säure, beispielsweise eines Säurehalogenids, Esters oder Anhydrids, in Gegenwart einer Base, beispielsweise mindestens eines Amins, wie Triethylamin, Diisopropylethylamin, Pyridin oder Lutidin, Alkalimetallcarbonats, Alkalimetallhydrogencarbonats oder Alkalimetallhydroxids oder von Kombinationen dieser Verbindungen, mit einer Verbindung der Formel (IV), worin R⁴ und R⁵ die oben zur Formel (I) angegebenen Bedeutungen haben,

HNR⁴R⁵ (IV)

umsetzt und gegebenenfalls anschließend den Rest R⁵ weiter derivatisiert.

Kollektionen aus Verbindungen der Formel (I), die nach oben genannten Schema synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe Road, Tollesbury, Essex, CM9 8SE, England oder H+P Labortechnik GmbH, Bruckmannring 28, 85764 Oberschleißheim, Deutschland oder der Firma Radleys, Shirehill, Saffron Walden, Essex, England, angeboten werden. Für die parallelisierte Aufreinigung von Verbindungen der allgemeinen Formel (1) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen kann die Herstellung von Verbindungen der allgemeinen Formel (I) vollständig oder partiell durch Festphasen-unterstützte-Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen-unterstützte-Synthesemethoden sind in der Fachliteratur hinreichend beschrieben, z.B. Barry A. Bunin in "The Combinatorial Index", Verlag Academic Press, 1998.

Die Verwendung von Festphasen-unterstützten-Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen, die Bibliotheken genannt werden.

Gegenstand der vorliegenden Erfindung sind auch Bibliotheken, die mindestens zwei Verbindungen der Formel (I) enthalten.

Die Verbindungen der Formel (I) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp. Aus der Ordnung der Isopoda z.B. Oniscus aselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung des Isoptera z.B. Reticulitermes spp..
Aus der Ordnung der Anoplura z.B. Phylloera vastatrix, Pemphigus spp., Pediculus humanus corporis, Haematopinus spp., Linognathus spp..
Aus der Ordnung der Mallophaga z.B. Trichodectes pp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Äcanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthonynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopsis, Ceratophyllus spp..
Aus der Ordnung der Arachnida z.B. Scorpio maurus, Latrodectus mactans.
Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.
Aus der Klasse der Gastropoda z.B. Deroceras spp., Arion spp., Lymnaea spp., Galba spp., Succinea spp., Biomphalaria spp., Bulinus spp., Oncomelania spp..
Aus der Klasse der Bivalva z.B. Dreissena spp..

Weiterhin lassen sich Protozoen, wie Eimeria, bekämpfen. Zu den pflanzenparasitären Nematoden, die erfindungsgemäß bekämpft werden können, gehören beispielsweise die wurzelparasitären Bodennematoden wie z.B. solche der Gattungen Meloidogyne (Wurzelgallennematoden, wie Meloidogyne incognita, Meloidogyne hapla und Meloidogyne javanica), Heterodera und Globodera (zystenbildende Nematoden, wie Globodera rostochiensis, Globodera pallida, Heterodera trifolii) sowie der Gattungen Radopholus wie Radopholus similis, Pratylenchus wie Pratyglenchus neglectus, Pratylenchus penetrans und Pratylenchus curvitatus;
Tylenchulus wie Tylenchulus semipenetrans, Tylenchorhynchus, wie Tylenchorhynchus dubius und Tylenchorhynchus claytoni, Rotylenchus wie Rotylenchus robustus, Heliocotylenchus wie Haliocotylenchus multicinctus, Belonoaimus wie Belonoaimus longicaudatus, Longidorus wie Longidorus elongatus, Trichodorus wie Trichodorus primitivus und Xiphinema wie Xiphinema index.

Ferner lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, beispielsweise Pflanzenschutzmittel, vorzugsweise insektizide, akarizide, ixodizide, nematizide, molluskizide oder fungizide, besonders bevorzugt insektizide und akarizide Mittel, die eine oder mehrere Verbindungen der Formel (I) neben geeigneten Formulierungshilfsmitteln enthalten.

Zur Herstellung der erfindungsgemäßen Mittel gibt man den Wirkstoff und die weiteren Zusätze zusammen und bringt sie in eine geeignete Anwendungsform.

Die Erfindung betrifft auch Mittel, insbesondere insektizide und akarizide Mittel, die die Verbindungen der Formel (I) neben geeigneten Formulierungshilfsmitteln enthalten.

Die erfindungsgemäßen Mittel enthalten die Wirkstoffe der Formeln (I) im allgemeinen zu 1 bis 95 Gew.-%. Sie können auf verschiedene Art formuliert werden, je nachdem wie es durch die biologischen und/oder chemischphysikalischen Parameter vorgegeben ist. Als Formulierungsmöglichkeiten kommen daher beispielsweise in Frage:

Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in: Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, d.h. Träger- und/oder oberflächenaktive Stoffe, wie Inertmaterialien, Tenside, Lösungsmittel und weitere Zusatzstoffe sind ebenfalls bekannt und beispielsweise beschrieben in: Watkins, "Handbook of Insecticide Dust Diluents and Carriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Cadodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise gewünschtenfalls in Mischung mit Düngemitteln - granuliert werden.

In Spritzpulvern beträgt die Wirkstoffkonzentration üblicherweise etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kg/ha oder mehr Aktivsubstanz, vorzugsweise liegt sie jedoch zwischen 0,001 und 5 kg/ha Wirkstoff.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:

### 1. aus der Gruppe der Phosphorverbindungen

Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, Isazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;

### 2. aus der Gruppe der Carbamate

Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;

### 3. aus der Gruppe der Carbonsäureester

Acrinathrin, Allethrin, Alphametrin, 5-Benzyl-3-furylmethyl-(E)-(1 R)-cis-2,2-dimethyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1RS)-trans-3-(4-tert.butylphenyl)-2,2-dimethylcyclopropanecarboxylate (NCl 85193), Cycloprothrin, Cyfluthrin, Cyhalothrin, Cythithrin, Cypermethrin, byphenothrin, Deltamethrin, Empenthrin, Esfenvalerate, Fenfluthrin, Fenpropathrin, Fenvalerate, Flubrocythrinate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);

### 4. aus der Gruppe der Amidine

Amitraz, Chlordimeform;

### 5. aus der Gruppe der Zinnverbindungen

Cyhexatin, Fenbutatinoxide;

### 6. Sonstige

Abamectin, ABG-9008, Acequinocyl, Azadirachtin, Acetamiprid, Anagrapha falcitera, AKD-1022, AKD-3059, AKD-3088, AL-9811, ANS-118, Bacillus thuringiensis, Beauveria bassianea, Bensultap, Bifenazate (D-2341), Binapacryl, BJL-932, Bromopropylate, BAJ-2740 (Spirodiclofen), BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chloproxyfen, Clothianidine, Chromafenozide (ANS-118), A-184699, Cyclopropancarbonsäure-(2-naphthylmethyl)ester (Ro12-0470), Cyromazin, CM-002X, DBI-3204, Diacloden (Thiamethoxam), Diafenthiuron, N-(3,5-Dichlor-4-(1,1,2,3,3,3-hexafluor-1-propyloxy)phenyl)-carbamoyl)-2-chlorbenzcarboximid-säureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granulose- und Kernpolyederviren, Fenpyroximate, Fenthiocarb, Fluacrypyrim, Flufenzine, Flubenzimine, Flucycloxuron, Flufenoxuron, Flufenprox (ICI-A5683), Fluproxyfen, FMC-F6028, Gamma-HCH, Halofenozide (RH-0345), Halofenprox (MTI-732), Hexaflumuron (DE_473), Hexythiazox, HOI-9004, Hydramethylnon (AC 217300), Lufenuron, Imidacloprid, Indoxacarb (DPX-MP062), Kanemite (AKD-2023), M-020, MTI-446, Ivermectin, M-020, IKA-2000, IKI-220, MKI-245, Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), NC-196, NNI-0001, Nidintefuran, Pyriproxyfen (S-71639), Pirydaryl, Protrifenbute, Pyriproxy-fen, NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601, Silafluofen, Silomadine (CG-177), Spinosad, SU-9118, Tebufenozide, Tebufenpyrad (MK-239), Teflubenzuron, Tetradifon, Tetrasul, Thiacloprid, Thiocyclam, Thiamethoxam, TI-435, Tolfenpyrad (OMI-88), Triazamate (RH-7988), Triflumuron, Verbutin, Vertalec (Mykotal), YI-5301 und Yi-6101.

Die oben genannten Kombinationspartner stellen bekannte Wirkstoffe dar, die zum großen Teil in Ch.R Worthing, S.B. Walker, The Pesticide Manual, 12. Auflage, British Crop Protection Council Famham, 2000 beschrieben sind.

Als Fungizide, die mit den erfindungsgemäßen Verbindungen der Formel (I) kombiniert werden können, sind z.B. folgende Produkte zu nennen:

Aldimorph, Andoprim, Anilazine, BAS 480F, BAS 450F, Benalaxyl, Benodanil, Benomyl, Binapacryl, Bitertanol, Bromuconazol, Buthiobate, Captafol, Captan, Carbendazim, Carboxin, CGA 173506, Cyprofurarn, Dichlofluanid, Dichlomezin, Diclobutrazol, Diethofencarb, Difenconazol (CGA 169374), Difluconazole, Dimethirimol, Dimethomorph, Diniconazole, Dinocap, Dithianon, Dodemorph, Dodine, Edifenfos, Ethirimol, Etridiazot, Fenarimol, Fenfuram, Fenpiclonil, Fenpropidin, Fenpropimorph, Fentinacetate, Fentinhydroxide, Ferimzone (TF164), Fluazinam, Fluobenzimine, Fluquinconazole, Fluorimide, Flusilazole, Flutolanil, Fluthafol, Folpet, Fosetylaluminium,Fuberidazole, Fulsulfamide (MT.-F 651), Furalaxyl, Furconazol, Furmecyclox, Guazatine, Hexaconazole, ICI A5504, Imazalil, Imibenconazole, Iprobenfos, Iprodione, Isoprothiolane, KNF 317, Kupferverbindungen wie Cu-oxychlorid, Oxine-Cu, Cu-oxide, Mancozeb, Maneb, Mepanipyrim (KIF 3535), Metconazol, Mepronil, Metalaxyl, Methasulfocarb, Methfuroxam, MON 24000, Myclobutanil, Nabam, Nitrothalido-propyl, Nuarimol, Ofurace, Oxadixyl, Oxycarboxin, Penconazol, Pencycuron, PP 969, Probenazole, Propineb, Prochloraz, Procymidon, Propamocarb, Propiconazol, Prothiocarb, Pyracarbolid, Pyrazophos, Pyrifenox, Pyroquilon, Rabenzazole, RH7592, Schwefel, Tebuconazole, TF 167, Thiabendazole, Thicyofen, Thiofanate-methyl, Thiram, Tolclofos-methyl, Tolylfluanid, Triadimefon, Triadimenol, Tricyclazole, Tridemorph, Triflumizol, Triforine, Validamycin, Vinchlozolin, XRD 563, Zineb, Natriumdodecyl-sulfonat, Natriumdodecyl-sulfat, Natriüm-C13/C15-atkoholethersulfonat, Natrium-cetostearylphosphatester, Dioctyl-natrium-sulfosuccinat, Natrium-isopropyl-naphthalenesulfonat, Natrium-methylenebisnaphthalene-sulfonat, Cetyl-trimethyl-ammoniumchlorid, Salze von langkettigen primären, sekundären oder tertiären Aminen, Alkylpropyleneamine, Lauryl-pyrimidiniumbromid, ethoxylierte quarternierte Fettamine, Alkyl-dimethyl-benzyl-ammoniumchlorid und 1-Hydroxyethyl-2-alkyl-imidazolin.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Gegenstand der Erfindung ist daher die Verwendung von Verbindungen der Formel (I) und deren Salzen zur Bekämpfung von Schadorganismen.

Weiterhin Gegenstand der Erfindung ist ein Verfahren zur Bekämpfung von schädlichen Insekten, Spinnentieren und/oder Nematoden, wobei man eine wirksame Menge einer Verbindung der Formel (I) oder ihrer Salze auf die Schädlinge oder den Ort der gewünschten Wirkung appliziert.

Die erfindungsgemäßen Wirkstoffe eignen sich auch zur Bekämpfung von Endo- und Ektoparasiten auf dem human- und veterinärmedizinischen Gebiet bzw. auf dem Gebiet der Tierhaltung. Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Gegenstand der Erfindung ist daher auch die Verwendung einer Verbindung der Formel (I) oder eines ihrer Salze zur Herstellung eines Human- und/oder Tierarzneimittels, vorzugsweise eines Tierarzneimittels, insbesondere zur Kontrolle von Ekto- und/oder Endoparasiten.

Die erfindungsgemäßen Verbindungen der Formel (I) können demgemäß auch besonders vorteilhaft zur Behandlung von Warmblütern, insbesondere in der Viehhaltung (z.B. Rinder, Schafe, Schweine und Geflügel wie Hühner, Gänse usw.) eingesetzt werden. In einer bevorzugten Ausführungsform der Erfindung werden den Tieren die Verbindungen, gegebenenfalls in geeigneten Formulierungen und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Neben lethaler Wirkung auf Schädlinge zeichnen sich die Verbindungen der Formel (1) oder deren Salze auch durch einen ausgeprägten Repellenteffekt aus.

Repellent im Sinne der Beschreibung ist ein Stoff oder Stoffgemisch, das abwehrend oder vertreibend auf andere Lebewesen, insbesondere Schädlinge und Lästlinge wirkt. Der Begriff umfaßt dabei auch Effekte wie den Antifeeding-Effekt, wobei die Nahrungsaufnahme gestört oder verhindert wird (fraßabweisender Effekt), Unterdrückung der Eiablage oder eine Beeinflussung der Populationsentwicklung.

Gegenstand der Erfindung ist daher auch die Verwendung von Verbindungen der Formel (I) oder deren Salze zur Erzielung der genannten Effekte, insbesondere bei den in den biologischen Beispielen benannten Schädlingen.

Gegenstand der Erfindung ist auch ein Verfahren zur Abwehr oder zur Vertreibung von Schadorganismen, wobei man eine oder mehrere Verbindungen der Formel (I) oder deren Salze an dem Ort ausbringt, von dem die Schadorganismen ferngehalten oder vertrieben werden sollen.

Ausbringen kann im Falle einer Pflanze beispielsweise eine Behandlung der Pflanze oder auch des Saatguts bedeuten.

Es ist, was die Beeinflussung von Populationen angeht, von Interesse, dass die Effekte auch hintereinander bei der Entwicklung einer Population beobachtet werden, wobei sie sich aufaddieren können. Hierbei kann der Einzeleffekt selbst nur einen Wirkungsgrad von deutlich unter 100% haben und insgesamt am Ende doch eine 100%ige Wirkung erreicht werden.

Außerdem zeichnen sich die Verbindungen der Formel (I) oder ihre Salze dadurch aus, dass man - will man die oben angeführten Effekte ausnutzen - zu einem früheren Zeitpunkt als bei einer direkten Bekämpfung üblich das Mittel appliziert. Der Effekt hält häufig lange Zeit an, so dass eine Wirkungsdauer von mehr als 2 Monaten erreicht wird.

Die Effekte treten bei Insekten, Spinnentieren und den anderen der oben genannten Schädlinge auf.

Neben den bisher genannten Applikationsverfahren zeigen die erfindungsgemäßen Wirkstoffe der Formel (I) eine hervorragende systemische Wirkung. Die Wirkstoffe können daher auch über Pflanzenteile, unterirdische wie oberirdische (Wurzel, Stengel, Blatt), in die Pflanzen eingebracht werden, wenn die Wirkstoffe in flüssiger oder fester Form in die direkte Umgebung der Pflanze appliziert werden (z.B. Granulate in der Erdapplikation, Applikation in gefluteten Reisfeldem).

Daneben sind die erfindungsgemäßen Wirkstoffe in besonderer Weise zu Behandlung von vegetativem und generativem Vermehrungsmaterial einsetzbar, wie z.B. von Saatgut von beispielsweise Getreide, Gemüse, Baumwolle, Reis, Zuckerrübe und anderen Kultur- und Zierpflanzen, von Zwiebeln, Stecklingen und Knollen weiterer vegetativ vermehrter Kultur- und Zierpflanzen. Die Behandlung hierfür kann vor der Saat bzw. dem Pflanzvorgang erfolgen (z.B. durch spezielle Techniken des Seedcoatings, durch Beizung in flüssiger oder fester Form oder Seedboxtreatment), während des Saatvorgangs bzw. des Pflanzens oder nach dem Saat- bzw. Pflanzvorgang durch spezielle Applikationstechniken (z.B. Saatreihenbehandlung). Die angewandte Wirkstoffmenge kann entsprechend der Anwendung in einem größerem Bereich schwanken. Im allgemeinen liegen die Aufwandmengen zwischen 1 g und 10 kg Wirkstoff pro Hektar Bodenfläche.

Die Verbindungen der Formel (I) können auch zur Bekämpfung von tierischen Schädlingen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten, wie bestimmten Insekten oder Mikroorganismen, wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung in wirtschaftlich bedeutenden transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten.

Bei der Anwendung in transgenen Kulturen, insbesondere mit Insektenresistenzen treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Schädlingsspektrum, das bekämpft werden kann oder veränderte Aufwandmengen, die für die Applikation eingesetzt werden können.

Gegenstand der Erfindung ist deshalb auch die Verwendung von Verbindungen der Formel (I) zur Bekämpfung von Schadorganismen in transgenen Kulturpflanzen.

Die Anwendung der erfindungsgemäßen Verbindungen beinhaltet neben direkter Applikation auf die Schädlinge jede andere Applikation, bei der Verbindungen der Formel (I) auf die Schädlinge wirken. Solche indirekten Applikationen können beispielsweise in der Anwendung von Verbindungen liegen, die, beispielsweise im Boden, der Pflanze oder dem Schädling, zu Verbindungen der Formel (I) zerfallen oder abgebaut werden.

Auf den Inhalt der deutschen Patentanmeldung 10148290.6, deren Priorität die vorliegende Anmeldung beansprucht, sowie der beiliegenden Zusammenfassung wird ausdrücklich Bezug genommen; er gilt durch das Zitat als Bestandteil dieser Beschreibung.

Nachfolgende Beispiele dienen zur Erläuterung der Erfindung, ohne sie damit einzuschränken.

### A. Chemische Beispiele

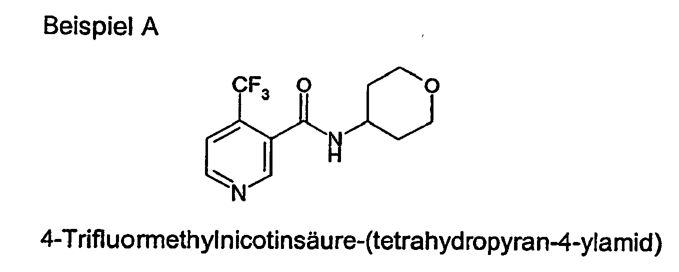

Zu einer Lösung von 0,30g (3,0mmol) 4-Amino-tetrahydropyran (hergestellt durch reduktive Aminierung von Tetrahydro-pyran-4-on; NH₃/H₂/Ni, 100bar, 50°C) und 0,35g (3.5mmol) Triethylamin in 25ml Dichlormethan wurde bei 0°C eine Lösung von 0,63g (3,0mmol) 4-Trifluornicotinsäurechlorid in wenig Dichlormethan zugetropft. Man rührte noch zwei Stunden bei Raumtemperatur, versetzte mit gesättigter Kochsalzlösung und trennte die Phasen. Die wäßrige Phase wurde noch zweimal mit Dichlormethan ausgerührt. Die vereinigten organischen Phasen wurden getrocknet und eingeengt. Man erhielt 0,46g (67,8% d. Th.) Produkt als farblosen Feststoff vom Schmelzpunkt 149 -150°C.

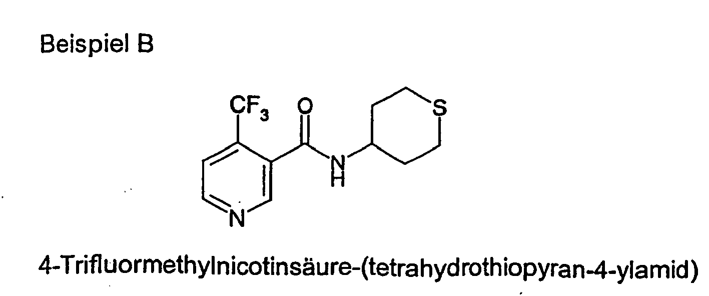

Analog Beispiel A wurden äquimolare Mengen 4-Amino-tetrahydro-thiopyran (hergestellt ausgehend von Tetrahydro-thiopyran-4-on über das entsprechende Oxim und anschließende Lithiumalanat-Reduktion in Tetrahydrofuran) und 4-Trifluormethyl-nicotinsäurechlorid in Gegenwart von Triethylamin umgesetzt. Man erhielt das Amid nach Chromatographie an Kieselgel (Ethylacetat) als farblosen Feststoff vom Schmelzpukt 161-163° C. (Ausbeute 51,3% d. Th.)

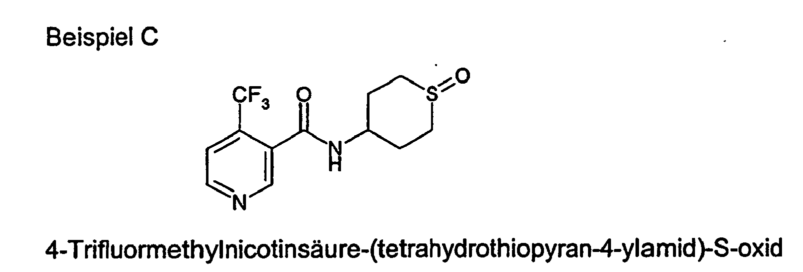

Zu einer Lösung von 1,45g (5mmol) 4-Trifluormethylnicotinsäure-(tetrahydrothiopyran-4-ylamid) (Beispiel B) in 50ml Dichlormethan tropfte man bei 0° C eine Lösung von 1,23g (5mmol) 70%ige 3-Chlorperbenzoesäure in 25ml Dichlormethan. Man rührte 6 Stunden bei Raumtemperatur, rührte mit Sodalösung aus und trocknete die organische Phase. Nach Einengen verblieb ein farbloses Öl, das nach Verreiben mit Ethylacetat kristallisierte. Nach dem Absaugen erhielt man 1,0g (65,3% d. Th.) Produkt vom Schmelzpunkt 243 - 244° C

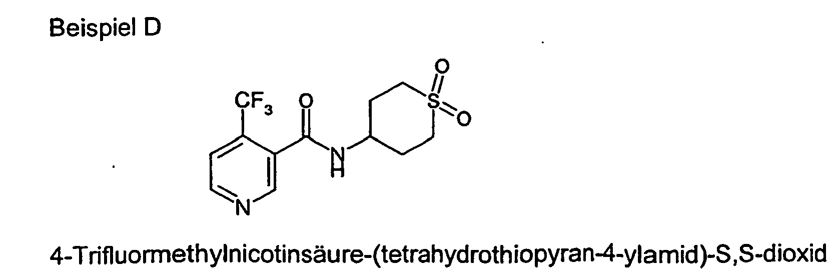

Zu einer Lösung von 1,45g (5mmol) 4-Trifluormethylnicotinsäure-(tetrahydrothiopyran-4-ylamid) (Beispiel B) in 50ml Dichlormethan tropfte man bei 0° C eine Lösung von 2,46g (10mmol) 70%iger 3-Chlorperbenzoesäure in 50ml Dichlormethan. Man rührte 6 Stunden bei Raumtemperatur, rührte mit Sodalösung aus und trocknete die organische Phase. Nach Einengen verblieb ein farbloses Öl, das nach Verreiben mit Ethylacetat kristallisierte. Nach dem Absaugen erhielt man 1,3g (80,7% d. Th.) Produkt vom Schmelzpunkt 248° C (Zersetzung).

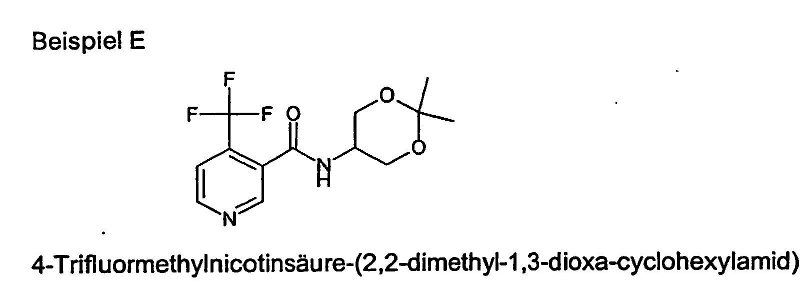

2,64g (10mmol) N-(4-Trifluormethylnicotinoyl)-serinol und 4,20g (40mmol) Acetondimethylacetal wurden in 50ml Toluol in Gegenwart von 0,50g p-Toluolsulfonsäurehydrat 12 Stunden bei 80° C gerührt, wobei entstehendes Methanol abdestilliert wurde. Nach Abkühlen wurde mit verdünnter Natronlauge und Wasser ausgerührt und die organische Phase getrocknet und eingeengt. Man erhielt 1,5g Produkt (49,3% d. Th.) als farblosen Feststoff vom Schmelzpunkt 155-156° C.

Herstellung des Ausgangsprodukts N-(4-Trifluormethylnicotinoyl)-serinol 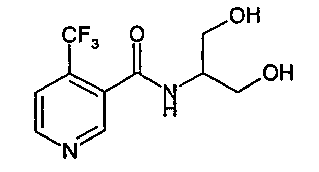 3,9g (20mmol) 4-Trifluormethylnicotinsäure und 3,2g (20mmol) Carbonyldiimidazol wurden in 100ml trockenem Tetrahydrofuran bei 40 - 50° C 6 Stunden gerührt. Nach Abkühlen gab man 1,8g (20mmol) Serinol zu und rührte weitere 4 Stunden bei 50 - 60° C. Das nach Abziehen des Lösemittels erhaltene Rohprodukt wurde durch Chromatographie an Kieselgel gereinigt. Man erhielt 2,9g Produkt (55,3% d.Th) vom Schmelzpunkt 155-156° C.

In analoger Weise können die in den nachfolgenden Tabellen aufgeführten Verbindungen hergestellt werden.

**Tabelle 1**

| | | |
|---|---|---|
| 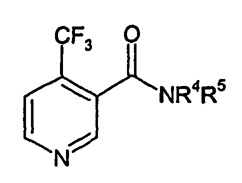 | | |

| Bsp.-Nr. | NR⁴R⁵ | Smp. [°C] |
|---|---|---|
| 1 | 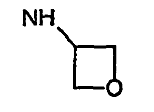 | |
| 2 | 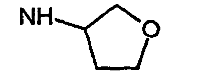 | |
| 3 | 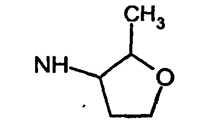 | Öl |
| 4 | 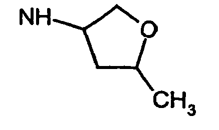 | |
| 4 | 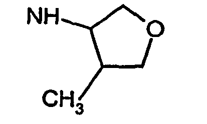 | |
| 5 | 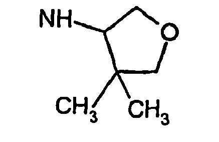 | |
| 6 | 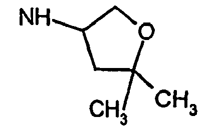 | |
| 7 | 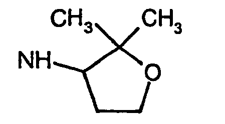 | |
| 8 | 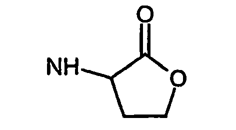 | 121-122 |
| 9 | 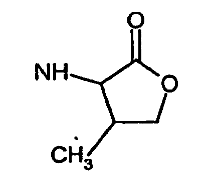 | |
| 9 | 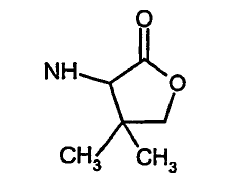 | 168-171 |
| 10 | 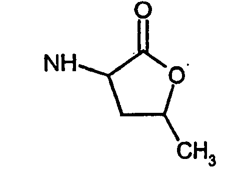 | 119 -121 |
| 11 | 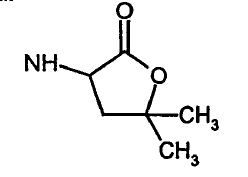 | 140 - 142 |
| 12 | 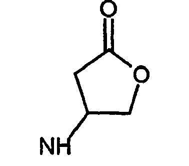 | 83-84 |
| 13 | 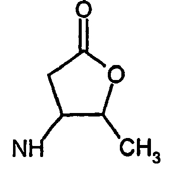 | |
| 14 | 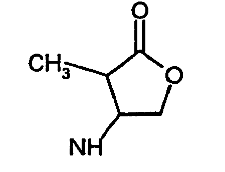 | |
| 15 | 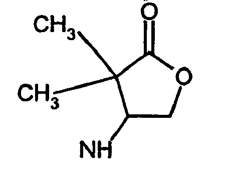 | |
| 16 | 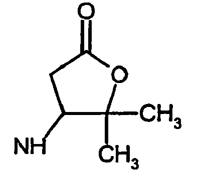 | |
| 17 | 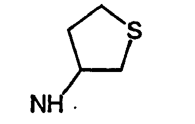 | 78-80 |
| 18 | 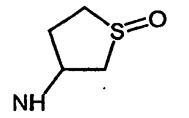 | 153-154 |
| 19 | 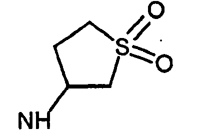 | 168-169 |
| 20 | 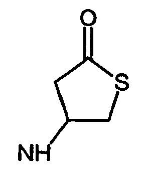 | |
| 21 | 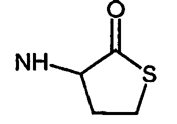 | 151-153 |
| 22 | 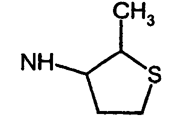 | 112-116 (Isomerengem.) |
| 23 | 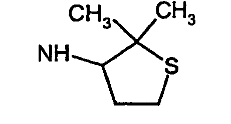 | Öl |
| 24 | 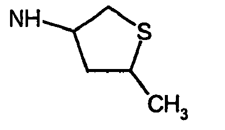 | 118-121 |
| 25 | 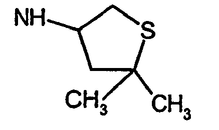 | Öl |
| 26 | 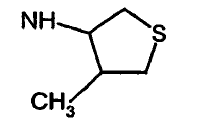 | cis-Isomer. Öl trans-Isomer: Öl |
| 27 | 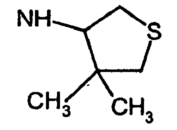 | |
| 28 | 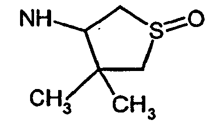 | |
| 29 | 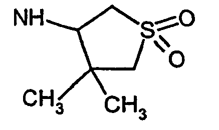 | |
| 30 | 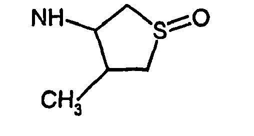 | cis-Amino-methyl: Öl trans-Amino-methyl: 105-108 |
| 3.1 | 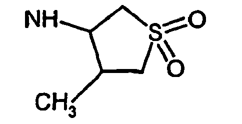 | |
| 32 | 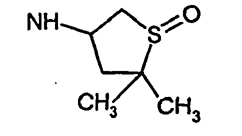 | |
| 33 | 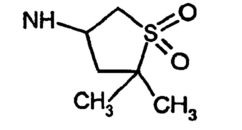 | |
| 34 | 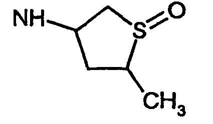 | |
| 35 | 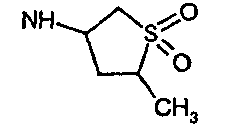 | 150-152 |
| 36 | 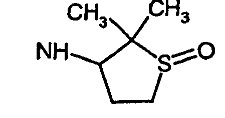 | |
| 37 | 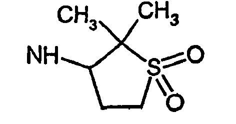 | |
| 38 | 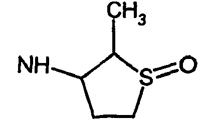 | Isomerengemisch, Öl |
| 39 | 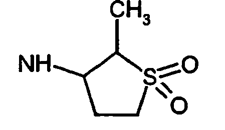 | Isomerengemisch, Öl |
| 40 | 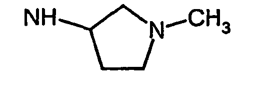 | |
| 41 | 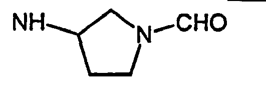 | |
| 42 | 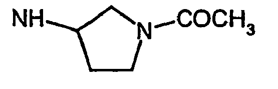 | |
| 43 | 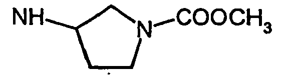 | |
| 44 | 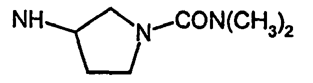 | |
| 45 | 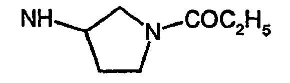 | |
| 46 | 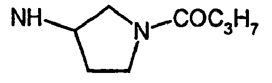 | |
| 47 | 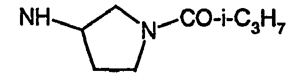 | |
| 48 | 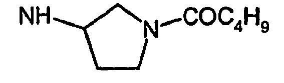 | |
| 49 | 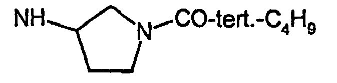 | |
| 50 | 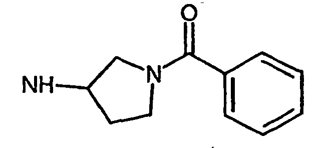 | |
| 51 | 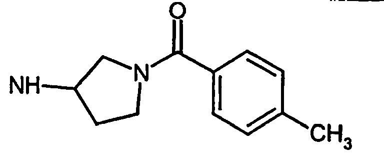 | |
| 52 | 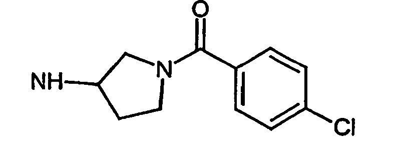 | |
| 53 | 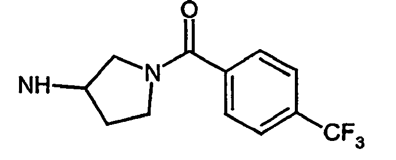 | |
| 54 | 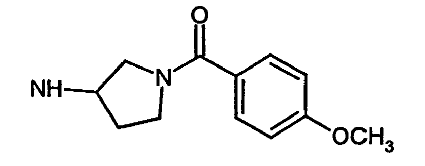 | |
| 55 | 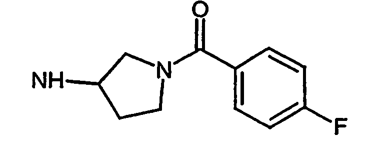 | |
| 56 | 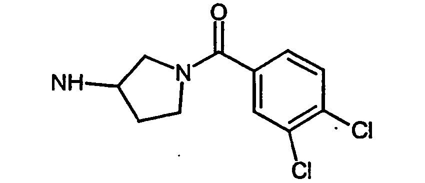 | |
| 57 | 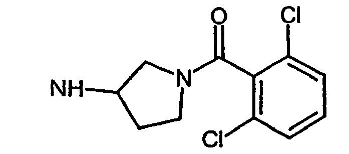 | |
| 58 | 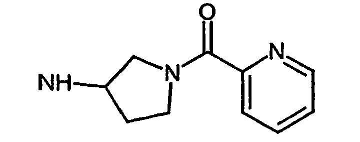 | |
| 59 | 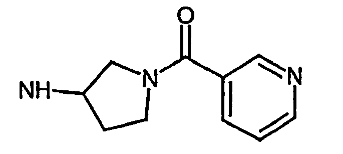 | |
| 60 | 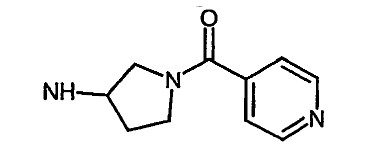 | |
| 61 | 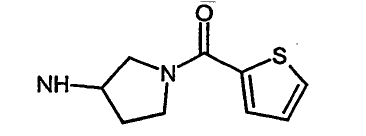 | |
| 62 | 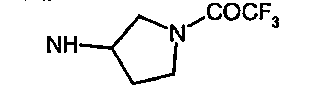 | |
| 63 | 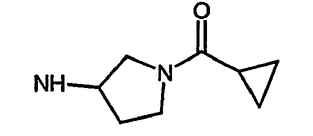 | |
| 64 | 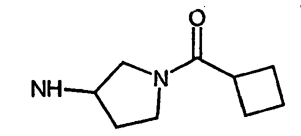 | |
| 65 | 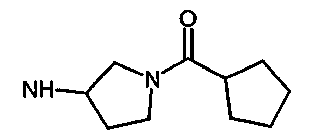 | |
| 66 | 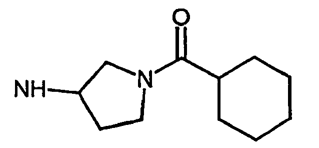 | |
| 67 | 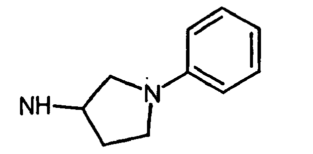 | |
| 68 | 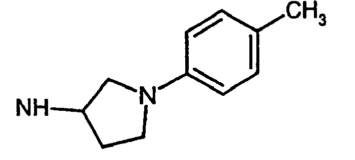 | |
| 69 | 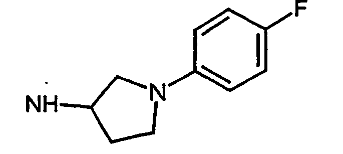 | |
| 70 | 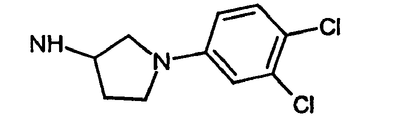 | |
| 71 | 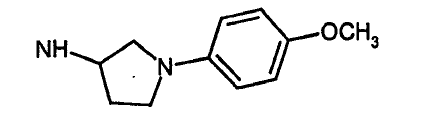 | |
| 72 | 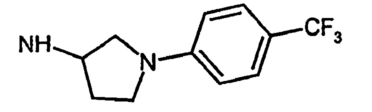 | |
| 73 | 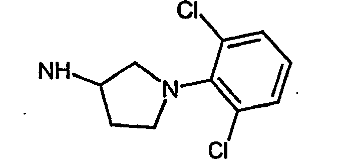 | |
| 74 | 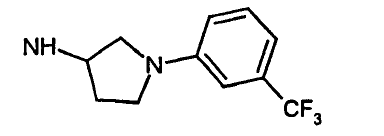 | |
| 75 | 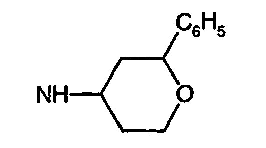 | |
| 76 | 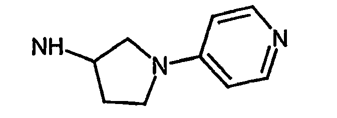 | |
| 77 | 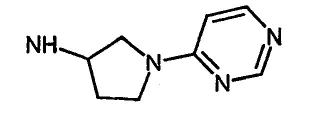 | |
| 78 | 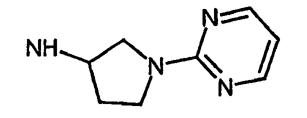 | |
| 79 | 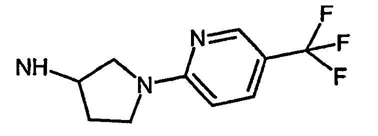 | |
| 80 | 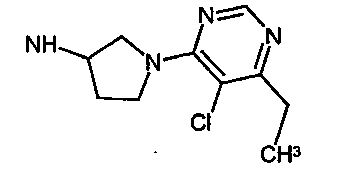 | |
| 81 | 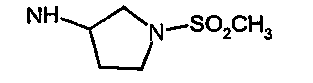 | |
| 82 | 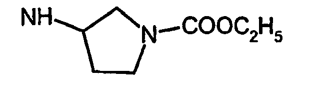 | |
| 83 | 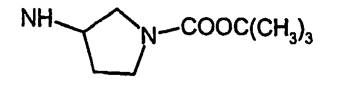 | 107-108 |
| 84 | 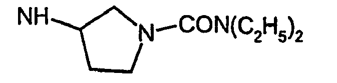 | |
| 85 | 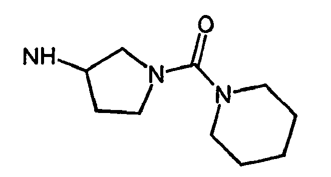 | |
| 86 | 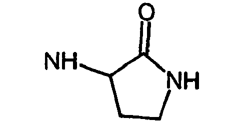 | |
| 87 | 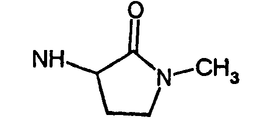 | |
| 88 | 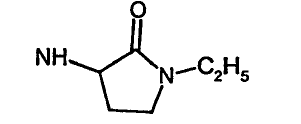 | |
| 89 | 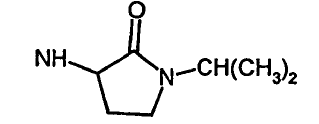 | |
| 90 | 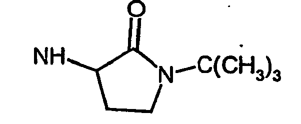 | |
| 91 | 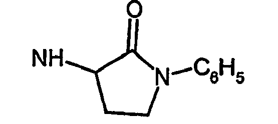 | 170-171 |
| 92 | 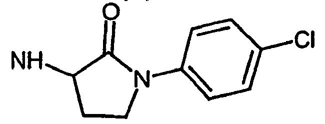 | |
| 93 | 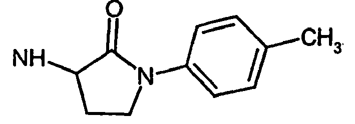 | |
| 94 | 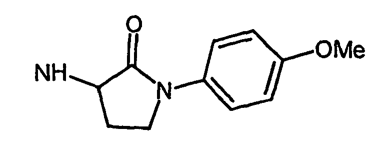 | |
| 95 | 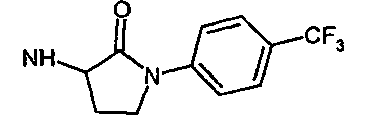 | |
| 96 | 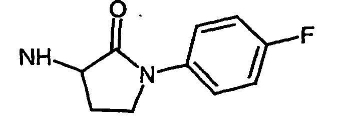 | |
| 97 | 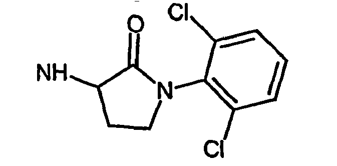 | |
| 98 | 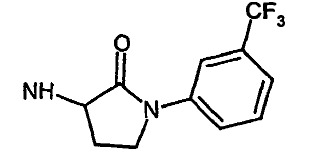 | 197-197 |
| 99 | 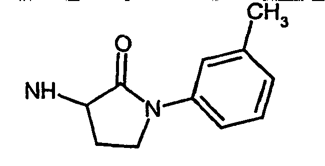 | 130 - 132 |
| 100 | 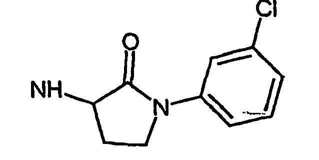 | 195-198 |
| 101 | 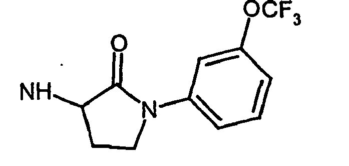 | 180-181 |
| 102 | 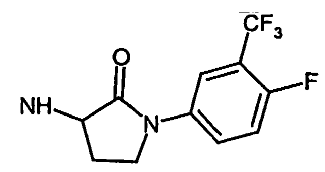 | 161-163 |
| 103 | 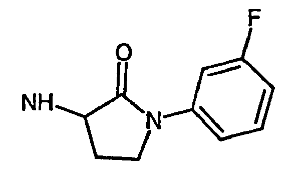 | 168-171 |
| 104 | 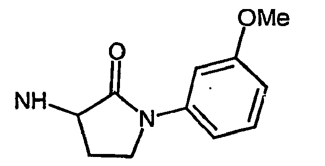 | 164-167 |
| 105 | 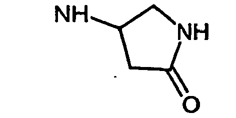 | |
| 107 | 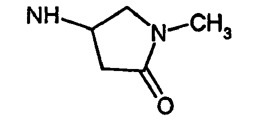 | |
| 108 | 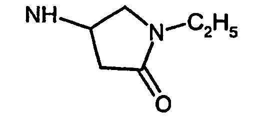 | |
| 109 | 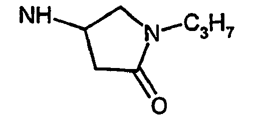 | |
| 110 | 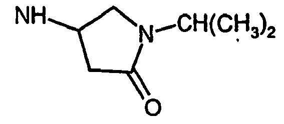 | |
| 111 | 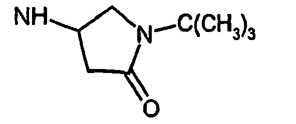 | |
| 112 | 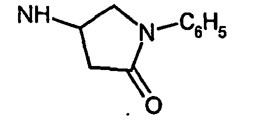 | |
| 113 | 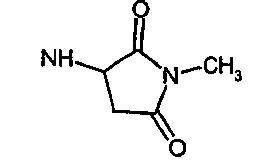 | |
| 114 | 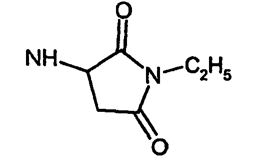 | |
| 115 | 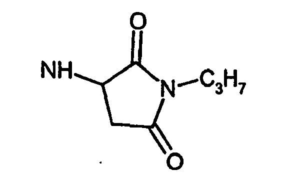 | |
| 116 | 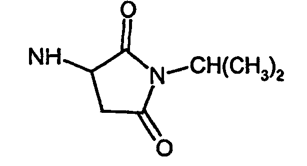 | |
| 117 | 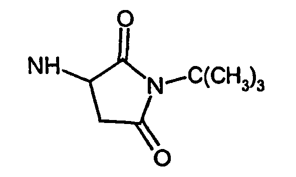 | |
| 118 | 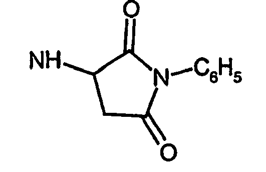 | |
| 119 | 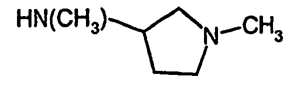 | Öl |
| 120 | 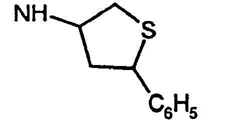 | |
| 121 | 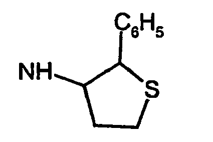 | |
| 122 | 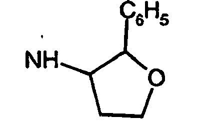 | |
| 123 | 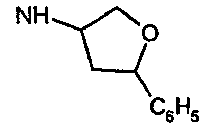 | |
| 124 | 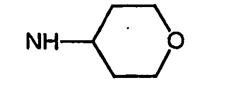 | 149-150 |
| 125 | 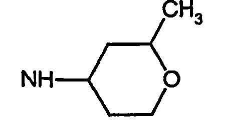 | |
| 126 | 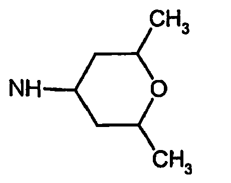 | |
| 127 | 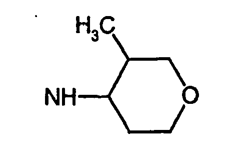 | |
| 128 | 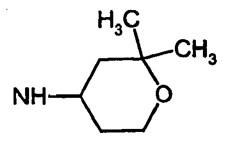 | 138 |
| 129 | 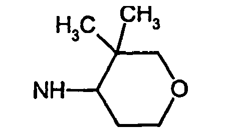 | |
| 130 | 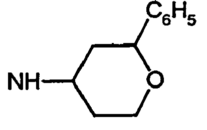 | |
| 131 | 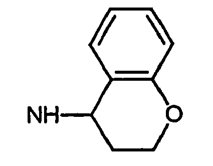 | 197-199 |
| 132 | 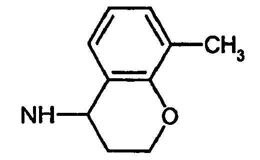 | 216-217 |
| 133 | 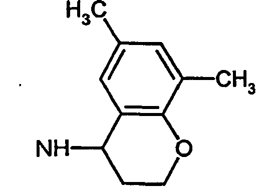 | |
| 134 | 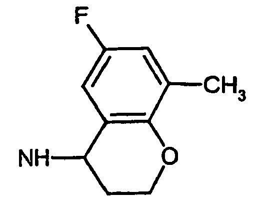 | 217-219 |
| 135 | 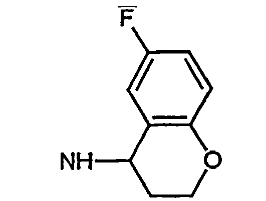 | 200-202 |
| 136 | 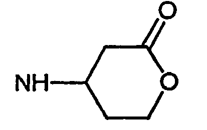 | |
| 137 | 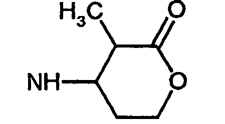 | |
| 138 | 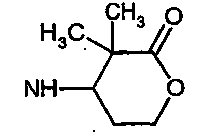 | |
| 139 | 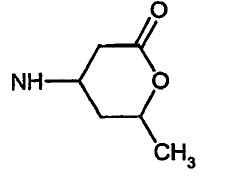 | |
| 140 | 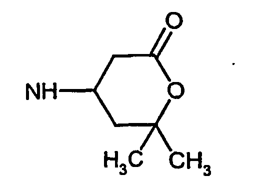 | |
| 141 | 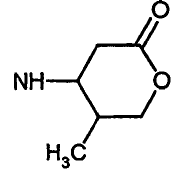 | |
| 142 | 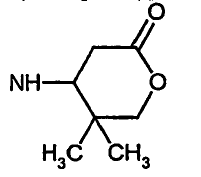 | |
| 143 | 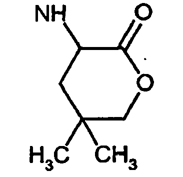 | |
| 144 | 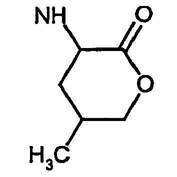 | |
| 145 | 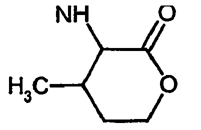 | |
| 146 | 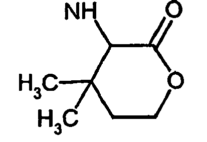 | |
| 147 | 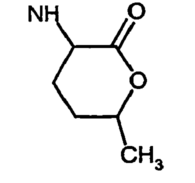 | |
| 148 | 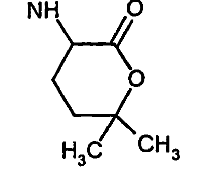 | |
| 149 | 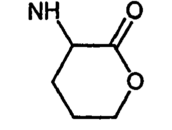 | |
| 150 | 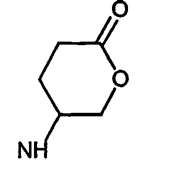 | |
| 151 | 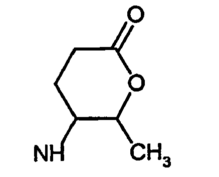 | |
| 152 | 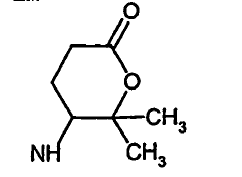 | |
| 153 | 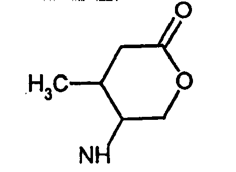 | |
| 154 | 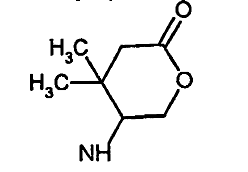 | |
| 155 | 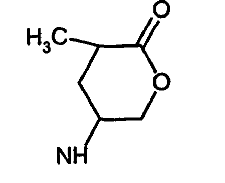 | |
| 156 | 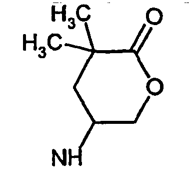 | |
| 157 | 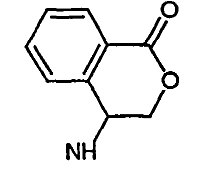 | |
| 158 | 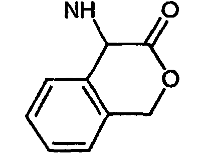 | |
| 159 | 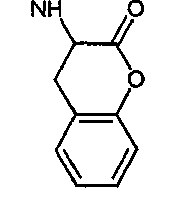 | |
| 160 | 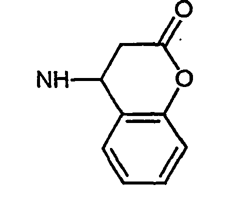 | |
| 161 | 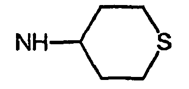 | 161-162 |
| 162 | 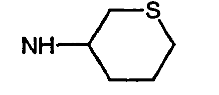 | 168-170 |
| 163 | 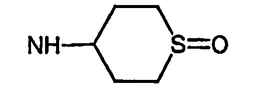 | 243 |
| 164 | 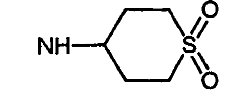 | 248 |
| 165 | 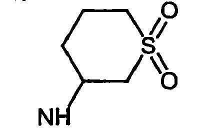 | 208-209 |
| 166 | 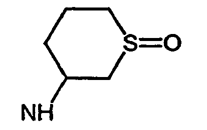 | Öl |
| 167 | 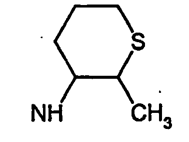 | |
| 168 | 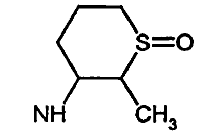 | |
| 169 | 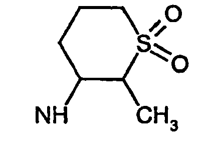 | |
| 170 | 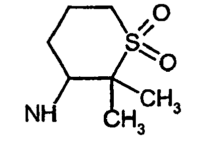 | |
| 171 | 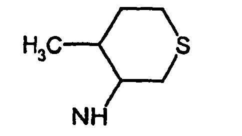 | |
| 172 | 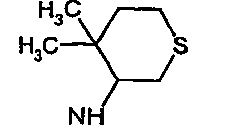 | |
| 173 | 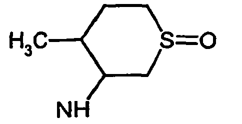 | |
| 174 | 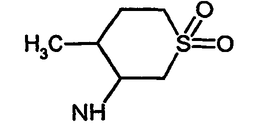 | |
| 175 | 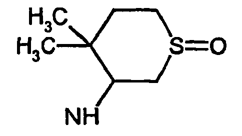 | |
| 176 | 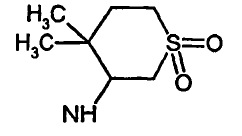 | |
| 177 | 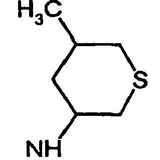 | |
| 178 | 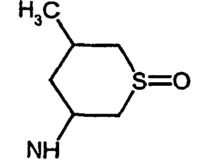 | |
| 179 | 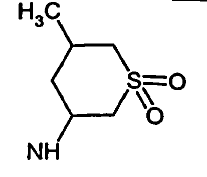 | |
| 180 | 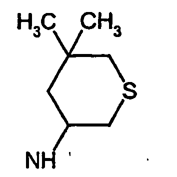 | |
| 181 | 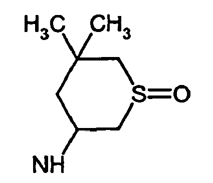 | |
| 182 | 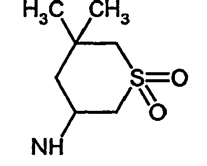 | |
| 183 | 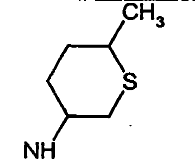 | |
| 184 | 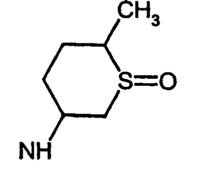 | |
| 185 | 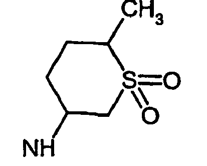 | |
| 186 | 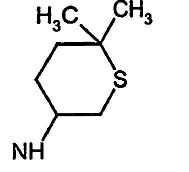 | |
| 187 | 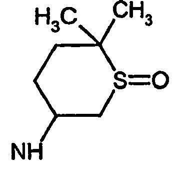 | |
| 188 | 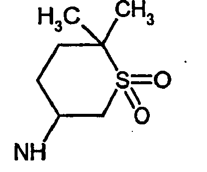 | |
| 189 | 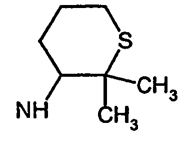 | |
| 190 | 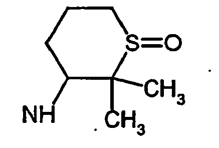 | |
| 191 | 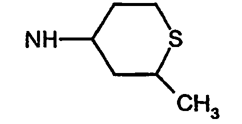 | |
| 192 | 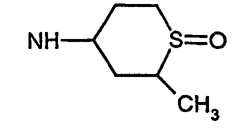 | |
| 193 | 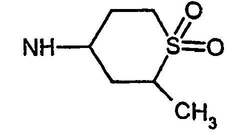 | |
| 194 | 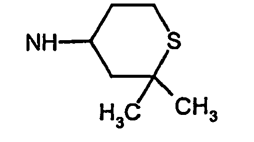 | |
| 195 | 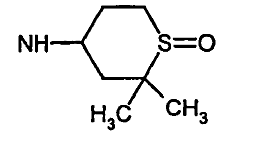 | |
| 196 | 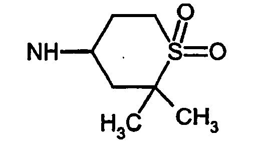 | |
| 197 | 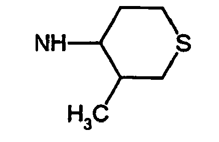 | |
| 198 | 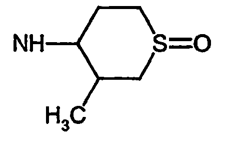 | |
| 199 | 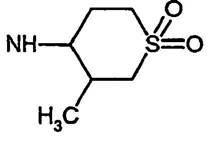 | |
| 200 | 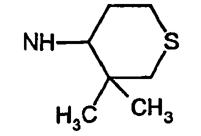 | |
| 201 | 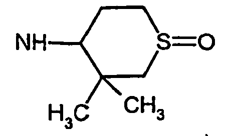 | |
| 202 | 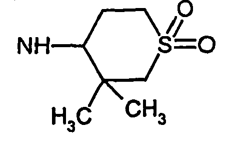 | |
| 203 | 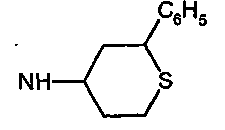 | |
| 204 | 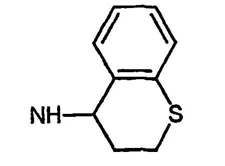 | |
| 205 | 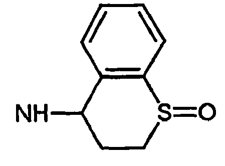 | |
| 206 | 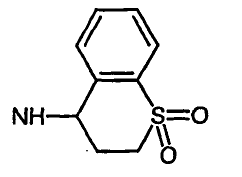 | |
| 207 | 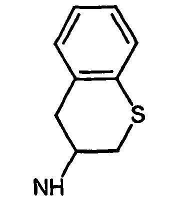 | |
| 208 | 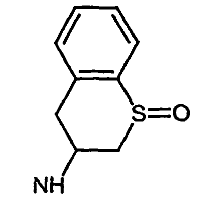 | |
| 209 | 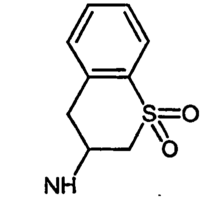 | |
| 210 | 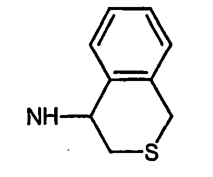 | |
| 211 | 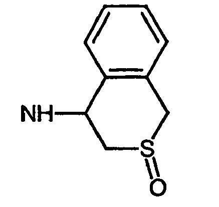 | |
| 212 | 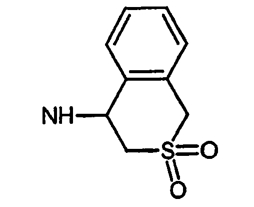 | |
| 213 | 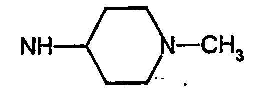 | |
| 214 | 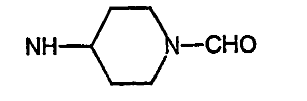 | |
| 215 | 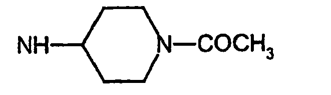 | 207-209 |
| 216 | 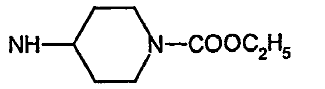 | 145-146 |
| 217 | 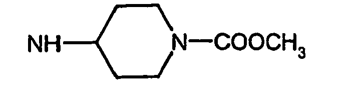 | |
| 218 | 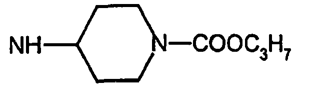 | |
| 219 | 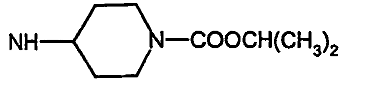 | |
| 220 | 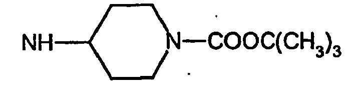 | 93-96 |
| 221 | 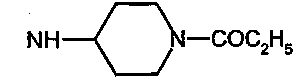 | |
| 222 | 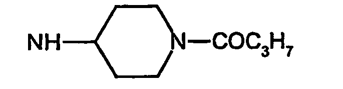 | |
| 223 | 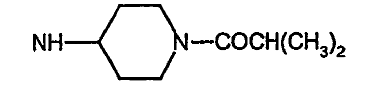 | |
| 224 | 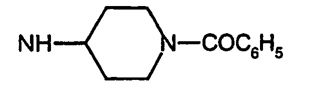 | |
| 225 | 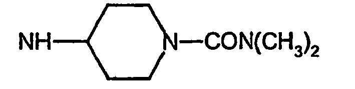 | |
| 226 | 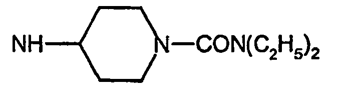 | |
| 227 | 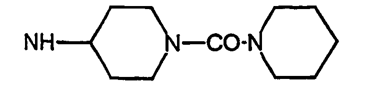 | |
| 228 | 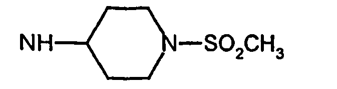 | |
| 229 | 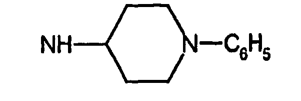 | |
| 230 | 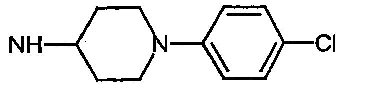 | |
| 231 | 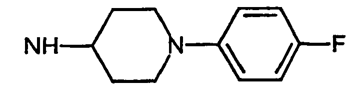 | 193 |
| 232 | 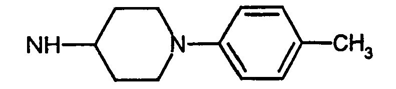 | |
| 233 | 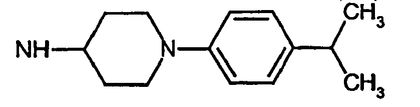 | 152-153 |
| 234 | 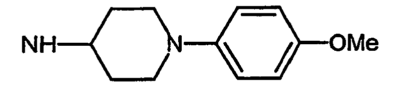 | 178 |
| 235 | 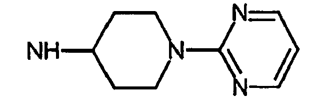 | |
| 236 | 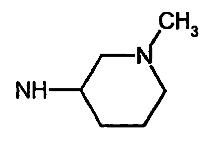 | |
| 237 | 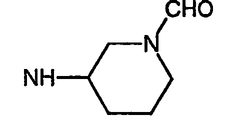 | |
| 238 | 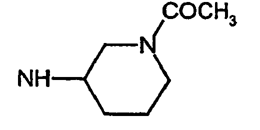 | 172-174 |
| 239 | 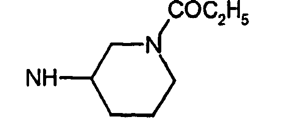 | |
| 240 | 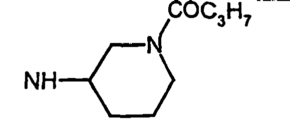 | |
| 241 | 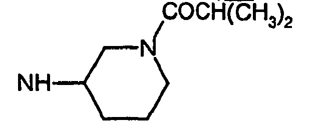 | |
| 242 | 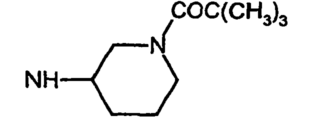 | |
| 243 | 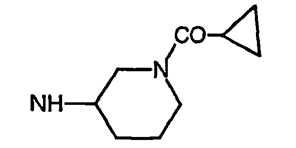 | |
| 244 | 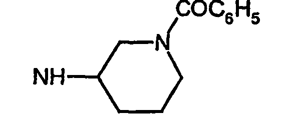 | |
| 245 | 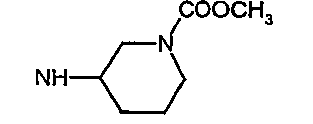 | |
| 246 | 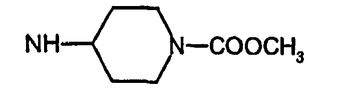 | |
| 247 | 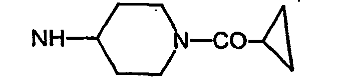 | |
| 248 | 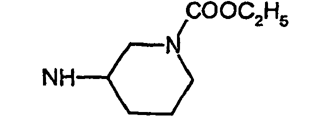 | |
| 249 | 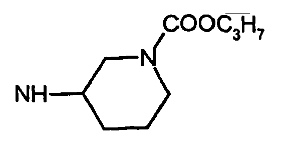 | |
| 250 | 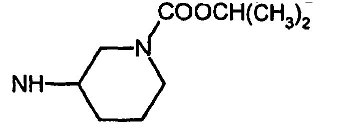 | |
| 251 | 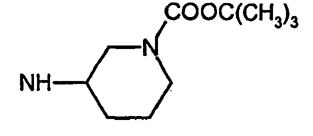 | 153-156 |
| 252 | 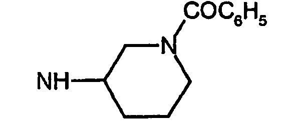 | |
| 253 | 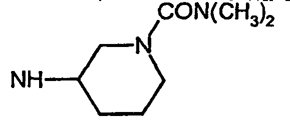 | |
| 254 | 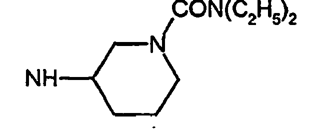 | |
| 255 | 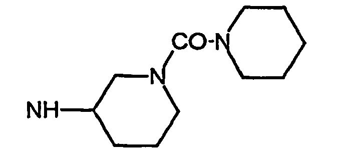 | |
| 256 | 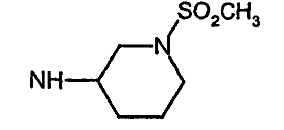 | |
| 257 | 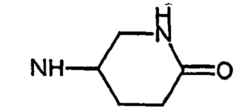 | |
| 258 | 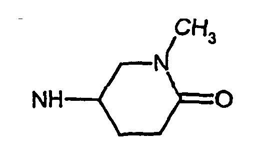 | |
| 259 | 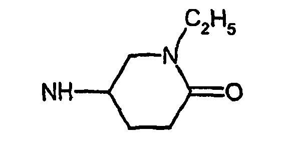 | |
| 260 | 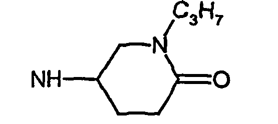 | |
| 261 | 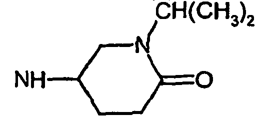 | |
| 262 | 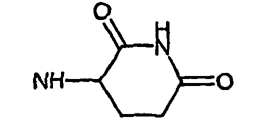 | |
| 263 | 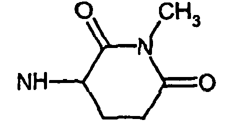 | |
| 264 | 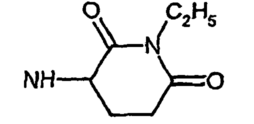 | |
| 265 | 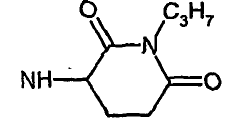 | |
| 266 | 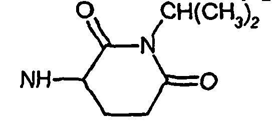 | |
| 267 | 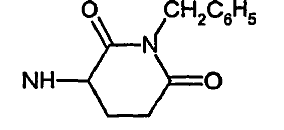 | |
| 268 | 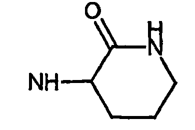 | |
| 269 | 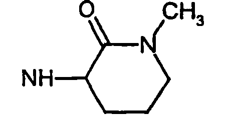 | |
| 270 | 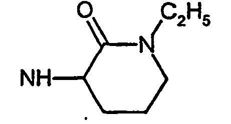 | |
| 271 | 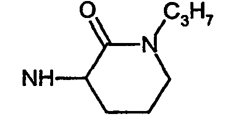 | |
| 272 | 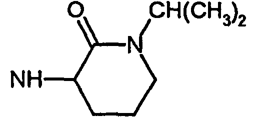 | |
| 273 | 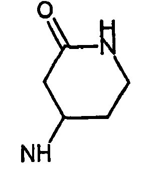 | |
| 274 | 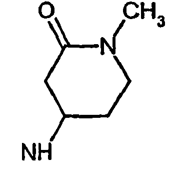 | |
| 275 | 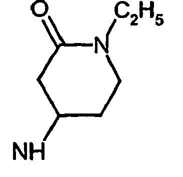 | |
| 276 | 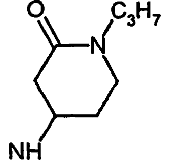 | |
| 277 | 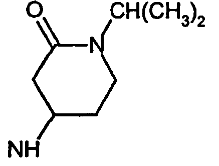 | |
| 278 | 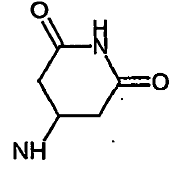 | |
| 279 | 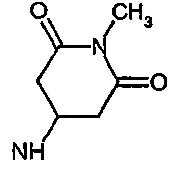 | |
| 280 | 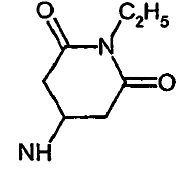 | |
| 281 | 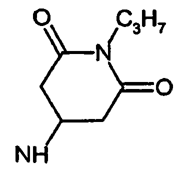 | |
| 282 | 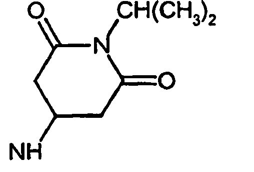 | |
| 283 | 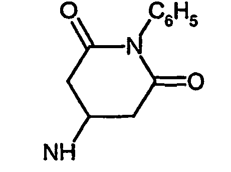 | |
| 284 | 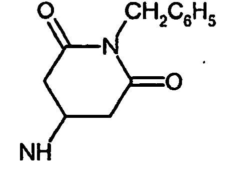 | |
| 285 | 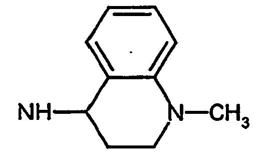 | |
| 286 | 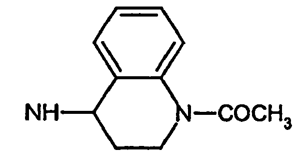 | |
| 287 | 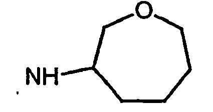 | |
| 288 | 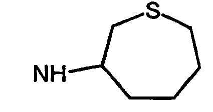 | |
| 289 | 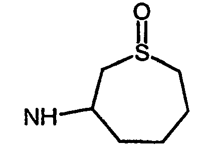 | |
| 290 | 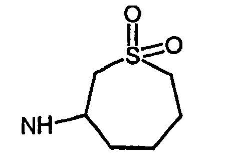 | |
| 291 | 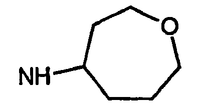 | |
| 292 | 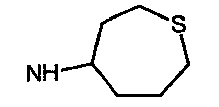 | |
| 293 | 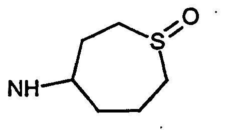 | |
| 294 | 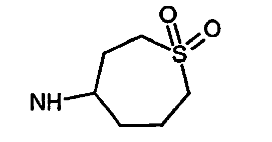 | |
| 295 | 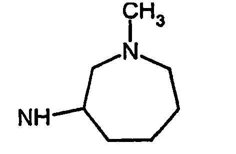 | |
| 296 | 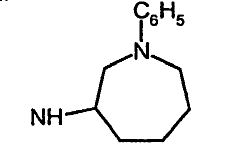 | |
| 297 | 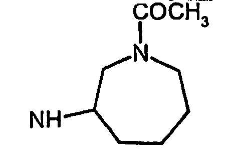 | |
| 298 | 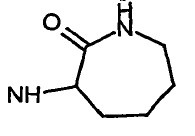 | |
| 299 | 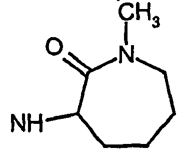 | |
| 300 | 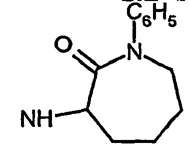 | |
| 301 | 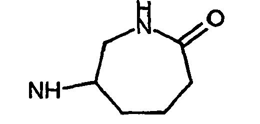 | |
| 302 | 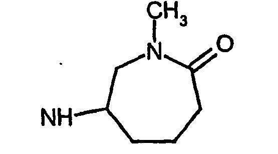 | |
| 303 | 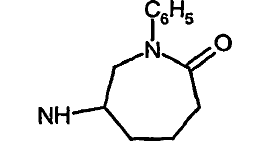 | |
| 304 | 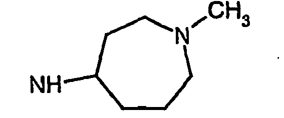 | |
| 305 | 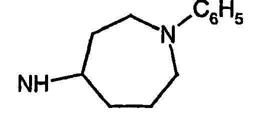 | |
| 306 | 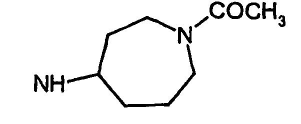 | |
| 307 | 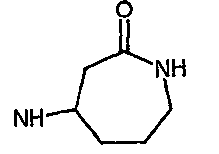 | |
| 308 | 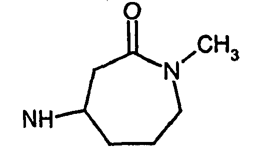 | |
| 309 | 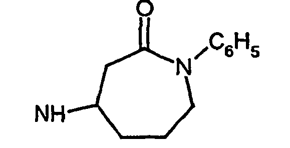 | |
| 310 | 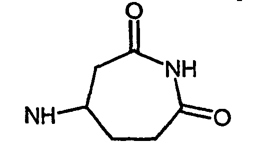 | |
| 311 | 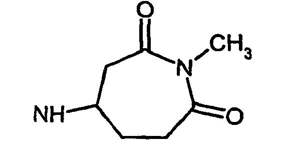 | |
| 312 | 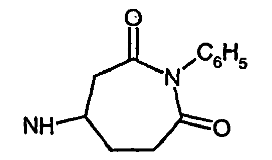 | |
| 313 | 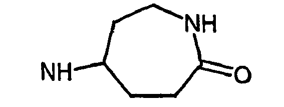 | |
| 314 | 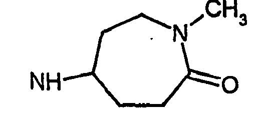 | |
| 315 | 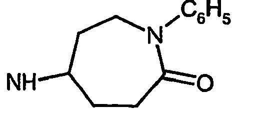 | |
| 316 | 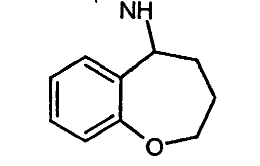 | |
| 317 | 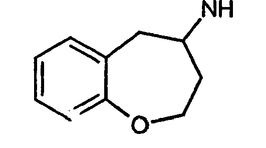 | |
| 318 | 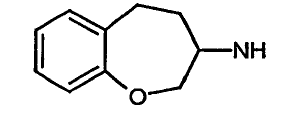 | |
| 319 | 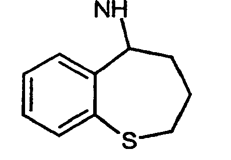 | |
| 320 | 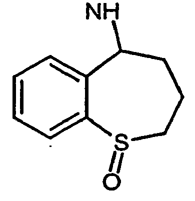 | |
| 321 | 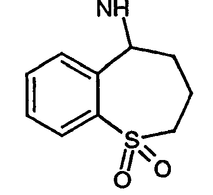 | |
| 322 | 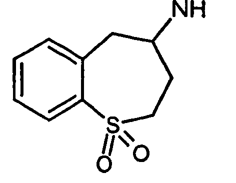 | |
| 323 | 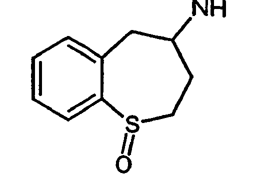 | |
| 324 | 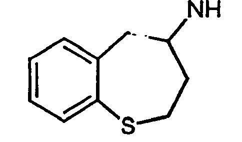 | |
| 325 | 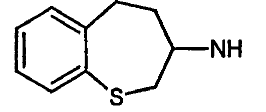 | |
| 326 | 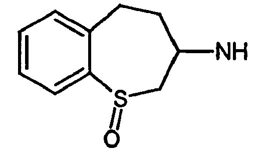 | |
| 327 | 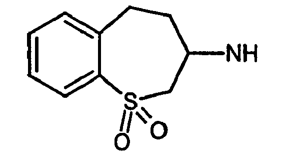 | |
| 328 | 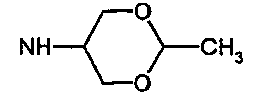 | 191-192 |
| 329 | 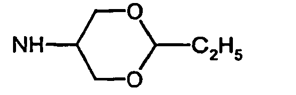 | |
| 330 | 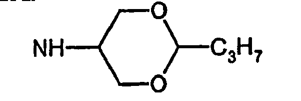 | |
| 331 | 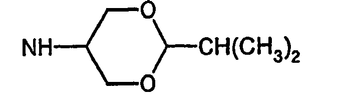 | Öl |
| 332 | 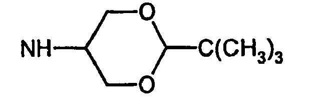 | |
| 333 | 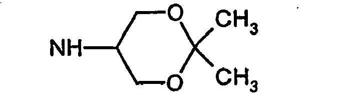 | 155-156 |
| 334 | 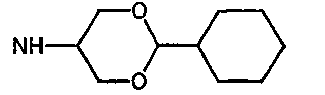 | 133-134 |
| 335 | 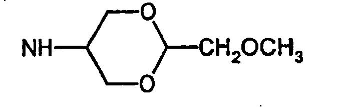 | Öl |
| 336 | 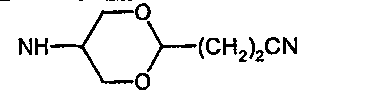 | Öl |
| 337 | 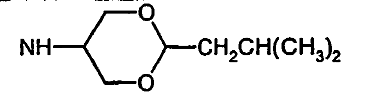 | 95 - 96 |
| 338 | 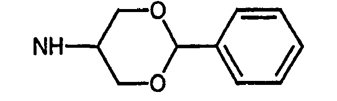 | 164 -165 |
| 339 | 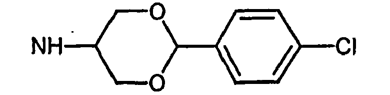 | |
| 340 | 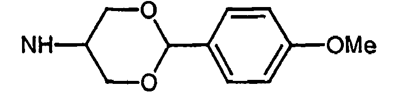 | |
| 341 | 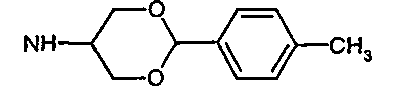 | |
| 342 | 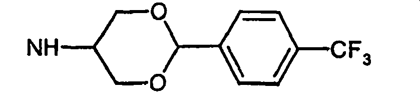 | |
| 343 | 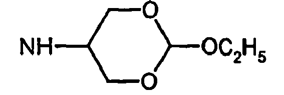 | 104 |
| 344 | 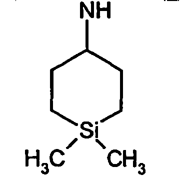 | Öl |
| 345 | 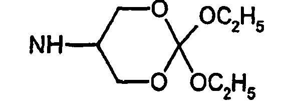 | 122-123 |
| 346 | 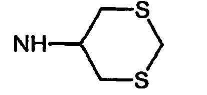 | |
| 347 | 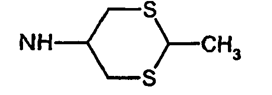 | |
| 348 | 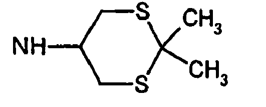 | |
| 349 | 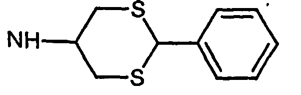 | |
| 350 | 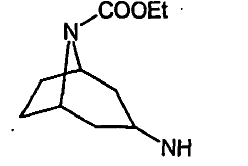 | |
| 351 | 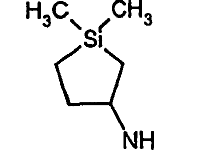 | |
| 352 | 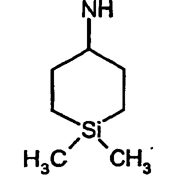 | |
| 353 | 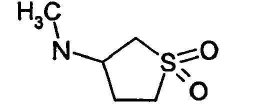 | 38-41 |
| 354 | 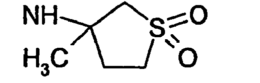 | 149-152 |
| 355 | 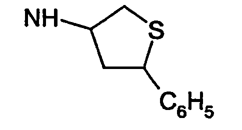 | 152-154 |
| 356 | 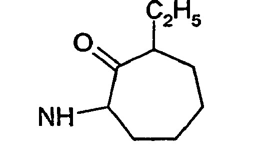 | 38-41 |
| 357 | 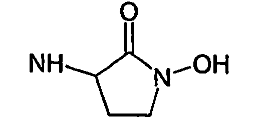 | |
| 358 | 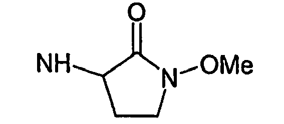 | |
| 359 | 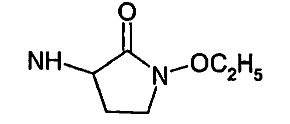 | |
| 360 | 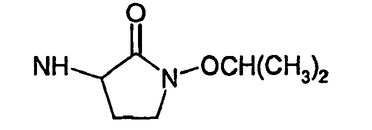 | |
| 361 | 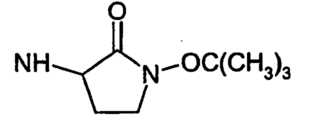 | |
| 362 | 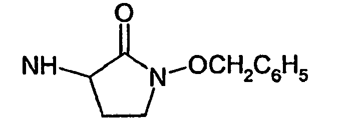 | |
| 363 | 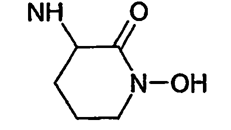 | |
| 364 | 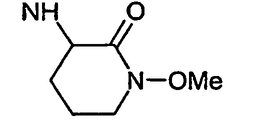 | |
| 365 | 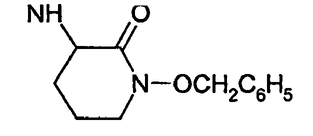 | |
| 366 | 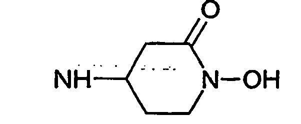 | |
| 367 | 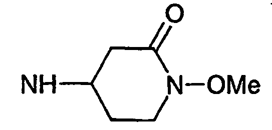 | |
| 368 | 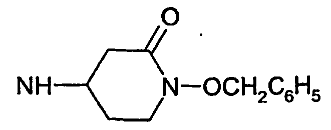 | |
| 369 | 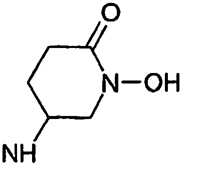 | |
| 370 | 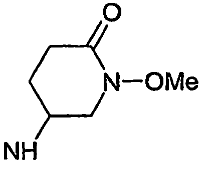 | |
| 371 | 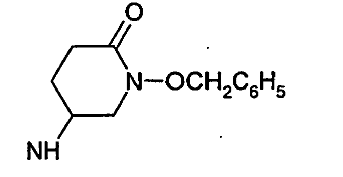 | |

**Tabelle 2**

| 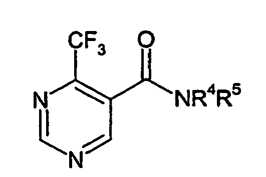 | | |
|---|---|---|
| Bsp.-Nr. | NR⁴R⁵ | Smp. [°C] |
| 372 | 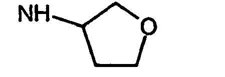 | |
| 373 | 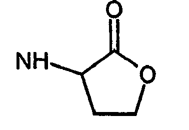 | |
| 374 | 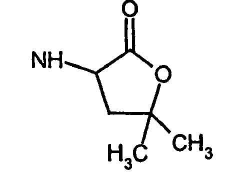 | |
| 375 | 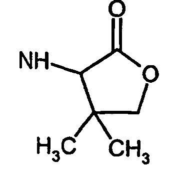 | |
| 376 | 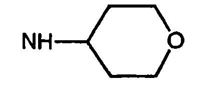 | |
| 377 | 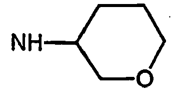 | |
| 378 | 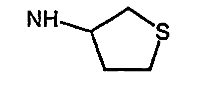 | |
| 379 | 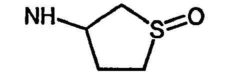 | |
| 380 | 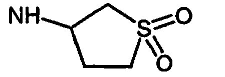 | |
| 381 | 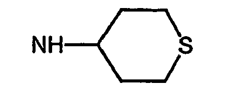 | |
| 382 | 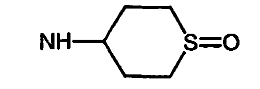 | |
| 383 | 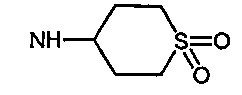 | |
| 384 | 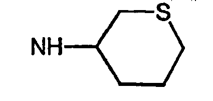 | |
| 385 | 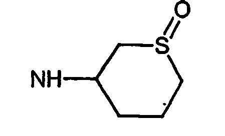 | |
| 386 | 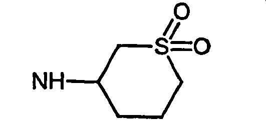 | |
| 387 | 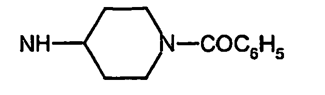 | |
| 388 | 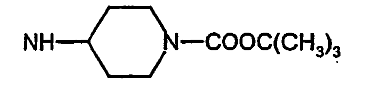 | |

### B. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

In den folgenden Beispielen 1 bis 3 werden Verbindungen als aktiv angesehen, wenn sie bei einer Konzentration von 500 ppm (bezogen auf den Gehalt an Wirkstoff) oder weniger eine Wirkung auf die Schadorganismen von 50% oder mehr aufweisen.

### Beispiel 1

Angekeimte Ackerbohnen-Samen (Vicia faba) mit Keimwurzeln wurden in mit Leitungswasser gefüllte Braunglasfläschen übertragen und anschließend mit ca. 100 schwarzen Bohnenblattläusen (Aphis fabae) belegt. Pflanzen und Blattläuse wurden dann für 5 Sekunden in eine wäßrige Lösung der zu prüfenden und formulierten Verbindung getaucht. Nach dem Abtropfen wurden Pflanze und Tiere in einer Klimakammer gelagert (16 Stunden Licht/Tag, 25°C, 40-60% RF). Nach 3 und 6 Tagen Lagerung wurde die Mortalität der Verbindung auf die Blattläuse festgestellt. Die Verbindungen gemäß der folgenden Beispiele waren aktiv: 3, 8, 9, 11, 17, 18, 19, 21, 22 (cis), 22 (trans), 24, 30 (cis), 30 (trans), 31 (cis), 124, 134, 135, 161, 163, 164, 166, 203, 216, 219, 353 und 356.

### Beispiel 2

Angekeimte Ackerbohnen-Samen (Vicia faba) mit Keimwurzeln wurden in mit Leitungswasser gefüllte Braunglasfläschen übertragen. Vier Milliliter einer wäßrigen Lösung der zu prüfenden und formulierten Verbindung wurden in das Braunglasfläschen hineinpipettiert. Anschließend wurde die Ackerbohne mit ca. 100 schwarzen Bohnenblattläusen (Aphis fabae) stark belegt. Pflanze und Blattläuse wurden dann in einer Klimakammer gelagert (16 Stunden Licht/Tag, 25°C, 40-60% RF). Nach 3 und 6 Tagen Lagerung wurde die wurzelsystemische Wirkung der Verbindung als Mortalität der Blattläuse festgestellt. Die Verbindungen gemäß der folgenden Beispiele waren aktiv: 3, 8, 9, 10, 11, 12, 17, 18, 19, 21, 22 (cis), 22 (trans), 24, 30 (cis), 30 (trans), 31 (cis), 35, 124, 128, 134, 135, 161, 162, 163, 164, 165, 166, 203, 216, 219, 237, 250, 353 und 356.

### Beispiel 3

Buschbohnen (Phaseolus vulgaris) mit je zwei gut entwickelten Blättern wurden mit einer wässerigen Lösung der zu prüfenden und formulierten Verbindung tropfnaß gespritzt. Nach dem Abtrocknen wurden jeweils eine behandelte, eine unbehandelte und eine stark mit Weißen Fliegen (Trialeurodes vaporariorum) infizierte Bohnenpflanze zusammengestellt. Die Pflanzen wurden im Gewächshaus bei 25°C und 60% relativer Feuchte aufgestellt. Nach 48 Stunden wurden die Anzahl der gelegten Eier auf der behandelten und der unbehandelten Bohnepflanze bestimmt. Der Grad der Repellent-Wirkung ergab sich aus dem relatriven Vergleich der Anzahl der Eier auf der unbehandelten Pflanze (entspricht jeweils 100%) mit der Anzahl Eier auf der behandelten Pflanze. Die Verbindung gemäß dem folgenden Beispiel war in diesem Repellent-Versuch aktiv: 19.

### Beispiel 4

Sojabohnenpflanzen mit je drei gut entwickelten Blättern wurden mit einer wässerigen Lösung der zu prüfenden und formulierten Verbindung tropfnaß gespritzt. Nach dem Abtrocknen wurden jeweils eine behandelte, eine unbehandelte und eine mit Thrips der Spezies Frankliniella occidentalis infizierte Pflanze zusammengestellt. Die Pflanzen wurden in einer Klimakammer (23°C, 50 - 60 % relative Luftfeuchtigkeit) gelagert. Nach 14 Tagen wurde der Blattschaden bei den behandelten und unbehandelten Pflanzen bestimmt. Der Grad der Repellent-Wirkung ergab sich aus dem relativen Vergleich des Blattschadens der unbehandelten Pflanze (entspricht jeweils 100 %) mit dem der behandelten Pflanze. Die Verbindung gemäß dem folgenden Beispiel war in diesem Repellent-Versuch aktiv: 19.

## Patentansprüche

1. Amide der Formel (I) und deren Salze, 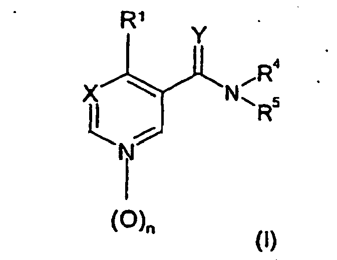 wobei die Symbole und Indizes folgende Bedeutungen haben:
X ist CH oder N;
Y ist O oder S;
n ist 0 oder 1;
R¹ ist (C₁-C₄)-Haloalkyl;
R⁴ ist Wasserstoff, (C₁-C₁₀)-Alkyl, (C₃-C₁₀)-Cycloalkyl, (C₃-C₁₀)-Alkenyl oder (C₃-C₁₀)-Alkinyl, wobei in den genannten Alkyl-, Cycloalkyl-, Alkenyl- oder Alkinyl-Gruppen bis zu drei Wasserstoffatome durch Halogen ersetzt sein können, im Falle von Fluor auch bis zur Maximalanzahl;
R⁵ ist ein nicht-aromatischer Heterocyclus enthaltend mindestens ein Sauerstoff-, Schwefel-, Stickstoff- und/oder Siliziumringatom, der gegebenenfalls mit einer bis sechs einwertigen Gruppen substituiert ist, und der gegebenenfalls Teil eines spirocyclischen, kondensierten oder bicyclischen Ringsystems sein kann.

2. Amide der Formel (I) und deren Salze nach Anspruch 1, **dadurch gekennzeichnet, dass** R⁵ ein vier- bis achtgliedriger nicht-aromatischer Heterocyclus ist, der ein Sauerstoffringatom, ein Schwefelringatom, ein Stickstoffringatom, ein Siliziumringatom, zwei Sauerstoffringatome oder zwei Schwefelringatome aufweist, wobei die Schwefelringatome in der Form -S(O)ₚ- vorliegen, und p 0, 1 oder 2 bedeutet.

3. Amide der Formel (I) und deren Salze nach Anspruch 1 oder 2, wobei R⁵ ein nicht-aromatischer Heterocyclus der Formel (II) ist, 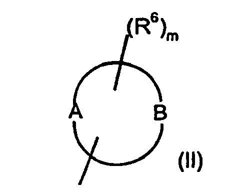 worin A und/oder B mindestens ein Sauerstoff-, Schwefel-, Stickstoff oder Siliziumringatom bedeutet, und der Ring zusätzlich eine oder zwei Carbonylgruppen enthalten kann, die zusammen mit den Heteroeinheiten eine Lacton-, Lactam- oder Imid-Einheit bilden können; und, falls A Sauerstoff und B Stickstoff bedeutet, diese Einheiten unmittelbar benachbart sein können und in allen anderen Fällen, für die A und B Heteroatomeinheiten bedeuten, diese durch mindestens eine gesättigte Kohlenstoff-Einheit getrennt sein müssen;
m 0 oder 1 bis 6 bedeutet;
R⁶ in Abhängigkeit von m gleich oder verschieden und jeweils (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl bedeutet, das gegebenenfalls mit einem oder mehreren gleichen oder verschiedenen Resten substituiert ist, oder einen Rest R⁷ darstellt, wobei
R⁷ Halogen, Cyano, Nitro, Hydroxy, Thio, Amino, (C₁-C₁₀)-Alkanoyl, (C₃-C₁₀)-Alkenoyl, (C₃-C₁₀)-Alkinoyl, (C₃-C₁₀)-Cycloalkanoyl, (C₁-C₁₀)-Alkoxy, (C₃-C₁₀)-Alkenyloxy, (C₃-C₁₀)-Alkinyloxy, (C₃-C₁₀)-Cycloalkoxy, (C₄-C₁₀)-Cycloalkenyloxy, (C₃-C₈)-Cycloalkyl-(C₁-C₄)-alkoxy, (C₄-C₈)-Cycloalkenyl-(C₁-C₄)-alkoxy, (C₃-C₈)-Cycloalkyl-(C₃-C₄)-alkenyloxy, (C₄-C₈)-Cycloalkenyl-(C₃-C₄)-alkenyloxy, (C₁-C₄)-Alkyl-(C₃-C₈)-cycloalkoxy, (C₂-C₄)-Alkenyl-(C₃-C₈)-cycloalkoxy, (C₂-C₄)-Alkinyl-(C₃-C₈)-cycloalkoxy, (C₁-C₄)-Alkyl-(C₄-C₈)-cycloalkenyloxy, (C₂-C₄)-Alkenyl-(C₄-C₈)-cycloalkenyloxy, (C₁-C₄)-Alkoxy-(C₁-C₄)-alkoxy, (C₁-C₄)-Alkoxy-(C₃-C₄)-alkenyloxy, Carbamoyl, (C₁-C₈)-Mono- oder -Dialkylcarbamoyl, (C₃-C₈)-Mono- oder -Dicycloalkylcarbamoyl, (C₁-C₈)-Alkoxycarbonyl, (C₃-C₈)-Cycloalkoxycarbonyl, (C₁-C₈)-Alkanoyloxy, (C₃-C₈)-Cycloalkanoyloxy, (C₁-C₈)-Alkanoylamino, (C₃-C₈)-Alkenoylamino, (C₃ C₈)-Cycloalkanoyl-amino, (C₃-C₈)-Cycloalkyl-(C₁-C₄)-alkanoylamino, die N-(C₁-C₄)-Alkylamino-Analogen der vier letztgenannten Reste, (C₁-C₁₀)-Alkylthio, (C₃-C₁₀)-Alkenylthio, (C₃-C₁₀)-Alkinylthio, (C₃-C₈)-Cycloalkylthio, (C₄-C₈)-Cycloalkenylthio, (C₃-C₈)-Cycloalkyl-(C₁-C₄)-alkylthio, (C₄-C₈)-Cycloalkenyl-(C₁-C₄)-alkylthio, (C₃-C₈)-Cycloalkyl-(C₃-C₄)-alkenylthio, (C₄-C₈)-Cycloalkenyl-(C₃-C₄)-alkenylthio, (C₁-C₄)-Alkyl-(C₃-C₈)-cycloalkylthio, (C₂-C₄)-Alkenyl-(C₃-C₈)-cycloalkylthio, (C₂-C₄)-Alkinyl-(C₃-C₈)-cycloalkylthio, (C₁-C₈)-Alkyl-(C₄-C₈)-cycloalkenylthio, (C₂-C₆)-Alkenyl-(C₄-C₆)-cycloalkenylthio, (C₁-C₁₀)-Alkylsulfinyl, (C₃-C₄)-Alkenylsulfinyl, (C₃-C₄)-Alkinylsulfinyl, (C₃-C₁₀)-Cycloalkylsulfinyl, (C₄-C₁₀)-Cycloalkenylsulfinyl, (C₃-C₈)-Cycloalkyl-(C₁-C₄)-alkylsulfinyl, (C₄-C₈)-Cycloalkenyl-(C₁-C₄)-alkylsulfinyl, (C₃-C₈)-Cycloalkyl-(C₃-C₄)-alkenylsulfinyl, (C₄-C₈)-Cycloalkenyl-(C₃-C₄)-alkenylsulfinyl, (C₁-C₄)-Alkyl-(C₃-C₈)-cycloalkylsulfinyl, (C₂-C₄)-Alkenyl-(C₃-C₈)-cycloalkylsulfinyl, (C₂-C₄)-Alkinyl-(C₃-C₈)-cycloalkylsulfinyl, (C₁-C₄)-Alkyl-(C₄-C₈)-cycloalkenylsulfinyl, (C₂-C₃)-Alkenyl-(C₄-C₈)-cycloalkenylsulfinyl, (C₁-C₁₀)-Alkylsulfonyl, (C₃-C₁₀)-Alkenylsulfonyl, (C₃-C₁₀)-Alkinylsulfonyl, (C₃-C₁₀)-Cycloalkylsulfonyl, (C₄-C₁₀)-Cycloalkenylsulfonyl, (C₃-C₈)-Cycloalkyl-(C₁-C₄)-alkylsulfonyl, (C₄-C₈)-Cycloalkenyl-(C₁-C₄)-alkylsulfonyl, (C₃-C₈)-Cycloalkyl-(C₃-C₄)-alkenylsulfonyl, (C₄-C₈)-Cycloalkenyl-(C₃-C₄)-alkenylsulfonyl, (C₁-C₄)-Alkyl-(C₃-C₈)-cycloalkylsulfonyl, (C₂-C₄)-Alkenyl-(C₃-C₈)-cycloalkylsulfonyl, (C₃-C₈)-Alkinyl-(C₃-C₈)-cycloalkylsulfonyl, (C₁-C₄)-Alkyl-(C₄-C₈)-cycloalkenylsulfonyl, (C₃-C₄)-Alkenyl-(C₄-C₈)-cycloalkenylsulfonyl, (C₁-C₁₀)-Alkylamino, (C₃-C₁₀)-Alkenylamino, (C₃-C₁₀)-Alkinylamino, (C₃-C₈)-Cycloalkylamino, (C₄-C₈)-Cycloalkenylamino, (C₃-C₈)-Cycloalkyl-(C₁-C₄)-alkylamino, (C₄-C₈)-Cycloalkenyl-(C₁-C₄)-alkylamino, (C₃-Cₐ)-Cycloalkyl-(C₃-C₄)-alkenylamino, (C₄-C₈)-Cycloalkenyl-(C₃-C₄)-alkenylamino. (C₁-C₄)-Alkyl-(C₃-C₈)-cycloalkylamino, (C₂-C₄)-Alkenyl-(C₃-C₈)-cycloalkylamino, (C₂-C₄)-Alkinyl-(C₃-C₈)-cycloalkylamino, (C₁-C₄)-Alkyl-(C₄-C₈)-cycloalkenylamino, (C₂-C₄)-Alkenyl-(C₄-C₈)-cycloalkenylamino, die N-(C₁-C₄)-Alkylamino-Analogen der vierzehn letztgenannten Reste, (C₁-C₁₀)-Trialkylsilyl, Aryl, Aroyl, Heterocyclylcarbonyl, Aryloxy, Arylthio, Arylamino, N-(C₁-C₄)-Alkyl-arylamino, die N-(C₁-C₄)-Alkylamino-Analogen der zwei letztgenannten Reste, Aryl-(C₁-C₄)-alkoxy, Aryl-(C₃-C₄)-alkenyloxy, Aryl-(C₁-C₄)-alkylthio, Aryl-(C₃-C₄)-alkenylthio, Aryl-(C₁-C₄)-alkylamino, Aryl-(C₃-C₄)-alkenylamino, das N-(C₁-C₄)-Alkylamino-Analoge des letztgenannten Restes, Aryl-(C₁-C₈)-dialkylsilyl, Diaryl-(C₁-C₈)-alkylsilyl, Triarylsilyl, Heterocyclyl, Heterocyclyloxy, Heterocyclylthio, oder Heterocyclylamino bedeutet, wobei der cyclische Teil der genannten Aryl- oder Heterocyclylreste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Dialkylamino, Trimethylsilyl oder (C₁-C₄)-Alkanoyl substituiert ist;
wobei gegebenenfalls zwei Alkylreste R⁶, wenn sie an das gleiche Kohlenstoffatom gebunden sind, zusammen mit diesem Kohlenstoffatom ein spirocyclisches Ringsystem bilden können, oder
gegebenenfalls zwei Alkylreste R⁶, die an verschiedene Kohlenstoffatome gebunden sind, zusammen mit dem aliphatischen Heterocyclus der Formel (II) ein kondensiertes oder bicyclisches Ringsystem bilden oder weiterhin das heteroaliphatische Ringsystem der Formel (II) und ein zusätzliches Aryl- oder Heteroaryl-System zusammen ein kondensiertes Ringsystem bilden;
und, falls R⁶ (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl bedeutet, die genannten Reste gegebenenfalls ein- oder mehrfach durch Reste R¹¹, substituiert sein können
R¹¹ die oben zu R⁷ angegebenen Bedeutungen hat, und gegebenenfalls in allen zu R⁶, R⁷ und R¹¹ genannten Gruppen an Kohlenstoff gebundener Wasserstoff durch bis zu drei Halogenatome, im Falle von Fluor auch bis zu Maximalanzahl, ersetzt sein kann.

4. Amide der Formel (I) und deren Salze nach Anspruch 3, wobei A und/oder B eine mindestens ein -O-, -S(O)_{0.1.2}-, -NR⁸- oder -SiR⁹R¹⁰- Ringatom enthaltende Einheit bedeutet, worin
R⁸ Wasserstoff, (C₁-C₁₀)-Alkyl, (C₃-C₁₀)-Alkenyl, (C₃-C₁₀)-Alkinyl, (C₃-C₁₀)-Cycloalkyl, (C₄-C₁₀)-Cycloalkenyl, Aryl, Heterocyclyl, (C₁-C₁₀)-Alkanoyl, (C₃-C₁₀)Alkenoyl, (C₃-C₁₀)-Alkinoyl, (C₄-C₈)-Cycloalkanoyl, Aroyl, Heterocyclylcarbonyl, Carbamoyl, (C₁-C₆)-Mono- oder -Dialkylcarbamoyl, (C₃-C₁₀)-Mono- oder -Dicycloalkylcarbamoyl, (C₁-C₁₀)-Alkoxycarbonyl, (C₃-C₁₀)-Cycloalkoxycarbonyl, (C₁-C₁₀)- Alkylsulfonyl, (C₃-C₁₀)-Alkenylsulfonyl, (C₃-C₆)-Alkinylsulfonyl, (C₃-C₁₀)-Cycloalkylsulfonyl, (C₄-C₁₀)-Cycloalkenylsulfonyl, (C₃-C₁₀)-Cycloalkyl-(C₁-C₁₀)-Alkylsulfonyl, (C₄-C₈)-Cycloalkenyl-(C₁-C₄)-alkylsulfonyl, (C₃-C₈)-Cycloalkyl-(C₃-C₄)-alkenylsulfonyl, (C₄-C₈)-Cycloalkenyl-(C₃-C₄)-alkenylsulfonyl, (C₁-C₄)-Alkyl-(C₃-C₈)-cycloalkylsulfonyl, (C₂-C₄)-Alkenyl-(C₃-C₈)-cycloalkylsulfonyl, (C₂-C₄)-Alkinyl-(C₃-C₈)-cycloalkylsulfonyl, (C₁-C₄)-alkyl-(C₄-C₈)-cycloalkenylsulfonyl, (C₂-C₄)-Alkenyl-(C₄-Cₐ)-cycloalkenylsulfonyl, Hydroxy, (C₁-C₁₀)-Alkoxy, (C₃-C₁₀)-Alkenyloxy, (C₃-C₁₀)-Alkinyloxy, (C₃-C₁₀)-Cycloalkoxy, (C₄-C₁₀)-Cycloalkenyloxy, (C₃-C₈)-Cycloalkyl-(C₁-C₄)-alkoxy, (C₄-C₈)-Cycloalkenyl-(C₁-C₄)-alkoxy, (C₃-C₈)-Cycloalkyl-(C₃-C₄)-alkenyloxy, (C₄-C₈)-Cycloalkenyl-(C₃-C₄)-alkenyloxy, (C₁-C₄)-Alkyl-(C₃-C₈)-cycloalkoxy, (C₂-C₄)-Alkenyl-(C₃-C₈)-cycloalkoxy, (C₂-C₄)-Alkinyl-(C₃-C₈)-cycloalkoxy, (C₁-C₄)-Alkyl-(C₄-C₈)-cycloalkenyloxy, (C₂-C₄)-Alkenyl-(C₄-C₈)-cycloalkenyloxy, (C₁-C₄)-Alkoxy-(C₁-C₄)-alkoxy, (C₁-C₄)-Alkoxy-(C₃-C₄)-alkenyloxy, Aryloxy, Aryl-(C₁-C₁₀)-alkoxy, Aryl-(C₃-C₁₀)-alkenyloxy oder Aryl-(C₃-C₁₀)-alkinyloxy bedeutet, und die vorstehend zu R⁸ genannten Reste gegebenenfalls durch einen oder mehrere Reste aus der Gruppe Cyano, Nitro, Amino, Hydroxy, Thio, (C₁-C₄)-Alkyl, (C₃-C₁₀)-Cycloalkyl, (C₁-C₄)-Alkoxy, (C₁-C₄)-Alkylthio, (C₁-C₄)-Alkylamino oder (C₁-C₄)-Alkanoyl substituiert sein können; R⁹ und R'° (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl, (C₈-C₁₀)-Cycloalkinyl, Aryl, Aryl-(C₁-C₄)-alkyl oder Heterocyclyl bedeutet
und gegebenenfalls in allen zu R⁸, R⁹ und R¹⁰ genannten Gruppen an Kohlenstoff gebundener Wasserstoff durch ein bis drei Halogenatome, im Falle von Fluor auch bis zur Maximalanzahl, ersetzt sein kann.

5. Amide der Formel (I) und deren Salze nach Anspruch 3, wobei R⁵ ein fünf- bis siebengliedriger nicht-aromatischer Heterocyclus der Formel (II) mit ein oder zwei Heteroatomeinheit(en) A und/oder B und zusätzlich einer Carbonylgruppe im Ring ist, die zusammen mit der oder den Heteroeinheit(en) eine Lacton- oder Lactam-Einheit bildet.

6. Amide der Formel (I) und deren Salze nach Anspruch 3, wobei R⁶ (C₁-C₁₀)-Alkyl, (C₂-C₁₀)-Alkenyl, (C₂-C₁₀)-Alkinyl, (C₃-C₁₀)-Cycloalkyl, (C₄-C₁₀)-Cycloalkenyl oder (C₈-C₁₀)-Cycloalkinyl bedeutet und die genannten Reste gegebenenfalls ein- oder mehrfach durch Reste R¹¹ substituiert sind, wobei R¹¹ eine der in Anspruch 3 angegebenen Bedeutungen hat.

7. Amide der Formel (I) und deren Salze nach Anspruch 3, wobei R⁷ Aryl, Aroyl, Heterocyclylcarbonyl, Aryloxy, Arylthio, Arylamino, N-(C₁-C₄)-Alkyl-arylamino, die N-(C₁-C₄)-Alkylamino-Analogen der zwei letztgenannten Reste, Aryl-(C₁-C₄)-alkoxy, Aryl-(C₃-C₄)-alkenyloxy, Aryl-(C₁-C₄)-alkylthio, Aryl-(C₁-C₄)-alkenylthio, Aryl-(C₁-C₄)-alkylamino, Aryl-(C₃-C₄)-alkenylamino, das N-(C₁-C₄)-Alkylamino-Analoge des letztgenannten Restes, Aryl-(C₁-C₈)-dialkylsilyl, Diaryl-(C₁-C₈)-alkylsilyl, Triarylsilyl, Heterocyclyl, Heterocyclyloxy, Heterocyclylthio und Heterocyclylamino bedeutet und der cyclische Teil der genannten Aryl- oder Heterocyclylreste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Alkylthio, (C₁-C₄)-Alkylarnino oder (C₁-C₄)-Alkanoyl substituiert ist.

8. Amide der Formel (I) und deren Salze nach einem oder mehreren der Ansprüche 1 bis 7, wobei X -CH- ist, Y -O- bedeutet und n 0 ist.

9. Amide der Formel (I) und deren Salze nach einem oder mehreren der Ansprüche 1 bis 8, wobei R¹ ein- oder mehrfach durch F und/oder Cl substituiertes (C₁-C₄)-Alkyl ist.

10. Amide der Formel (I) und deren Salze nach Anspruch 9, wobei R¹ CF₃, CHF₂ oder CF₂Cl ist.

11. Verfahren zur Herstellung der Amide der Formel (I) und deren Salze nach einem oder mehreren der Ansprüche 1 bis 10, umfassend die Umsetzung einer Carbonsäure der Formel (III), 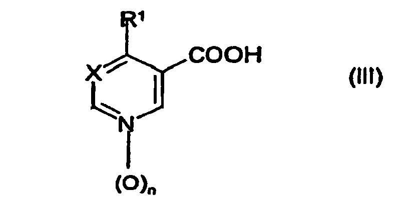 worin X, R¹ und n die in Anspruch 1 angegebenen Bedeutungen haben, in Form eines aktivierten Derivats dieser Säure, in Gegenwart einer Base mit einer Verbindung der Formel (IV), worin R⁴ und R⁵ die in Anspruch 1 angegebenen Bedeutungen haben
HNR⁴R⁵ (IV)

12. Mittel mit insektizider, akarizider, ixodizider, nematizider, molluskizider und/oder fungizider Wirkung **gekennzeichnet durch** einen Gehalt an mindestens einer Verbindung der Formel (I) oder einem ihrer Salze nach einem oder mehreren der Ansprüche 1 bis 10.

13. Mittel nach Anspruch 12 in Mischung mit Träger- und/oder oberflächenaktiven Stoffen.

14. Verwendung der Verbindungen der Formel (I) oder ihrer Salze nach einem oder mehreren der Ansprüche 1 bis 10 zur Bekämpfung von Schadorganismen aus der Gruppe Insekten und Spinnentiere, ausgenommen Verwendungen zur Behandlung des menschlichen oder tierischen Körpers.

15. Verwendung nach Anspruch 14 zur Bekämpfung von Schadorganismen in transgenen Kulturpflanzen.

16. Verwendung der Verbindungen der Formel (I) oder ihrer Salze nach einem oder mehreren der Ansprüche 1 bis 10, zur Abwehr oder Vertreibung von Schädlingen und/oder Lästlingen.

17. Verwendung der Verbindungen der Formel (I) oder ihrer Salze nach einem oder mehreren der Ansprüche 1 bis 10, zur Herstellung eines Humanarzneimittels und/oder eines Tierarzneimittels.

18. Verwendung der Verbindungen der Formel (I) oder ihrer Salze nach einem oder mehreren der Ansprüche 1 bis 10, zur Herstellung eines Arzneimittels zur Bekämpfung von von Endo- und Ektoparasiten.

19. Verfahren zur Bekämpfung von schädlichen Insekten, Spinnentieren und/oder Nematoden, wobei man eine wirksame Menge einer Verbindung der Formel (I) oder ihrer Satze nach einem oder mehreren der Ansprüche 1 bis 10 auf die Schädlinge oder der Ort der gewünschten Wirkung appliziert, ausgenommen Verfahren zur Behandlung des menschlichen oder tierischen Körpers.

20. Verfahren zur Abwehr oder zur Vertreibung von Schadorganismen, wobei man eine oder mehrere Verbindungen der Formel (I) oder deren Salze nach einem oder mehreren der Ansprüche 1 bis 10 an dem Ort ausbringt, von dem die Schadorganismen ferngehalten oder vertrieben werden sollen.

## Claims

1. Amides of Formula (I) and their salts, 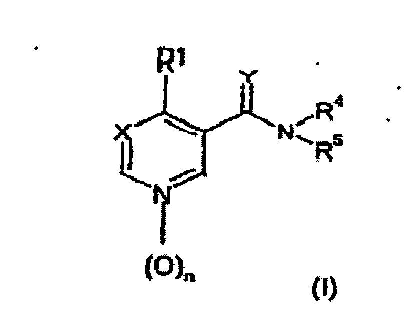 wherein the symbols and indices have the following meanings:
X is CH or N;
Y is O or S;
n is 0 or 1;
R¹ is (C₁-C₄)-haloalkyl;
R⁴ is hydrogen, (C₁-C₁₀)-alkyl, (C₃-C₁₀)-cycloalkyl, (C₃-C₁₀)-alkenyl, or (C₃-C₁₀)-alkinyl, wherein in the named alkyl, cycloalkyl, alkenyl or alkinyl groups up to three hydrogen atoms can be replaced by halogen, in the case of fluorine also up to the maximum number;
R⁵ is a non-aromatic heterocycle containing at least one oxygen, sulphur, nitrogen and/or silicon ring atom which is optionally substituted with a group with a valency of up to six, and which can optionally be part of a spirocyclic, condensed or bicyclic ring system.

2. Amides of Formula (I) and their salts according to claim 1, **characterized in that** R⁵ is a four- to eight-membered non-aromatic heterocycle which has an oxygen ring atom, a sulphur ring atom, a nitrogen ring atom, a silicon ring atom, two oxygen ring atoms or two sulphur ring atoms, wherein the sulphur ring atoms are present in the form -S(O)ₚ-, and p denotes 0, 1 or 2.

3. Amides of Formula (I) and their salts according to claim 1 or 2, wherein R⁵ is a non-aromatic heterocycle of Formula (II), 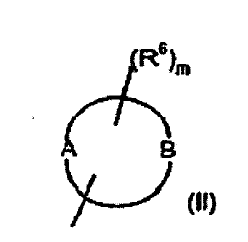 in which A and/or B denotes at least one oxygen, sulphur, nitrogen or silicon ring atom, and the ring can additionally contain one or two carbonyl groups which together with the hetero units can optionally form a lactone, lactam or imide unit; and, if A denotes oxygen and B nitrogen, these units can be directly adjacent and in all other cases, in which A and B denote hetero atom units, they must be separated by at least one saturated carbon unit;
m denotes 0 or 1 to 6,
R⁶ dependent on m is identical or different and in each case denotes (C₁-C₁₀)-alkyl, (C₂-C₁₀)-alkenyl, (C₂-C₁₀)-alkinyl, (C₃-C₁₀)-cycloalkyl, (C₄-C₁₀)-cycloalkenyl or (C₈-C₁₀)-cycloalkinyl, which is optionally substituted with one or more identical or different radicals, or represents a radical R⁷, wherein
R⁷ denotes halogen, cyano, nitro, hydroxy, thio, amino, (C₁-C₁₀)-alkanoyl, (C₃-C₁₀)-alkenoyl, (C₃-C₁₀)-alkinoyl, (C₃-C₁₀)-cycloalkanoyl, (C₁-C₁₀)-alkoxy, (C₃-C₁₀)-alkenyloxy, (C₃-C₁₀)-alkinyloxy, (C₃-C₁₀)-cycloalkoxy, (C₄-C₁₀)-cycloalkenyloxy, (C₃-C₈)-cycloalkyl-(C₁-C₄)-alkoxy, (C₄-C₈)-cycloalkenyl-(C₁-C₄)-alkoxy, (C₃-C₈)-cycloalkyl-(C₃-C₄)-alkenyloxy, (C₄-C₈)-cycloalkenyl-(C₃-C₄)-alkenyloxy, (C₁-C₄)-alkyl-(C₃-C₈)-cycloalkoxy, (C₂-C₄)-alkenyl-(C₃-C₈)-cycloalkoxy, (C₂-C₄)-alkinyl-(C₃-C₈)-cycloalkoxy, (C₁-C₄)-alkyl-(C₄-C₈)-cycloalkenyloxy, (C₂-C₄)-alkenyl-(C₄-C₈)-cycloalkenyloxy, (C₁-C₄)-alkoxy-(C₁-C₄)-alkoxy, (C₁-C₄)-alkoxy-(C₃-C₄)-alkenyloxy, carbamoyl, (C₁-C₈)-mono- or dialkylcarbamoyl, (C₃-C₈)-mono- or dicycloalkylcarbamoyl, (C₁-C₈)-alkoxycarbonyl, (C₃-C₈)-cycloalkoxycarbonyl, (C₁-C₈)-alkanoyloxy, (C₃-C₈)-cycloalkanoyloxy, (C₁-C₈)-alkanoylamino, (C₃-C₈)-alkenoylamino, (C₃-C₈)-cycloalkanoylamino, (C₃-C₈)-cycloalkyl-(C₁-C₄)-alkanoylamino, the N-(C₁-C₄)-alkylamino analogues of the four last-named radicals, (Cₗ-Cₗₒ)-alkylthio, (C₃-C₁₀)-alkenylthio, (C₃-C₁₀)-alkinylthio, (C₃-C₈)-cycloalkylthio, (C₄-C₈)-cycloalkenylthio, (C₃-C₈)-cycloalkyl-(C₁-C₄)-alkylthio, (C₄-C₈)-cycloalkenyl-(C₁-C₄)-alkylthio, (C₃-C₈)-cycloalkyl-(C₃-C₄)-alkenylthio, (C₄-C₈)-cycloalkenyl-(C₃-C₄)-alkenylthio, (C₁-C₄)-alkyl-(C₃-C₈)-cycloalkylthio, (C₂-C₄)-alkenyl-(C₃-C₈)-cycloalkylthio, (C₂-C₄)-alkinyl-(C₃-C₈)-cycloalkylthio, (C₁-C₈)-alkyl-(C₄-C₈)-cycloalkenylthio, (C₂-C₆)-alkenyl-(C₄-C₈)-cycloalkenylthio, (C₁-C₁₀)-alkylsulphinyl, (C₃-C₄)-alkenylsulphinyl, (C₃-C₄)-alkinylsulphinyl, (C₃-C₁₀)-cycloalkylsulphinyl, (C₄-C₁₀)-cycloalkenylsulphinyl, (C₃-C₈)-cycloalkyl-(C₁-C₄)-alkylsulphinyl, (C₄-C₈)-cycloalkenyl-(C₁-C₄)-alkylsulphinyl, (C₃-C₈)-cycloalkyl-(C₃-C₄)-alkenylsulphinyl, (C₄-C₈)-cycloalkenyl-(C₃-C₄)-alkenylsulphinyl, (C₁-C₄)-alkyl-(C₃-C₈)-cycloalkylsulphinyl, (C₂-C₄)-alkenyl-(C₃-C₈)-cycloalkylsulphinyl, (C₂-C₄)-alkinyl-(C₃-C₈)-cycloalkylsulphinyl, (C₁-C₄)-alkyl-(C₄-C₈)-cycloalkenylsulphinyl, (C₂-C₃)-alkenyl-(C₄-C₈)-cycloalkenylsulphinyl, (C₁-C₁₀)-alkylsulphonyl, (C₃-C₁₀)-alkenylsulphonyl, (C₃-C₁₀)-alkinylsulphonyl, (C₃-C₁₀)-cycloalkylsulphonyl, (C₄-C₁₀)-cycloalkenylsulphonyl, (C₃-C₈)-cycloalkyl-(C₁-C₄)-alkylsulphonyl, (C₄-C₈)-cycloalkenyl-(C₁-C₄)-alkylsulphonyl, (C₃-C₈)-cycloalkyl-(C₃-C₄)-alkenylsulphonyl, (C₄-C₈)-cycloalkenyl-(C₃-C₄)-alkenylsulphonyl, (C₁-C₄)-alkyl-(C₃-C₈)-cycloalkylsulphonyl, (C₂-C₄)-alkenyl-(C₃-C₈)-cycloalkylsulphonyl, (C₃-C₆)-alkinyl-(C₃-C₈)-cycloalkylsulphonyl, (C₁-C₄)-alkyl-(C₄-C₈)-cycloalkenylsulphonyl, (C₃-C₄)-alkenyl-(C₄-C₈)-cycloalkenylsulphonyl, (C₁-C₁₀)-alkylamino, (C₃-C₁₀)-alkenylamino, (C₃-C₁₀)-alkinylamino, (C₃-C₈)-cycloalkylamino, (C₄-C₈)-cycloalkenylamino, (C₃-C₈)-cycloalkyl-(C₁-C₄)-alkylamino, (C₄-C₈)-cycloalkenyl-(C₁-C₄)-alkylamino, (C₃-C₈)-cycloalkyl-(C₃-C₄)-alkenylamino, (C₄-C₈)-cycloalkenyl-(C₃-C₄)-alkenylamino, (C₁-C₄)-alkyl-(C₃-C₈)-cycloalkylamino, (C₂-C₄)-alkenyl-(C₃-C₈)-cycloalkylamino, (C₂-C₄)-alkinyl-(C₃-C₈)-cycloalkylamino, (C₁-C₄)-alkyl-(C₄-C₈)-cycloalkenylamino, (C₂-C₄)-alkenyl-(C₄-C₈)-cycloalkenylamino, the N-(C₁-C₄)-alkylamino analogues of the fourteen last-named radicals, (C₁-C₁₀)-trialkylsilyl, aryl, aroyl, heterocyclylcarbonyl, aryloxy, arylthio, arylamino, N-(C₁-C₄)-alkylarylamino, the N-(C₁-C₄)-alkylamino analogues of the two last-named radicals, aryl-(C₁-C₄)-alkoxy, aryl-(C₃-C₄)-alkenyloxy, aryl-(C₁-C₄)-alkylthio, aryl-(C₃-C₄)-alkenylthio, aryl-(C₁-C₄)-alkylamino, aryl-(C₃-C₄)-alkenylamino, the N-(C₁-C₄)-alkylamino analogues of the last-named radical, aryl-(C₁-C₈)-dialkylsilyl, diaryl-(C₁-C₈)-alkylsilyl, triarylsilyl, heterocyclyl, heterocyclyloxy, heterocyclylthio, or heterocyclylamino, wherein the cyclic part of the named aryl or heterocyclyl radicals is optionally substituted by one or more radicals from the group halogen, cyano, nitro, amino, hydroxy, thio, (C₁-C₄)-alkyl, (C₁-C₄)-haloalkyl, (C₃-C₈)-cycloalkyl, (C₁-C₄)-alkoxy, (C₁-C₄)-haloalkoxy, (C₁-C₄)-alkylthio, (C₁-C₄)-haloalkylthio, (C₁-C₄)-alkylamino, (C₁-C₄)-dialkylamino, trimethylsilyl or (C₁-C₄)-alkanoyl;
wherein optionally two alkyl radicals R⁶, if they are bonded to the same carbon atom, can form together with this carbon atom a spirocyclic ring system, or
optionally two alkyl radicals R⁶, which are bonded to different carbon atoms, form together with the aliphatic heterocycle of Formula (II) a condensed or bicyclic ring system or furthermore the heteroaliphatic ring system of Formula (II) and an additional aryl or heteroaryl system together form a condensed ring system;
and, if R⁶ denotes (C₁-C₁₀)-alkyl, (C₂-C₁₀)-alkenyl, (C₂-C₁₀)-alkinyl, (C₃-C₁₀)-cycloalkyl, (C₄-C₁₀)-cycloalkenyl or (C₈-C₁₀)-cycloalkinyl, the named radicals can optionally be substituted one or more times, preferably by radicals R¹¹,
R¹¹ has the meanings given above for R⁷, and optionally in all groups named for R⁶, R⁷ and R¹¹ hydrogen bonded to carbon can be replaced by up to three halogen atoms, in the case of fluorine also up to the maximum number.

4. Amides of Formula (I) and their salts according to claim 3, wherein A and/or B denotes a unit containing at least one -O-, -S(O)_{0,1,2}-, -NR⁸- or -SiR⁹R¹⁰- ring atom, in which
R⁸ denotes hydrogen, (C₁-C₁₀)-alkyl, (C₃-C₁₀)-alkenyl, (C₃-C₁₀)-alkinyl, (C₃-C₁₀)-cycloalkyl, (C₄-C₁₀)-cycloalkenyl, aryl, heterocyclyl, (C₁-C₁₀)-alkanoyl, (C₃-C₁₀)-alkenoyl, (C₃-C₁₀)-alkinoyl, (C₄-C₈)-cycloalkanoyl, aroyl, heterocyclylcarbonyl, carbamoyl, (C₁-C₆)-mono- or dialkylcarbamoyl, (C₃-C₁₀)-mono- or dicycloalkylcarbamoyl, (C₁-C₁₀)-alkoxycarbonyl, (C₃-C₁₀)-cycloalkoxycarbonyl, (C₁-C₁₀)-alkylsulphonyl, (C₃-C₁₀)-alkenylsulphonyl, (C₃-C₆)-alkinylsulphonyl, (C₃-C₁₀)-cycloalkylsulphonyl, (C₄-C₁₀)-cycloalkenylsulphonyl, (C₃-C₁₀)-cycloalkyl-(C₁-C₁₀)-alkylsulphonyl, (C₄-C₈)-cycloalkenyl-(C₁-C₄)-alkylsulphonyl, (C₃-C₈)-cycloalkyl-(C₃-C₄)-alkenylsulphonyl, (C₄-C₈)-cycloalkenyl-(C₃-C₄)-alkenylsulphonyl, (C₁-C₄)-alkyl-(C₃-C₈)-cycloalkylsulphonyl, (C₂-C₄)-alkenyl-(C₃-C₈)-cycloalkylsulphonyl, (C₂-C₄)-alkinyl-(C₃-C₈)-cycloalkylsulphonyl, (C₁-C₄)-alkyl-(C₄-C₈)-cycloalkenylsulphonyl, (C₂-C₄)-alkenyl-(C₄-C₈)-cycloalkenylsulphonyl, hydroxy, (C₁-C₁₀)-alkoxy, (C₃-C₁₀)-alkenyloxy, (C₃-C₁₀)-alkinyloxy, (C₃-C₁₀)-cycloalkoxy, (C₄-C₁₀)-cycloalkenyloxy, (C₃-C₈)-cycloalkyl-(C₁-C₄)-alkoxy, (C₄-C₈)-cycloalkenyl-(C₁-C₄)-alkoxy, (C₃-C₈)-cycloalkyl-(C₃-C₄)-alkenyloxy, (C₄-C₈)-cycloalkenyl-(C₃-C₄)-alkenyloxy, (C₁-C₄)-alkyl-(C₃-C₈)-cycloalkoxy, (C₂-C₄)-alkenyl-(C₃-C₈)-cycloalkoxy, (C₂-C₄)-alkinyl-(C₃-C₈)-cycloalkoxy, (C₁-C₄)-alkyl-(C₄-C₈)-cycloalkenyloxy, (C₂-C₄)-alkenyl-(C₄-C₈)-cycloalkenyloxy, (C₁-C₄)-alkoxy-(C₁-C₄)-alkoxy, (C₁-C₄)-alkoxy-(C₃-C₄)-alkenyloxy, aryloxy, aryl-(C₁-C₁₀)-alkoxy, aryl-(C₃-C₁₀)-alkenyloxy or aryl-(C₃-C₁₀)-alkinyloxy, and the radicals named above for R⁸ can optionally be substituted by one or more radicals from the group cyano, nitro, amino, hydroxy, thio, (C₁-C₄)-alkyl, (C₃-C₁₀)-cycloalkyl, (C₁-C₄)-alkoxy, (C₁- C₄)-alkylthio, (C₁-C₄)-alkylamino or (C₁-C₄)-alkanoyl; R⁹ and R¹⁰ denote (C₁-C₁₀)-alkyl, (C₂-C₁₀)-alkenyl, (C₂-C₁₀)-alkinyl, (C₃-C₁₀)-cycloalkyl, (C₄-C₁₀)-cycloalkenyl, (C₈-C₁₀)-cycloalkinyl, aryl, aryl-(C₁-C₄)-alkyl or heterocyclyl,
and optionally in all groups named for R⁸, R⁹ and R¹⁰ hydrogen bonded to carbon can be replaced by one to three halogen atoms, in the case of fluorine also up to the maximum number.

5. Amides of Formula (I) and their salts according to claim 3, wherein R⁵ is a five- to seven-membered non-aromatic heterocycle with one or two hetero atom units A and/or B, and also a carbonyl group in the ring, which forms together with the hetero unit or hetero units a lactone or lactam unit.

6. Amides of Formula (I) and their salts according to claim 3, wherein R⁶ denotes (C₁-C₁₀)-alkyl, (C₂-C₁₀)-alkenyl, (C₂-C₁₀)-alkinyl, (C₃-C₁₀)-cycloalkyl, (C₄-C₁₀)-cycloalkenyl or (C₈-C₁₀)-cycloalkinyl and the named radicals can optionally be substituted one or more times by radicals R¹¹, wherein R¹¹ has the meanings given in claim 3.

7. Amides of Formula (I) and their salts according to claim 3, wherein R⁷ denotes aryl, aroyl, heterocyclylcarbonyl, aryloxy, arylthio, arylamino, N-(C₁-C₄)-alkylarylamino, the N-(C₁-C₄)-alkylamino analogues of the two last-named radicals, aryl-(C₁-C₄)-alkoxy, aryl-(C₃-C₄)-alkenyloxy, aryl-(C₁-C₄)-alkylthio, aryl-(C₃-C₄)-alkenylthio, aryl-(C₁-C₄)-alkylamino, aryl-(C₃-C₄)-alkenylamino, the N-(C₁-C₄)-alkylamino analogues of the last-named radical, aryl-(C₁-C₈)-dialkylsilyl, diaryl-(C₁-C₈)-alkylsilyl, triarylsilyl, heterocyclyl, heterocyclyloxy, heterocyclylthio, and heterocyclylamino and the cyclic part of the named aryl or heterocyclyl radicals is optionally substituted by one or more radicals from the group halogen, cyano, nitro, amino, hydroxy, thio, (C₁-C₄)-alkyl, (C₁-C₄)-haloalkyl, (C₃-C₈)-cycloalkyl, (C₁-C₄)-alkoxy, (C₁-C₄)-alkylthio, (C₁-C₄)-alkylamino or (C₁-C₄)-alkanoyl.

8. Amides of Formula (I) and their salts according to one or more of claims 1 to 7, wherein X is -CH-, Y denotes -O- and n is 0.

9. Amides of Formula (I) and their salts according to one or more of claims 1 to 8, wherein R¹ is (C₁-C₄)-alkyl substituted one or more times by F and/or Cl.

10. Amides of Formula (I) and their salts according to claim 9, wherein R¹ is CF₃, CHF₂ or CF₂Cl.

11. Process for the preparation of amides of Formula (I) and their salts according to one or more of claims 1 to 10, comprising the conversion of a carboxylic acid of Formula (III), 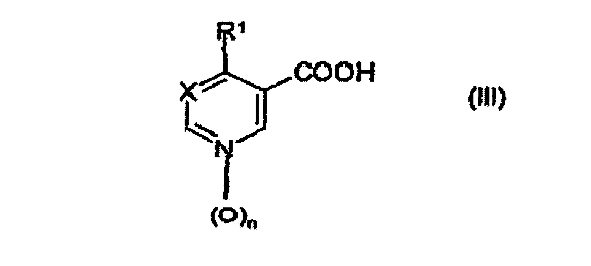 in which X, R¹ and n have the meanings given in claim 1, in the form of an activated derivative of this acid, in the presence of a base, with a compound of Formula (IV) in which R⁴ and R⁵ have the meanings given in claim 1
HNR⁴R⁵.

12. Agent with insecticidal, acaricidal, ixodicidal, nematicidal, molluscicidal or fungicidal effect, **characterized by** a content of at least one compound of Formula (I) or one of its salts according to one or more of claims 1 to 10.

13. Agent according to claim 12 mixed with carriers and/or surface-active substances.

14. Use of the compounds of Formula (I) or their salts according to one or more of claims 1 to 10 to control pest organisms from the group insects and arachnids, excluding uses for treating the human or animal body.

15. Use according to claim 14 to control pest organisms in transgenic cultivated plants.

16. Use of the compounds of Formula (I) or their salts according to one or more of claims 1 to 10, to defend against or to expel pests and/or harmful insects.

17. Use of the compounds of Formula (I) or their salts according to one or more of claims 1 to 10, for the preparation of a human medicinal product and/or an animal medicinal product.

18. Use of compounds of Formula (I) or their salts according to one or more of claims 1 to 10, for the preparation of a medicinal product to control endo- and ectoparasites.

19. Method for controlling harmful insects, arachnids and/or nematodes, wherein an effective quantity of a compound of Formula (I) or its salts according to one or more of claims 1 to 10 is applied to the pests or the site of the desired effect, excluding methods for treating the human or animal body.

20. Method to defend against or to expel pest organisms, wherein one or more compounds of Formula (I) or their salts according to one or more of claims 1 to 10 are deployed at the site from which the pest organisms are to be kept away or expelled.

## Revendications

1. Amides de formule (I) et leurs sels 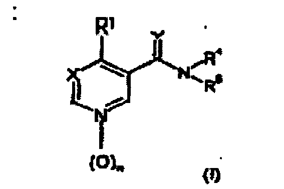 dans lesquels les symboles et indices ont les significations suivantes :
X est CH ou N ;
Y est O ou S ;
n vaut 0 ou 1 ;
R¹ est un groupe halogénoalkyle (en C₁ à C₄) ;
R⁴ est un atome d'hydrogène, un groupe alkyle (en C₁ à C₁₀), cycloalkyle (en C₃ ou C₁₀), alcényle (en C₃ à C₁₀) ou alcinyle (en C₃ à C₁₀), dans laquelle dans les groupes alkyle, cycloalkyle, alcényle ou alcinyle, jusqu'à trois atomes d'hydrogène peuvent être substitués par un atome d'halogène, dans le cas du fluor, également jusqu'au nombre maximal ;
R⁵ est un hétérocycle non aromatique contenant au moins un atome cyclique d'oxygène, de soufre, d'azote et/ou de silicium qui est éventuellement substitué par un à six groupes monovalents et qui peut éventuellement faire partie d'un système cyclique spirocyclique, condensé ou bicyclique.

2. Amides de formule (I) et leurs sels selon la revendication 1, **caractérisés en ce que** R⁵ est un hétérocycle non aromatique de quatre à huit chaînons qui présente un atome cyclique d'oxygène, un atome cyclique de soufre, un atome cyclique d'azote, un atome cyclique de silicium, deux atomes cycliques d'oxygène ou deux atomes cycliques de soufre, les atomes cycliques de soufre se présentant sous la forme - S(O)ₚ et p représentant 0,1 à 2.

3. Amides de formule (I) et leurs sels selon la revendication 1 ou 2, dans lesquels R⁵ est un hétérocycle non aromatique de formule (II) 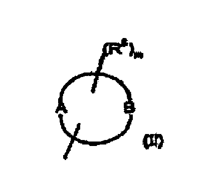 dans laquelle A et/ou B désignent au moins un atome d'oxygène, de soufre, d'azote ou de silicium, et le cycle peut comprendre en outre un ou deux groupes carbonyle qui peuvent former conjointement avec les motifs hétéro, un motif lactone, lactame ou imide ; et si A désigne un atome d'oxygène et B un atome d'azote, ces motifs peuvent être immédiatement voisins et dans tous les autres cas dans lesquels A et B désignent des hétéro-atomes, ceux-ci doivent être séparés par au moins un atome de carbone saturé ;
m vaut 0 ou 1 à 6 ;
chaque R⁶, en fonction de m, identique ou différent, désigne un groupe alkyle (en C₁ à C₁₀), alcényle (en C₂ à C₁₀), alcinyle (en C₂ à C₁₀), cycloalkyle (en C₃ à C₁₀), cycloalcényle (en C₄ à C₁₀) ou cycloalcinyle (en C₈ à C₁₀), qui peut être substitué par un ou plusieurs radicaux identiques ou différents ou représente un radical R⁷, sachant que
R⁷ désigne un atome d'halogène, un groupe cyano, nitro, hydroxy, thio, amino, alcanoyle (en C₁ à C₁₀), alcénoyl (en C₃ à C₁₀), alcinoyle (en C₃ à C₁₀), cycloalcanoyle (en C₃ à C₁₀), alcoxy (en C₁ à C₁₀), alcényloxy (en C₃ à C₁₀), alcinyloxy (en C₃ à C₁₀), cycloalcoxy (en C₃ à C₁₀), cycloalcényloxy (en C₄ à C₁₀), cycloalkyle (en C₃ à C₈)-alcoxy (en C₁ à C₄), cycloalcényle (en C₄ à C₈)-alcoxy (en C₁ à C₄), cycloalkyle (en C₃ à C₄)-alcényloxy (en C₃ à C₄), cycloalcényle (en C₄ à C₈)-alcényloxy (en C₃ à C₄), alkyle (en C₁ à C₄)-cyclo-alcoxy (en C₃ à C₈), alcényle (en C₂ à C₄)-cyclo-alcoxy (en C₃ à C₈), alcinyle (en C₂ à C₄)-cycloalcoxy (en C₃ à C₈), alkyle (en C₁ à C₄)-cycloalcényloxy (en C₄ à C₈), alcényle (en C₂ à C₄)-cycloalcényloxy (en C₄ à C₈), alcoxy (en C₁ à C₄)-alcoxy (en C₁ à C₄), alcoxy (en C₁ à C₄)-alcényloxy (en C₃ à C₄), carbamoyle, mono- ou di-alkyle (en C₁ à C₈)-carbamoyle, mono ou dicycloalkyle (en C₃ à C₈)-carbamoyle, alcoxycarbonyle (en C₁ à C₈), cyclo-alcoxycarbonyle (en C₃ à C₈), alcanoyloxy (en C₁ à C₈), cycloalcanoyloxy (en C₃ à C₈), alcénoyamino (en C₁ à C₈), alcanoylamino (en C₃ à C₈), cyclo-alcanoyl-amino (en C₃ à C₈), cycloalkyle (en C₃ à C₈)-alcanoyle (en C₁ à C₄)-amino, les analogues N-alkylamino (en C₁ à C₄) des quatre derniers radicaux cités, alkylthio (en C₁ à C₁₀), alcénylthio (en C₃ à C₁₀), alcinylthio (en C₃ à C₁₀), cycloalkylthio (en C₃ à C₈), cycloalcénylthio (en C₄ à C₈), cyclo-alkyle (en C₃ à C₈)-alkylthio (en C₁ à C₄), cycloalcényle (en C₄ à C₈)-alkylthio (en C₁ à C₄), cyclo-alkyle (en C₃ à C₈)-alcénylthio (en C₃ à C₄), cycloalcényle (en C₄ à C₈)-alcénylthio (en C₃ à ₄), alkyle (en C₁ à C₄)-cyloalkylthio (en C₃ à C₈), alcényle (en C₂ à C₄)-cyclo-alkylthio (en C₃ à C₈), alcinyle (en C₂ à C₄)-cyclo-alkylthio (en C₃ à C₈), alkyle (en C₁ à C₈)-cyclo-alcénylthio (en C₄ à C₈), alcényle (en C₂ à C₈)-cyclo-alcénylthio (en C₄ à C₈), alkylsulfinyle (en C₁ à C₁₀), alcénylsulfinyle (en C₃ à C₄), alcinylsulfinyle (en C₃ à C₄), cyclo-alkylsulfinyle (en C₃ à C₁₀), cycloalcénylsulfinyle (en C₄ à C₁₀), cyclo-alkyle (en C₃ à C₈)-alkylsulfinyle (en C₁ à C₄), cycloalcényle (en C₄ à C₈)-alkylsulfinyle (en C₁ à C₄), cycloalkyle (en C₃ à C₈)-alcénylsulfinyle (en C₃ à C₄), cyclo-alcényle (en C₄ à C₈)-alcénylsulfinyle (en C₃ à C₄), alkyle (en C₁ à C₄)-cyclo-alkylsulfinyle (en C₃ à C₈), alcényle (en C₂ à C₄)-cycloalkylsulfinyle (en C₃ à C₈), alcinyle (en C₂ à C₄)-cycloalkylsulfinyle (en C₃ à C₈), alkyle (en C₁ à C₄)-cycloalcénylsulfinyle (en C₄ à C₈), alcényle (en C₂ à C₃)-cyclo-alcénylsulfinyle (en C₄ à C₈), alkylsulfonyle (en C₁ à C₁₀), alcénylsulfonyle (en C₃ à C₁₀), alcinylsulfonyle (en C₃ à C₁₀), cyclo-alkylsulfonyle en (en C₃ à C₁₀), cyclo-alcénylsulfonyle (en C₄ à C₁₀), cyclo-alkyle (en C₃ à C₈)-akylsulfonyle (en C₁ à C₄), cyclo-alcényle (en C₄ à C₈)-alkylsulfonyle (en C₁ à C₄), cyclo-alkyle (en C₃ à C₈)-alcénylsulfonyle (en C₃ à C₄), cyclo-alcényle (en C₄ à C₈)-alcénylsulfonyle (en C₃ à C₄), alkyle (en C₁ à C₄)-cycloalkylsulfonyle (en C₃ à C₈), alcényle (en C₂ à C₄)-cycloalkylsulfonyle (en C₃ à C₈), alcinyle (en C₃ à C₆)-cyclo-alkylsulfonyle (en C₃ à C₈), alkyle (en C₁ à C₄)-cyclo-alcénylsulfonyle (en C₄ à C₈), alcényle (en C₃ à C₄)-cyclo-alcénylsulfonyle (en C₄ à C₈), alkylamino (en C₁ à C₁₀), alcénylamino (en C₃ à C₁₀), alcinylamino (en C₃ à C₁₀), cyclo-alkylamino (en C₃ à C₈), cyclo-alcénylamino (en C₄ à C₈), cyclo-alkyle (en C₃ à ₈)-alkylamino (en C₁ à C₄), cyclo-alcényle (en C₄ à C₈)-alkylamino (en C₁ à C₄), cycloalkyle (en C₃ à C₈)-alcénylamino (en C₃ à C₄), cyclo-alcényle (en C₄ à C₈)-alcénylamino (en C₃ à C₄), alkyle(en C₁ à C₄)-cyclo-alkylamino (en C₃ à C₈), alcényle (en C₂ à C₄)-cyclo-alkylamino (en C₃ à C₈), alcinyle (en C₂ à C₄)-cyclo-alkylamino (en C₃ à C₈), alkyle (en C₁ à C₄)-cyclo-alcénylamino (en C₄ à C₈), alcényle (en C₂ à C₄)-cyclo-alcénylamino (en C₄ à C₈), les analogues N-alkylamino en C₁ à C₄ des quatorze derniers radicaux cités, trialkylsilyle (en C₁ à C₁₀), aryle, aroyle, hétérocyclyl-carbonyle, aryloxy, arylthio, arylamino, N-alkyle (en C₁ à C₄)-arylamino, les analogues N-alkylamino (en C₁ à C₄) des deux derniers radicaux cités, arylalcoxy (en C₁ à C₄), arylalcényloxy (en C₃ à C₄), arylalkylthio (en C₁ à C₄), arylalcénylthio (en C₃ à C₄), arylalcylamino (en C₁ à C₄), aryl-alcénylamino (en C₃ à C₄), l'analogue N-alkylamino (en C₁ à C₄) du dernier radical cité, aryldialkylsilyle (en C₁ à C₈), diarylalkylsilyle (en C₁ à C₈), triarylsilyle, hétérocyclyle, hétérocyclyloxy, hétérocyclylthio ou hétérocyclylamino, la partie cyclique des radicaux aryle ou hétérocyclyle étant éventuellement substituée par un ou plusieurs radicaux du groupe comprenant un atome d'halogène, un groupe cyano, nitro, amino, hydroxy, thio, alkyle (en C₁ à C₄), halogénoalkyle (en C₁ à C₄), cycloalkyle (en C₃ à C₈), alcoxy (en C₁ à C₄), halogéno-alcoxy (en C₁ à C₄), alkylthio (en C₁ à C₄), halogéno-alkylthio (en C₁ à C₄), alkylamino (en C₁ à C₄), dialkylamino (en C₁ à C₄), triméthylsilyle ou alcanoyle (en C₁ à C₄) ;
dans laquelle deux radicaux alkyle R⁶, s'ils sont liés au même atome de carbone, peuvent former conjointement avec cet atome de carbone, un système cyclique spirocyclique, ou
, deux radicaux alkyle R⁶ qui sont liés à différents atomes de carbone, conjointement avec l'hétérocycle aliphatique de formule (II) peuvent former un système cyclique condensé ou bicyclique ou en outre le système cyclique hétéro-aliphatique de formule (II) et un système aryle ou hétéro-aryle supplémentaire peuvent former conjointement un système cyclique condensé ;
et si R⁶ représente un groupe alkyle (en C₁ à C₁₀), alcényle (en C₂ à C₁₀), alcinyle (en C₂ à C₁₀), cyclo-alkyle (en C₃ à C₁₀), cyclo-alcényle (en C₄ à C₁₀) ou cyclo-alcinyle (en C₈ à C₁₀), les radicaux cités peuvent être éventuellement mono- ou multi-substitués par les radicaux R¹¹,
R¹¹ a la signification mentionnée ci-dessus pour R⁷, et dans tous les groupes cités pour R⁶, R⁷ et R¹¹, les atomes d'hydrogène liés à des atomes de carbone peuvent être remplacés par jusqu'à trois atomes de carbone, dans le cas du fluor, également jusqu'à un nombre maximal.

4. Amides de formule (I) et leurs sels selon la revendication 3, dans lesquels A et/ou B désignent au moins un groupe -O-, -S(O)₀, _{1,2}-, -NR³- ou -SiR⁹R¹⁰- dans le cycle, où
R⁸ est un atome d'hydrogène, un groupe alkyle (en C₁ à C₁₀), alcényle (en C₃ à C₁₀), alcinyle (en C₃ à C₁₀), cycloalkyle (en C₃ à C₁₀), cyclo-alcényle (en C₄ à C₁₀), aryle, hétérocyclyle, alcanoyle (en C₁ à C₁₀), alcénoyle (en C₃ à C₁₀), alcinoyle (en C₃ à C₁₀), cycloalcanoyle (en C₄ à C₈), aroyle, hétérocyclylcarbonyle, carbamoyle, mono- ou dialkyle (en C₁ à C₆)-carbamoyle, mono- ou dicycloalkyle (en C₃ à C₁₀)-carbamoyle, alcoxycarbonyle (en C₁ à C₁₀), cycloalcoxycarbonyle (en C₃ à C₁₀), alkylsulfonyle (en C₁ à C₁₀), alcénylsulfonyle (en C₃ à C₁₀), alcinylsulfonyle (en C₃ à C₆), cyclo-alkylsulfonyle (en C₃ à C₁₀), cyclo-alkcénylsulfonyle (en C₄ à C₁₀), cycloalkyle (en C₃ à C₁₀)-alkylsulfonyle (en C₁ à C₁₀), cyclo-alcényle (en C₄ à C₈)-alkylsulfonyle (en C₁ à C₄), cyclo-alkyle (en C₃ à C₈)-alcénylsulfonyle (en C₃ à C₄), cyclo-alcényle (en C₄ à C₈)-alcénylsulfonyle (en C₃ à C₄), alkyle (en C₁ à C₄)-cycloalkylsulfonyle (en C₃ à C₈), alcényle (en C₂ à C₄)-cycloalkylsulfonyle (en C₃ à C₈), alcinyle (en C₂ à C₄)-cyclo-alkylsulfonyle (en C₃ à C₈), alkyle (en C₁ à C₄)-cycloalcénylsulfonyle (en C₄ à C₈), alcényle (en C₂ à C₄)-cyclo-alcénylsulfonyle (en C₄ à C₈), hydroxy, alcoxy (en C₁ à C₁₀), alcényloxy (en C₃ à C₁₀), alcinyloxy (en C₃ à C₁₀), cyclo-alcoxy (en C₃ à C₁₀), cyclo-alcényloxy (en C₄ à C₁₀), cycloalkyle (en C₃ à C₈)-alcoxy (en C₁ à C₄), cyclo-alcényle (en C₄ à C₈)-alcoxy (en C₁ à C₄), cycloalkyle (en C₃ à C₈)-alcényloxy (en C₃ à C₄), cycloalcényle (en C₄ à C₈)-alcényloxy (en C₃ à C₄), alkyle (en C₁ à C₄)-cyclo-alcoxy (en C₃ à C₈), alcényle (en C₂ à C₄)-cycloalcoxy (en C₃ à C₈), alcinyle (en C₂ à C₄)-cycloalcoxy (en C₃ à C₈), alkyle (en C₁ à C₄)-cyclo-alcényloxy (en C₄ à C₈), alcényle (en C₂ à C₄)-cyclo-alcényloxy (en C₄ à C₈), alcoxy (en C₁ à C₄)-alcoxy (en C₁ à C₄), alcoxy (en C₁ à C₄)-alcényloxy (en C₃ à C₄), aryloxy, arylalcoxy (en C₁ à C₁₀), arylalcényloxy (en C₃ à C₁₀) ou arylalcinyloxy (en C₃ à C₁₀) et les radicaux cités précédemment pour R⁸ peuvent être substitués par un ou plusieurs radicaux du groupe cyano, nitro, amino, hydroxy, thio, alkyle (en C₁ à C₄), cycloalkyle (en C₃ à C₁₀), alcoxy (en C₁ à C₄), alkylthio (en C₁ à C₄), alcylamino (en C₁ à C₄) ou alcanoyle (en C₁ à C₄) ;
R⁹ et R¹⁰ représentent un groupe alkyle (en C₁ à C₁₀), alcényle (en C₂ à C₁₀), alcinyle (en C₂ à C₁₀), cycloalkyle (en C₃ à C₁₀), cyclo-alcényle (en C₄ à C₁₀), cyclo-alcinyle (en C₈ à C₁₀), aryle, arylalkyle (en C₁ à C₄) ou hétérocyclyle,
et éventuellement dans tous les groupes cités pour R⁸, R⁹ et R¹⁰, les atomes d'hydrogène liés à des atomes de carbone peuvent être remplacés par un à trois atomes d'halogène, dans le cas du fluor, également jusqu'au nombre maximal.

5. Amides de formule (I) et leurs sels selon la revendication 3, dans lesquels R⁵ est un hétérocycle de formule (II) non aromatique de cinq à sept chaînons avec un ou plusieurs hétéroatomes A et/ou B et en outre, un groupe carbonyle dans le cycle, qui forme conjointement avec le ou les hétéroatomes, un motif lactone ou lactame.

6. Amides de formule (I) et leurs sels selon la revendication 3, dans lesquels R⁶ est un groupe alkyle (en C₁ à C₁₀), alcényle (en C₂ à C₁₀), alcinyle (en C₂ à C₁₀), cycloalkyle (en C₃ à C₁₀), cycloalcényle (en C₄ à C₁₀) ou cyclo-alcinyle (en C₈ à C₁₀) et les radicaux cités sont éventuellement mono- ou multi-substitués par R¹¹, R¹¹ ayant les significations mentionnées dans la revendication 3.

7. Amides de formule (I) et leurs sels selon la revendication 3, dans lesquels R⁷ représente un groupe aryle, aroyle, hétérocyclyl-carbonyle, aryloxy, arylthio, arylamino, N-alkyle (en C₁ à C₄)-arylamino, les analogues N-alkylamino (en C₁ à C₄) des deux derniers radicaux cités, aryl-alcoxy (en C₁ à C₄), arylalcényloxy (en C₃ à C₄), arylalkylthio (en C₁ à C₄), aryl-alcénylthio (en C₃ à C₄), aryl-alkylamino (en C₁ à C₄), aryl-alcénylamino (en C₃ à C₄), l'analogue N-alkylamino (en C₁ à C₄) du dernier radical cité, aryl-dialkylsilyle (en C₁ à C₈), diaryl-alkylsilyle (en C₁ à C₈), triarylsilyle, hétérocyclyle, hétérocyclyloxy, hétérocyclylthio et hétérocyclylamino et la partie cyclique des radicaux aryle ou hétérocyclyle est substituée éventuellement par un ou plusieurs radicaux du groupe formé par un atome d'halogène, les groupes cyano, nitro, amino, hydroxy, thio, alkyle (en C₁ à C₄), halogéno-alkyle (en C₁ à C₄), cyclo-alkyle (en C₃ à C₈), alcoxy (en C₁ à C₄), alkylthio (en C₁ à C₄), alkylamino (en C₁ à C₄) ou alcanoyle (en C₁ à C₄).

8. Amides de formule (I) et leurs sels selon une ou plusieurs des revendications 1 à 7, dans lesquelles X est -CH-, Y désigne -O- et n vaut 0.

9. Amides de formule (I) et leurs sels selon une ou plusieurs des revendications 1 à 8, dans lesquels R¹ est un groupe alkyle en C₁₋₄ mono- ou multi-substitué par F et/ou Cl.

10. Amides de formule (I) et leurs sels selon la revendication 9, dans laquelle R¹ est CF₃, CHF₂ ou CF₂Cl.

11. Procédé de fabrication des amides de formule (I) et leurs sels selon une ou plusieurs des revendications 1 à 10, comprenant la conversion d'un acide carboxylique de formule (III) 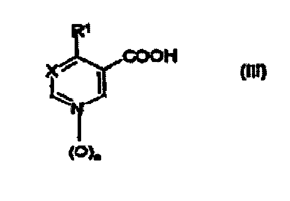 dans laquelle X, R' et n ont les significations indiquées à la revendication 1, sous la forme d'un dérivé activé de cet acide, en présence d'une base, avec un composé de formule (IV), dans laquelle R⁴ et R⁵ ont les significations indiquées à la revendication 1
HNR⁴R⁵ (IV).

12. Agent ayant un effet insecticide, acaricide, ixodicide, nématicide, mollusquicide et/ou fongicide, **caractérisé par** une teneur en au moins un composé de formule (I) ou l'un de leurs sels selon une ou plusieurs des revendications 1 à 10.

13. Agent selon la revendication 12 en mélange avec un véhicule et/ou des substances tensioactives.

14. Utilisation d'un composé de formule (I) ou d'un de ses sels selon l'une ou plusieurs des revendications 1 à 10 pour lutter contre des organismes nuisibles choisis parmi les insectes et les arachnides, à l'exception de l'utilisation pour le traitement du corps humain ou animal.

15. Utilisation selon la revendication 14, pour lutter contre les organismes nuisibles dans les plantes de culture transgénique.

16. Utilisation d'un composé de formule (I) ou d'un de ses sels selon l'une ou plusieurs des revendications 1 à 10, pour repousser ou éliminer les nuisibles et/ou les parasites.

17. Utilisation des composés de formule (I) ou de leurs sels selon une ou plusieurs des revendications 1 à 10, pour fabriquer un médicament de médecine humaine ou de médecine animale.

18. Utilisation des composés de formule (I) ou de leurs sels selon une ou plusieurs des revendications 1 à 10, pour fabriquer un médicament pour lutter contre les endo- ou ectoparasites.

19. Procédé de lutte contre les insectes nuisibles, les arachnides et/ou les nématodes, dans lequel on applique une quantité efficace d'un composé de formule (I) ou de ses sels selon une ou plusieurs des revendications 1 à 10 sur les nuisibles ou sur le lieu d'effet souhaité, à l'exception d'un procédé de traitement de l'organisme humain ou animal.

20. Procédé pour repousser ou chasser les organismes nuisibles, dans lequel on applique un ou plusieurs composés de formule (I) ou leurs sels selon une ou plusieurs des revendications 1 à 10 sur le lieu duquel les organismes nocifs doivent être éloignés ou chassés.
